# EUROPEAN PATENT SPECIFICATION

(11) **EP 1 551 357 B1**
(45) Date of publication and mention of the grant of the patent: **16.07.2014**
(21) Application number: 03799822.6
(22) Date of filing: 15.09.2003
(51) Int. Cl.: A61K 6/00

(54) **GROUP B STREPTOCOCCUS VACCINE**
GRUPPE B STREPTOKOKKEN-VAKZINE
VACCIN CONTRE LES STREPTOCOQUES DU GROUPE B

(30) Priority: 13.09.2002 US 410839 P
(43) Date of publication of application: 13.07.2005
(73) Proprietor: Novartis Vaccines and Diagnostics, Inc., Emeryville, CA 94608 (US)
(72) Inventor: RAPPUOLI, Rino, Chiron Corporation, Emeryville, CA 94662-8097 (US); TELFORD, John, 53100 Siena (IT); GRANDI, Guido, 53100 Siena (IT)
(74) Representative: Marshall, Cameron John
(86) International application number: PCT/US2003/029167
(87) International publication number: WO 2004/041157

(56) References cited:
- WO-A2-02/34771
- US-B1- 6 372 222
- US-B1- 6 426 074
- US-B1- 6 426 074

## Description

### TECHNICAL FIELD

This invention relates to polysacchandes from the bacteria *Streptococcus agalactiae* (GBS) and to their use immmumsation

### BACKGROUND ART

Once thought to infect only cows, the Gram-positive bacterium *Streptococcus agalactiae* (or "group B streptococcus", abbreviated to "GBS" (Ref. 1) is now known to cause serious disease, bacteremia and meningitis, in immunocompromised individuals and m neonates. There are two types of neonatal inflection. The first (early onset, usually within 5 days of birth) is manifested by bacteremia and pneumonia. It is contracted vertically as a baby passes through the birth canal. GBS colonises the vagina of about 25% of young women, and approximately 1% of infants born via a vaginal birth to colonised mothers will become infected. Mortality is between 50-70%. The second is a meningitis that occurs 10 to 60 days after birth. If pregnant women are vaccinated with type III capsule so that the infants are passively immunized, the incidence of the late onset meningitis is reduced but is not entirely eliminated.

The "B" in "GBS" refers to the Lancefield classification, which is based on the antigenicity of a carbohydrate which is soluble in dilute acid and called the C carbohydrate. Lancefield identified 13 types of C carbohydrate, designated A to O, that could be serologically differentiated. The organisms that most commonly infect humans are found in groups A, B, D, and G. Within group B, strains can be divided into at least 9 serotypes (Ia, Ib, Ia/c, II, III, IV, V, VI, VII and VIII) based on the structure of their polysacchande capsule. In the past, serotypes la, Ib, II, and III were equally prevalent in normal vaginal carnage and early onset sepsis in newborns. Type V GBS has emerged as an important cause of GBS infection in the USA, however, and strains of types VI and VIII have become prevalent among Japanese women.

The genome sequence of a serotype V strain 2603 V/R has been published (Ref. 2) and various polypeptides for use a vaccine antigens have been identified (Ref. 3). The vaccines currently m clinical tnals, however, are based on polysacchande antigens. These suffer from serotype-specificity and poor immunogenicity, and so there is a need for effective vaccines against *S.agalactiae* inflection.

It is an object of the invention to provide further and improved GBS vaccines.

### DISCLOSURE OF THE INVENTION

The inventors have realised that saccharide-based vaccines can be improved by using them in combination with polypeptide antigens, and *vice versa,* such that the polypeptide and the saccharide each contribute to the immunological response in a recipient. The combination is particularly advantageous where the saccharide and polypeptide are from different GBS serotypes.

The combined antigens may be present as a simple combination where separate saccharide and polypeptide antigens are administered together, or they may be present as a conjugated combination, where the saccharide and polypeptide antigens are covalently linked to each other.

Thus the invention provides an immunogenic composition comprising (i) one or more GBS polypeptide antigens and (ii) one or more GBS saccharide antigens. The polypeptide and the polysaccharide may advantageously be covalently linked to each other to form a conjugate.

Between them, the combined polypeptide and saccharide antigens preferably cover two or more GBS serotypes (*e.g*. 2, 3, 4, 5, 6, 7, 8 or more serotypes). The serotypes of the polypeptide and saccharide antigens may or may not overlap. For example, the polypeptide might protect against serogroup II or V, while the saccharide protects against either serogroups Ia, Ib, or III. Preferred combinations protect against the following groups of serotypes: (1) serotypes Ia and Ib, (2) serotypes Ia and II, (3) serotypes Ia and III, (4) serotypes Ia and IV, (5) serotypes Ia and V, (6) serotypes Ia and VI, (7) serotypes Ia and VII, (8) serotypes Ia and VIII, (9) serotypes Ib and II, (10) serotypes Ib and III, (11) serotypes Ib and IV, (12) serotypes Ib and V, (13) serotypes Ib and VI, (14) serotypes Ib and VII, (15) serotypes Ib and VIII, 16) serotypes is and III, (17) serotypes II and IV, (18) serotypes II and V, (19) serotypes II and VI, (20) serotype II and VII, (21) serotypes II and VII, (22) serotypes III and IV, (23) serotypes III and V, (24) serotypes III and VI, (25) serotypes III and VII, (26) serotypes III and VIII, (27) serotypes IV and V, (28) serotypes IV and VI, (29) serotypes IV and VII, (30) serotypes IV and VIII, (31) serotypes V and VI, (32) serotypes V and VII, (33) serotypes V and VIII, (34) serotypes VI and VII, (35) serotypes VI and VIII, and (36) serotypes VII and VIII.

Still more preferably, the combinations protect against the following groups of serotypes: (1), serotypes Ia and II, (2) serotypes Ia and V, (3) serotypes Ib and II, (4) serotypes Ib and V, (5) serotypes III and II, and (6) serotypes III and V. Most preferably, the combinations protect against serotypes III and V.

Protection against serotypes II and V is preferably provided by polypeptide antigens. Protection against serotypes Ia, Ib and/or III may be polypeptide or saccharide antigens.

Preferably, the immunogenic composition comprises one or more serogroup V antigens or fragments thereof selected from the antigen group consisting of GBS 80, GBS 91, GBS 104, GBS 147, GBS 173, GBS 276, GBS 305, GBS 313, GBS 322, GBS 328, GBS 330, GBS 338, GBS 358, GBS 361, GBS 404, GBS 656, GBS 690, and GBS 691. Preferably, the composition comprises a composition of at least two of these GBS antigens or a fragment thereof.

In one embodiment, the immunogenic composition comprises a GBS sacchande antigen and at least two GBS polypeptide antigens or fragments thereof, wherein said GBS sacchande antigen comprises a saccharide selected from GBS serotype Ia, Ib, and III, and wherein said GBS polypeptide antigens comprise a combination of at least two polypeptide or a fragment thereof selected from the antigen group consisting of GBS 80, GBS 91, GBS 104, GBS 147, GBS 173, GBS 276, GBS 305, GBS 313, GBS 322, GBS 328, GBS 330, GBS 338, GBS 358, GBS 361, GBS 404, GBS 656, GBS 690, and GBS 691.

Preferably, the combmation comprises GBS 80 or a fragment thereof. In one embodiment, the GBS polypeptide antigens comprise a combination of two GBS antigens or fragments thereof selected from the antigen group consisting of (1) GBS 80 and GBS 91, (2) GBS 80 and GBS 104, (3) GBS 80 and GBS 147, (4) GBS 80 and GBS 173, (5) GBS 80 and GBS 276, (6) GBS 80 and GBS 305, (7) GBS 80 and GBS 313, (8) GBS 80 and GBS 322, (9) GBS 80 and GBS 328, (10) GBS 80 and GBS 330, (11) GBS 80 and GBS 338, (12) GBS 80 and GBS 358, (13) GBS 80 and GBS 361, (14) GBS 80 and GBS 404, (14) GBS 80 and GBS 404, (15) GBS 80 and GBS 656, (16) GBS 80 and GBS 690, and (17) GBS 80 and GBS 691.

Still more preferably, the combmation is selected from the antigen group consisting of (1) GBS 80 and GBS 338; (2) GBS 80 and GBS 361, (3) GBS 80 and GBS 305, (4) GBS 80 and GBS 328, (5) GBS 80 and GBS 690, (6) GBS 80 and GBS 691 and (7) GBS 80 and GBS 147. Even more preferably, the combination comprises GBS 80 and GBS 691.

In one embodiment, the composition comprises a combination at least three GBS polypeptide antigens. Preferably, this combmation comprises GBS 80 and GBS 691.

Preferably, the immunogenic composition further comprises a GBS polypeptide or a fragment thereof of serogroup II

### The polypeptide antigen

The polypeptide is preferably: (a) a polypeptide comprising an amino acid sequence selected from the group consisting of the even-numbered SEQ IDs 2-10966 from Ref. 3; (b) a polypeptide comprising an amino acid sequence having sequence identity to an ammo acid sequence from in (a); or (c) a polypeptide comprising a fragment of an amino acid sequence from (a).

Within (a), preferred SEQ IDs are those which encode GBS1 to GBS689 (see Table IV of reference 3)

Within (b), the degree of sequence identity may vary depending on the amino acid sequence (a) in question, but is preferably greater than 50% (e g 60%, 70%, 80%, 90%, 95%, 99% or more). Polypeptides within (b) include homologs, orthologs, allelic variants and functional mutants of (a). Typically, 50% identity or more between two proteins is considered to be an indication of functional equivalence. Identity between proteins is preferably determined by the Smith-Waterman homology search algorithm as implemented in the MPSRCH program (Oxford Molecular), using an affine gap search with parameters *gap open penalty=12* and *gap extension penalty=1.*

Within (c), the length of the fragment may vary depending on the amino acid sequence (a) in question, but the fragment is preferably at least 7 consecutive ammo acids from the sequences of (a) e.g. 8, 10, 12, 14, 16, 18, 20, 30, 40, 50, 60, 70, 80, 90, 100, 150, 200 or more. Preferably the fragment comprises one or more epitopes from the sequence. Other preferred fragments are the N-terminal signal peptides of SEQ IDs 1-10966 from Ref. 3, SEQ IDs 1-10966 from Ref. 3 without their N-termmal signal peptides, and SEQ IDs 1-10966 from Ref. 3 wherein up to 10 amino acid residues (*i.e* 1, 2, 3, 4, 5, 6, 7, 8, 9 or 10 residues) are deleted from the N-terminus and/or the C-terminus e.g. the N-terminal ammo acid residue may be deleted.

The polypeptides can, of course, be prepared by various means (*e.g.* recombinant expression, purification from GBS, chemical synthesis *etc*.) and in various forms (*e.g*. native, fusions, glycosylated, non-glycosylated *etc.*). They are preferably prepared in substantially pure form (*i.e.* substantially free from other streptococcal or host cell proteins) or substantially isolated form.

Preferred polypeptide antigens are: GBS 80, GBS 91, GBS 104, GBS 147, GBS 173, GBS 276, GBS 305, GBS 313, GBS 322, GBS 328, GBS 330, GBS 338, GBS 358, GBS 361, GBS 404, GBS 656, GBS 690, and GBS 691, including polypeptides having amino acid sequences with sequence identity thereto *etc.*

The nucleotide and amino acid sequences of GBS80 in Ref. 3 are SEQ ID 8779 and SEQ ID 8780. These sequences are set forth below as SEQ ID NOS 1 and 2:
**SEQ ID NO. 1**
**SEQ ID NO: 2**

The nucleotide and amino acid sequences of GBS 91 in Ref. 3 are SEQ ID 8937 and SEQ ID 8938. These sequences are set forth below as SEQ ID NOS 3 and 4:
**SEQ ID NO. 3**
**SEQ ID NO. 4**

The nucleotide and amino acid sequences of GBS 104 in Ref. 3 are SEQ ID 8777 and SEQ ID 8778. These sequences are set forth below as SEQ ID NOS 5 and 6:
**SEQ ID NO. 5**
**SEQ ID NO. 6**

The nucleotide and amino acid sequences of GBS 147 in Ref. 3 are SEQ ID 8525 and SEQ ID 8526. These sequences are set forth below as SEQ ID NOS 7 and 8:
**SEQ ID NO. 7**
**SEQ ID NO. 8**

The nucleotide and amino acid sequences of GBS 173 in Ref. 3 are SEQ ID 8787 and SEQ ID 8788. These sequences are set forth below as SEQ ID NOS 9 and 10:
**SEQ ID NO.9**
**SEQ ID NO. 10**

The nucleotide and amino acid sequences of GBS 276 in Ref. 3 are SEQ ID 8941 and SEQ ID 8942. These sequences are set forth below as SEQ ID NOS 11 and 12:
**SEQ ID NO.11**
**SEQ ID NO. 12**

The nucleotide and amino acid sequences of GBS 305 in Ref. 3 are SEQ ID 207 and SEQ ID 208. These sequences are set forth below as SEQ ID NOS 13 and 14:
**SEQ ID NO. 13**
**SEQ ID NO. 14**

The nucleotide and amino acid sequences of GBS 313 are in Ref. 3 are SEQ ID 4089 and SEQ ID 4090. These sequences are set forth as SEQ ID NOS 15 and 16 below:
**SEQ ID NO. 15**
**SEQ ID NO. 16**

The nucleotide and amino acid sequences of GBS 322 in Ref. 3 are SEQ ID 8539 and SEQ ID 8540. These sequences are set forth below as SEQ ID NOS 17 and 18:
**SEQ ID NO. 17**
**SEQ ID NO. 18**

The nucleotide and amino acid sequences of GBS 328 in Ref. 3 are SEQ ID 6015 and SEQ ID 6016. These sequences are set forth below as SEQ ID NOS 19 and 20:
**SEQ ID NO. 19**
**SEQ ID NO. 20**

The nucleotide and amino acid sequences of GBS 330 in Ref. 3 are SEQ ID 8791 and SEQ ID 8792. These sequences are set forth below as SEQ ID NOS 21 and 22:
**SEQ ID NO. 21**
**SEQ ID NO. 22**

The nucleotide and amino acid sequences of GBS 338 in Ref. 3 are SEQ ID 8637 and SEQ ID 8638. These sequences are set forth below as SEQ ID NOS 23 and 24:
**SEQ ID NO. 23**
**SEQ ID NO. 24**

The nucleotide and amino acid sequences of GBS 358 in Ref. 3 are SEQ ID 3183 and SEQ ID 3184. These sequences are set forth below as SEQ ID NOS 25 and 26:
**SEQ ID NO. 25**
**SEQ ID NO. 26**

The nucleotide and amino acid sequences of GBS 361 in Ref. 3 are SEQ ID 8769 and SEQ ID 8770. These sequences are set forth below as SEQ ID NOS 27 and 28:
**SEQ ID NO. 27**
**SEQ ID NO. 28**

The nucleotide and amino acid sequences of GBS 404 in Ref. 3 are SEQ ID 8799 and SEQ ID 8800. These sequences are set forth below as SEQ ID NOS 29 and 30:
**SEQ ID NO. 29**
**SEQ ID NO. 30**

The nucleotide and amino acid sequences of GBS 656 in Ref. 3 are SEQ ID 9323 and SEQ ID 9324. These sequences are set forth below as SEQ ID NOS 31 and 32:
**SEQ ID NO. 31**
**SEQ ID NO. 32**

The nucleotide and amino acid sequences of GBS 690 in Ref. 3 are SEQ ID 9965 and SEQ ID 9966. These sequences are set forth as SEQ ID NOS 33 and 34 below:
**SEQ ID NO. 33**
**SEQ ID NO. 34**

The nucleotide and amino acid sequences of GBS 691 in Ref. 3 are SEQ ID 3691 and SEQ ID 3692. These sequences are set forth as SEQ ID NOS 35 and 36 below:
**SEQ ID NO. 35**
**SEQ ID NO. 36**

Other preferred polypeptide antigens include: GBS4 (SEQ ID 2 from Ref. 3); GBS22 (SEQ ID 8584 from Ref. 3); and GBS85 (SEQ ID 216 from Ref. 3), including polypeptides having amino acid sequences with sequence identity thereto *etc.*

The polypeptide is preferably not a C protein (alpha or beta or epsilon) or a R protein (Rib). The nucleotide and amino acid sequences of GBS 4 in Ref. 3 are SEQ ID 1 and SEQ ID 2.

These sequences are set forth below as SEQ ID NOS 37 and 38:
**SEQ ID NO. 37**
**SEQ ID NO. 38**

The nucleotide and amino acid sequences of GBS 22 in Ref. 3 are SEQ 8583 and SEQ ID 8584. These sequences are set forth below as SEQ ID NOS 39 and 40:
**SEQ ID NO. 39**
**SEQ ID NO. 40**

The nucleotide and amino acid sequences of GBS 85 in Ref. 3 are SEQ ID 215 and SEQ ID 216. These sequences are set forth below as SEQ ID NOS 41 and 42:
**SEQ ID NO. 41**
**SEQ ID NO. 42**

GBS polypeptides of the invention may be present in the composition as individual separate polypeptides. It is preferred, however, that two or more (*i.e.* 2, 3, 4, 5, 6, 7, 8, 9, 10, 11, 12, 13, 14, 15, 16, 17, 18, 19 or 20) of the antigens are expressed as a single polypeptide chain (a 'hybrid' polypeptide). Hybrid polypeptides offer two principal advantages: first, a polypeptide that may be unstable or poorly expressed on its own can be assisted by adding a suitable hybrid partner that overcomes the problem; second, commercial manufacture is simplified as only one expression and purification need be employed in order to produce two polypeptides which are both antigenically useful.

The hybrid polypeptide may comprise two or more polypeptide sequences from the first antigen group. Accordingly, the invention includes a composition comprising a first amino acid sequence and a second amino acid sequence, wherein said first and second ammo acid sequences are selected from a GBS antigen or a fragment thereof. Preferably, the first and second ammo acid sequences in the hybrid polypeptide comprise different epitopes.

The hybrid polypeptide may comprise one or more polypeptide sequences from different GBS serotypes Accordingly, the invention includes a composition comprising a first ammo acid sequence and a second amino acid sequence, said first amino acid sequence and said second amino acid sequence selected from a GBS serotype selected from the group consisting of serotypes Ia, Ib, Ia/c, II, III, IV, V, VI, VII and VIII The first and second amino acid sequence may be from the same GBS serotype or they may be from different GBS serotypes. Preferably, the first and second amino acid sequence are selected a GBS serotype selected from the group consisting of serotypes II and V Most preferably, at least one of the first and second ammo acid sequences is from GBS serotype V Preferably, the first and second ammo acid sequences in the hybrid polypeptide comprise difference epitopes.

In one embodiment, the hybrid polypeptide comprises one or more GBS antigens from serotype V. Preferably, the hybrid polypeptide comprises a first amino acid sequence and a second amino acid sequence, said first amino acid sequence and said second ammo acid sequence comprising a GBS antigen or a fragment thereof selected from the group consisting of GBS 80, GBS 91, GBS 104, GBS 147, GBS 173, GBS 276, GBS 305, GBS 313, GBS 322, GBS 328, GBS 330, GBS 338, GBS 358, GBS 361, GBS 404, GBS 656, GBS 690, and GBS 691. Preferably, the GBS antigen or fragment thereof is selected from the group consisting of GBS 80 and GBS 691. Preferably, the first and second amino acid sequences in the hybrid polypeptide comprise difference epitopes.

Hybrids consisting of ammo acid sequences from two, three, four, five, six, seven, eight, nine, or ten GBS antigens are preferred In particular, hybrids consisting of amino acid sequences from two, three, four, or five GBS antigens are preferred

Different hybrid polypeptides may be mixed together in a single formulation. Within such combinations, a GBS antigen may be present in more than one hybrid polypeptide and/or as a non-hybnd polypeptide. It is preferred, however, that an antigen is present either as a hybrid or as a non-hybrid, but not as both.

Preferably, the GBS antigen in one of the hybrid polypeptides is GBS 80 or a fragment thereof. Accordingly, examples of two-antigen hybrids for use m the invention may comprise: (1) GBS 80 and GBS 91, (2) GBS 80 and GBS 104, (3) GBS 80 and GBS 147, (4) GBS 80 and GBS 173, (5) GBS 80 and GBS 276, (6) GBS 80 and GBS 305, (7) GBS 80 and GBS 313, (8) GBS 80 and GBS 322, (9) GBS 80 and GBS 328, (10) GBS 80 and GBS 330, (11) GBS 80 and GBS 338, (12) GBS 80 and GBS 358, (13) GBS 80 and GBS 361, (14) GBS 80 and GBS 404, (14) GBS 80 and GBS 404, (15) GBS 80 and GBS 656, (16) GBS 80 and GBS 690, and (17) GBS 80 and GBS 691. Preferably, a two-antigen hybrid for use m the invention comprises GBS 80 and GBS 691.

Hybrid polypeptides can be represented by the formula **NH₂-A-{-X-L-}*ₙ*-B-COOH,** wherein: X is an ammo acid sequence of a GBS antigen or a fragment thereof, L is an optional linker amino acid sequence; A is an optional N-terminal amino acid sequence; B is an optional C-terminal ammo acid sequence; and *n* is 2, 3, 4, 6, 6, 7, 8, 9, 10, 11, 12, 13, 14 or 15

If a -X- moiety has a leader peptide sequence in its wild-type form, this may be included or omitted in the hybrid protein. In some embodiments, the leader peptides will be deleted except for that of the -X- moiety located at the N-terminus of the hybnd protein *i.e*. the leader peptide of X₁ will be retained, but the leader peptides of X₂ ... Xₙ will be omitted. This is equivalent to deleting all leader peptides and using the leader peptide of X₁ as moiety -A-.

For each *n* instances of {-X-L-}, linker ammo acid sequence -L- may be present or absent. For instance, when -*n*=2 the hybrid may be NH₂-X₁-L₁-X₂-L₂-COOH, NH₂-X₁-X₂-COOH, NH₂-X₁-L₁-X₂-COOH, NH₂-X₁-X₂-L₂-COOH, *etc*. Linker ammo acid sequence(s) -L- will typically be short (*e.g.* 20 or fewer amino acids *i.e* 19, 18, 17, 16, 15, 14, 13, 12, 11, 10, 9, 8, 7, 6, 5, 4, 3, 2, 1). Examples comprise short peptide sequences which facilitate cloning, poly-glycine linkers (*i.e* comprising Gly*ₙ* where *n* = 2, 3, 4, 5, 6, 7, 8, 9, 10 or more), and histidine tags (*i.e*. His*ₙ* where *n* = 3, 4, 5, 6, 7, 8, 9, 10 or more). Other suitable linker amino acid sequences will be apparent to those skilled in the art. A useful linker is GSGGGG (SEQ ID 1), with the Gly-Ser dipeptide being formed from a *Bam*HI restriction site, thus aiding cloning and manipulation, and the (Gly)₄ tetrapeptide being a typical poly-glycme linker.

-A- is an optional N-terminal amino acid sequence This will typically be short (*e.g*. 40 or fewer amino acids *i.e* 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1) Examples include leader sequences to direct protein trafficking, or short peptide sequences which facilitate cloning or purification (*e.g.* histidine tags *i.e* His*ₙ* where *n* = 3, 4, 5, 6, 7, 8, 9, 10 or more) Other suitable N-terminal amino acid sequences will be apparent to those skilled in the art. If X₁ lacks its own N-terminus methionine, -A-is preferably an oligopeptide (*e.g.* with 1, 2, 3, 4, 5, 6, 7 or 8 amino acids) which provides a N-terminus methionine.

-B- is an optional C-terminal amino acid sequence. This will typically be short (*e.g*. 40 or fewer amino acids *i.e*. 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1) Examples include sequences to direct protein trafficking, short peptide sequences which facilitate cloning or purification (*e.g*. comprising histidine tags *i.e* His*ₙ* where *n* = 3, 4, 5, 6, 7, 8, 9, 10 or more), or sequences which enhance protein stability. Other suitable C-terminal amino acid sequences will be apparent to those skilled in the art

Most preferably, *n* is 2 or 3.

### The saccharide antigen

The saccharide antigen is generally the capsular polysaccharide of a GBS or a derivative thereof Suitable derivatives include oligosaccharide (*e.g*. from 3 to 150, preferably 8 to 100, monosacchande units) fragments of the polysaccharide (*e.g* refs 12 to 16), de-acetylated sacchandes (Ref. 16), N-acroylated sacchandes (16), sacchandes with terminal aldehyde groups, *etc*

The sacchande is preferably conjugated to a carrier molecule to enhance immunogenicity *(e.g.* see refs. 4 to 23 *etc.)* In some embodiments of the invention the GBS saccharide is conjugated to a GBS protein as defined above, thereby giving a polypeptide/saccharide combination of the invention in a single molecule. In other embodiments the GBS sacchande is conjugated to a non-GBS protein, in which case the conjugate will be combined with a separate GBS protein to give a polypeptide/sacchande combination of the invention.

Non-GBS carrier polypeptides include tetanus toxoid, the *N.meningitidis* outer membrane protein (24), synthetic peptides (25, 26), heat shock proteins (27, 28), pertussis proteins (29, 30), protein D from *H.influenzae* (31), cytokines (32), lymphokines (32), hormones (32), growth factors (32), toxin A or B from *C.difficile* (33), iron-uptake proteins (34) *etc.* Preferred carrier proteins are the CRM197 diphtheria toxoid (35) and tetanus toxoid.

The sacchande and polypeptide are joined covalently. This may involve a direct covalent bond between the saccharide and polypeptide, or indirect coupling via a linker or spacer may be used (*e.g* via a B-propionamido linker (16), *etc*.). Any suitable conjugation chemistry may be used (*e.g* reductive amination (21) *etc*.). Linkage is preferably via a terminal sacchande in the polysaccharide.

A single carrier molecule may carry saccharide antigens of a single type (*e.g*. saccharides derived from a single GBS serotype) or may carry multiple different antigens (*e.g.* saccharides derived from multiple GBS serotypes, all conjugated to the same carrier).

The saccharides can, of course, be prepared by various means (*e.g*. purification of the sacchande from GBS, chemical synthesis, *etc*.), in various sizes (*e.g*. full-length, fragmented, *etc*.) and may be derivatised for linking to carriers. They are preferably prepared in substantially pure form (*i.e*. substantially free from other streptococcal sacchandes) or substantially isolated form. Processes for prepanng capsular polysacchandes from GBS are well known in the art (*e.g*. refs. 36 to 39) and processes for prepanng oligosaccharides from polysacchandes are also known (*e.g*. hydrolysis, sonication, enzymatic treatment, treatment with a base followed by nitrosation, *etc* (12 to 16))

As an alternative to using a sacchande antigen in non-conjugated combinations, a peptide mimetic of the GBS capsular polysacchande may be used (*e.g* 40). Suitable peptides can be selected by techniques such as phage display using protective anti-saccharide antibodies As a further alternative, an anti-idiotypic antibody may be used instead of a sacchande antigen (*e.g* ref. 41)

### Prime/boost schedules

Polypeptide/saccharide combinations of the invention may be given as single doses or as part of a pnme/boost schedule. In a prime/boost schedule, the combinations may be used as the priming dose, the boosting dose(s), or both.

If a combination is used for both pnming and boosting, it is preferred to use the same combination both times. If a combination is used for only one of priming and boosting, it is preferred that the other dose should use the polypeptide or sacchande on which the combination is based. Thus the invention provides a prime-boost schedule where either (i) one of the saccharide and polypeptide antigens is used for priming an immune response and a combination are used for boosting the response, or (ii) combined saccharide and polypeptide antigens are used for priming an immune response but only one is used for boosting the response

Various timings for priming and boosting are suitable for use with the invention. In one embodiment, a priming dose is given to a child and a booster is given to a teenager (13-18 years) or young adult (19-25 years). In another embodiment, a priming dose is given to a teenager or young adult and a booster is given dunng pregnancy. In another embodiment, a priming dose is given to a female who intends to become pregnant and a booster is given during pregnancy.

### Immunogenic pharmaceutical compositions

Polypeptide/sacchande combinations are formulated as immunogenic compositions, and more preferably as compositions suitable for use as a vaccine in humans (*e.g*. children or adults). Vaccines of the invention may either be prophylactic (*i.e* to prevent infection) or therapeutic (*i.e* to treat disease after infection), but will typically be prophylactic Accordingly, the invention includes a method for the therapeutic or prophylactic treatment of GBS infection in an animal susceptible to GBS infection comprising administering to said animal a therapeutic or prophylactic amount of the immunogenic compositions of the invention.

The composition of the invention is preferably stenle.

The composition of the invention is preferably pyrogen-free.

The composition of the invention generally has a pH of between 6.0 and 7 0, more preferably to between 6.3 and 6.9 *e.g*. 6.6±0.2 The composition is preferably buffered at this pH.

Other components suitable for human administration are disclosed in reference 42

Vaccines of the invention may be administered in conjunction with other immunoregulatory agents. In particular, compositions will usually include an adjuvant. Preferred further adjuvants include, but are not limited to, one or more of the following set forth below:

### A Mineral Containing Compositions

Mineral containing compositions suitable for use as adjuvants in the invention include mineral salts, such as aluminum salts and calcium salts. The invention includes mineral salts such as hydroxides (*e.g.* oxyhydroxides), phosphates (*e.g* hydroxyphoshpates, orthophosphates), sulphates, *etc.* {*e.g*. see chapters 8 & 9 of ref. 43}), or mixtures of different mineral compounds, with the compounds taking any suitable form (*e.g.* gel, crystalline, amorphous, *etc*.), and with adsorption being preferred. The mineral containing compositions may also be formulated as a particle of metal salt See ref 44.

### B Oil-Emulsions

Oil-emulsion compositions suitable for use as adjuvants in the invention include squalene-water emulsions, such as MF59 (5% Squalene, 0.5% Tween 80, and 0 5% Span 85, formulated into submicron particles using a microfluidizer). See ref 45

Complete Freund's adjuvant (CFA) and incomplete Freund's adjuvant (IFA) may also be used as adjuvants in the invention.

### C. Saponin Formulations

Saponin formulations, may also be used as adjuvants m the invention. Sapomns are a heterologous group of sterol glycosides and triterpenoid glycosides that are found in the bark, leaves, stems, roots and even flowers of a wide range of plant species Saponin from the bark of the *Quillaia saponaria* Molina tree have been widely studied as adjuvants. Saponin can also be commercially obtained from *Smilax ornata* (sarsapnlla), *Gypsophilla paniculata* (brides veil), and *Saponana officianalis* (soap root). Saponin adjuvant formulations include purified formulations, such as QS21, as well as lipid formulations, such as ISCOMs.

Saponin compositions have been purified using High Performance Thin Layer Chromatography (HP-LC) and Reversed Phase High Performance Liquid Chromatography (RP-HPLC) Specific purified fractions using these techniques have been identified, including QS7, QS17, QS18, QS21, QH-A, QH-B and QH-C. Preferably, the saponin is QS21. A method of production of QS21 is disclosed in U.S. Patent No. 5,057,540 Saponin formulations may also comprise a sterol, such as cholesterol (see WO 96/33739).

Combinations of saponms and cholesterols can be used to form unique particles called Immunostimulating Complexs (ISCOMs) ISCOMs typically also include a phospholipid such as phosphatidylethanolamine or phosphatidylcholine. Any known saponin can be used in ISCOMs Preferably, the ISCOM includes one or more of Quil A, QHA and QHC ISCOMs are further described in EP 0 109 942, WO 96/11711 and WO 96/33739. Optionally, the ISCOMS may be devoid of additional detergent. See ref. 46.

A review of the development of saponin based adjuvants can be found at ref. 47

### C. Virosomes and Virus Like Particles (VLPs)

Virosomes and Virus Like Particles (VLPs) can also be used as adjuvants in the invention These structures generally contain one or more proteins from a virus optionally combined or formulated with a phospholipid. They are generally non-pathogenic, non-replicating and generally do not contain any of the native viral genome. The viral proteins may be recombmantly produced or isolated from whole viruses. These viral proteins suitable for use in virosomes or VLPs include proteins derived from influenza virus (such as HA or NA), Hepatitis B virus (such as core or capsid proteins), Hepatitis E virus, measles virus, Sindbis virus, Rotavirus, Foot-and-Mouth Disease virus, Retrovirus, Norwalk virus, human Papilloma virus, HIV, RNA-phages, Qß-phage (such as coat proteins), GA-phage, fr-phage, AP205 phage, and Ty (such as retrotransposon Ty protein p1). VLPs are discussed further in WO 03/024480, WO 03/024481, and Refs 48, 49, 50 and 51. Virosomes are discussed further in, for example, Ref 52

### D. Bacterial or Microbial Denvatives

Adjuvants suitable for use m the invention include bacterial or microbial derivatives such as:

### (1) Non-toxic derivatives of enterobacterial lipopolysaccharide (LPS)

Such derivatives include Monophosphoryl lipid A (MPL) and 3-O-deacylated MPL (3dMPL). 3dMPL is a mixture of 3 De-O-acylated monophosphoryl lipid A with 4, 5 or 6 acylated chains A preferred "small particle" form of 3 De-O-acylated monophosphoryl lipid A is disclosed in EP 0 689 454. Such "small particles" of 3dMPL are small enough to be sterile filtered through a 0.22 micron membrane (see EP 0 689 454). Other non-toxic LPS derivatives include monophosphoryl lipid A mimics, such as aminoalkyl glucosaminide phosphate derivatives e g RC-529. See Ref. 53

### (2) Lipid A Derivatives

Lipid A derivatives include derivatives of lipid A from *Escherichia coli* such as OM-174. OM-174 is described for example in Ref. 54 and 55.

### (3) Immunostimulatory oligonucleotides

Immunostimulatory oligonucleotides suitable for use as adjuvants m the invention include nucleotide sequences containing a CpG motif (a sequence containing an unmethylated cytosine followed by guanosine and linked by a phosphate bond). Bactenal double stranded RNA or oligonucleotides containing palmdromic or poly(dG) sequences have also been shown to be immunostimulatory.

The CpG's can include nucleotide modifications/analogs such as phosphorothioate modifications and can be double-stranded or single-stranded Optionally, the guanosine may be replaced with an analog such as 2'-deoxy-7-deazaguanosine See ref. 56, WO 02/26757 and WO 99/62923 for examples of possible analog substitutions. The adjuvant effect of CpG oligonucleotides is further discussed in Refs. 57, 58, WO 98/40100, U.S. Patent No. 6,207,646, U.S. Patent No. 6,239,116, and U.S Patent No. 6,429,199.

The CpG sequence may be directed to TLR9, such as the motif GTCGTT or TTCGTT See ref. 59. The CpG sequence may be specific for inducing a Th1 immune response, such as a CpG-A ODN, or it may be more specific for inducing a B cell response, such a CpG-B ODN. CpG-A and CpG-B ODNs are discussed in refs. 60, 61 and WO 01/95935 Preferably, the CpG is a CpG-A ODN. Preferably, the CpG oligonucleotide is constructed so that the 5' end is accessible for receptor recognition. Optionally, two CpG oligonucleotide sequences may be attached at their 3' ends to form "immunomers" See, for example, refs. 62, 63, 64 and WO 03/035836.

### (4) ADP-ribosylating toxins and detoxified detivatives thereof.

Bactenal ADP-nbosylating toxins and detoxified derivatives thereof may be used as adjuvants in the invention. Preferably, the protein is derived from *E*. *coli* (i.e., E. coli heat labile enterotoxm "LT), cholera ("CT"), or pertussis ("PT") The use of detoxified ADP-ribosylating toxins as mucosal adjuvants is described in WO 95/17211 and as parenteral adjuvants in WO 98/42375. Preferably, the adjuvant is a detoxified LT mutant such as LT-K63, LT-R72, and LTR192G. The use of ADP-ribosylating toxins and detoxified derivaties thereof, particularly LT-K63 and LT-R72, as adjuvants can be found in Refs. 65, 66, 67, 68, 69, 70, 71 and 72 each of which is specifically incorporated by reference herein in their entirety. Numerical reference for amino acid substitutions is preferably based on the alignments of the A and B subunits of ADP-ribosylating toxins set forth in Domenighini et al., Mol. Microbiol (1995) 15(6):1165 - 1167.

### E. Human Immunomodulators

Human immunomodulators suitable for use as adjuvants in the invention include cytokines, such as mterleukins (*e.g* IL-1, IL-2, IL-4, IL-5, IL-6, IL-7, IL-12, *etc*.), interferons (*e.g.* mterferon-γ), macrophage colony stimulating factor, and tumor necrosis factor.

### F. Bioadhesives and Mucoadhesives

Bioadhesives and mucoadhesives may also be used as adjuvants in the invention. Suitable bioadhesives include estenfied hyaluromc acid microspheres (Ref 73) or mucoadhesives such as cross-linked derivatives of poly(acrylic acid), polyvinyl alcohol, polyvinyl pyrolhdone, polysacchandes and carboxymethylcellulose. Chitosan and derivatives thereof may also be used as adjuvants in the invention E.g., ref 74. ,

### G. Microparticles

Microparticles may also be used as adjuvants in the invention. Microparticles (*i.e.* a particle of ∼100nm to ∼150µm in diameter, more preferably ∼200nm to ∼30µm in diameter, and most preferably ∼500nm to ∼10µm in diameter) formed from matenals that are biodegradable and non-toxic (*e.g*. a poly(α-hydroxy acid), a polyhydroxybutync acid, a polyorthoester, a polyanhydride, a polycaprolactone, *etc*.), with poly(lactide-co-glycolide) are preferred, optionally treated to have a negatively-charged surface (*e.g* with SDS) or a positively-charged surface (*e.g*. with a cationic detergent, such as CTAB).

### H. Liposomes

Examples of liposome formulations suitable for use as adjuvants are described in U.S Patent No. 6,090,406, U.S. Patent No 5,916,588, and EP 0 626 169

### I. Polyoxyethylene ether and Polyoxyethylene Ester Formulations

Adjuvants suitable for use m the invention include polyoxyethylene ethers and polyoxyethylene esters. Ref. 75 Such formulations further include polyoxyethylene sorbitan ester surfactants in combination with an octoxynol (Ref. 76) as well as polyoxyethylene alkyl ethers or ester surfactants in combination with at least one additional non-ionic surfactant such as an octoxynol (Ref. 77).

Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether

### J Polyphosphazene (PCPP)

PCPP formulations are described, for example, in Ref. 78 and 79

### K. Muramyl peptides

Examples of muramyl peptides suitable for use as adjuvants m the invention include N-acetyl-muramyl-L-threonyl-D-isoglutamine (thr-MDP), N-acetyl-normuramyl-L-alanyl-D-isoglutamine (nor-MDP), and N-acetylmuramyl-L-alanyl-D-isoglutaminyl-L-alanine-2-(1'-2'-dipalmitoyl-sn- glycero-3-hydroxyphosphoryloxy)-ethylamine MTP-PE)

### L. Imidazoquinolone Compounds.

Examples of imidazoquinolone compounds suitable for use adjuvants in the invention include Imiquamod and its homologues, descnbed further m Ref. 80 and 81.

The invention may also comprise combinations of aspects of one or more of the adjuvants identified above For example, the following adjuvant compositions may be used in the invention:
(1) a saponin and an oil-in-water emulsion (ref 82);
(2) a saponin (e.g , QS21) + a non-toxic LPS derivative (e.g., 3dMPL) (see WO 94/00153);
(3) a saponin (e.g. , QS21) + a non-toxic LPS derivative (e.g., 3dMPL) + a cholesterol,
(4) a saponin (e g QS21) + 3dMPL + IL-12 (optionally + a sterol) (Ref. 83), combinations of 3dMPL with, for example, QS21 and/or oil-in-water emulsions (Ref. 84);
(5) SAF, containing 10% Squalane, 0.4% Tween 80, 5% pluronic-block polymer L121, and thr-MDP, either microfluidized into a submicron emulsion or vortexed to generate a larger particle size emulsion.
(6) Ribᵢ™ adjuvant system (RAS), (Ribi Immunochem) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™), and
(7) one or more mineral salts (such as an aluminum salt) + a non-toxic derivative of LPS (such as 3dPML).

Aluminium salts and MF59 are preferred adjuvants for parenteral immunisation. Mutant bacterial toxins are preferred mucosal adjuvants.

The composition may include an antibiotic.

GBS polypeptide(s) and sacchande(s) m the compositions of the invention will be present in 'immunologically effective amounts' *i.e*. the administration of that amount to an individual, either in a single dose or as part of a series, is effective for treatment or prevention of disease. This amount vanes depending upon the health and physical condition of the individual to be treated, age, the taxonomic group of individual to be treated (*e.g.* non-human primate, pnmate, *etc*.), the capacity of the individual's immune system to synthesise antibodies, the degree of protection desired, the formulation of the vaccine, the treating doctor's assessment of the medical situation, and other relevant factors It is expected that the amount will fall in a relatively broad range that can be determined through routine trials.

Typically, the compositions of the invention are prepared as injectables. Direct delivery of the compositions will generally be parenteral (*e.g* by injection, either subcutaneously, mtrapentoneally, intravenously or intramuscularly or delivered to the interstitial space of a tissue) or mucosal (*e.g* oral or intranasal [85,86]). The compositions can also be admimstered into a lesion. The invention provides a syringe containing a composition of the invention.

Once formulated, the compositions of the invention can be administered directly to the subject. The subjects to be treated can be animals, in particular, human subjects can be treated The vaccines are particularly useful for vaccinating children and teenagers, and more particularly females.

As well as GBS polypeptides and saccahrides, the composition of the invention may comprise further antigens For example, the composition may comprise one or more of the following further antigens
- antigens from *Helicobacter pylori* such as CagA [87 to 90], VacA [91, 92], NAP [93, 94, 95], HopX [*e.g.* 96], HopY [*e.g.* 96] and/or urease.
- a saccharide antigen from *N.meningitidis* serogroup A, C, W135 and/or Y, such as the oligosaccharide disclosed in ref 97 from serogroup C [see also ref 98] or the oligosaccharides of ref 99
- a saccharide antigen from *Streptococcus pnenmoniae* [*e.g.* 100, 101, 102].
- an antigen from hepatitis A virus, such as inactivated virus [*e.g*. 103, 104].
- an antigen from hepatitis B virus, such as the surface and/or core antigens [*e.g*. 104, 105].
- an antigen from *Bordetella pertussis,* such as pertussis holotoxin (PT) and filamentous haemagglutinin (FHA) from *B. pertussis,* optionally also in combination with pertactin and/or agglurinogens 2 and 3 [*e.g.* refs. 106 & 107].
- a diphtheria antigen, such as a diphtheria toxoid [*e.g.* chapter 3 of ref 108] *e.g.* the CRM₁₉₇ mutant [*e.g.* 109].
- a tetanus antigen, such as a tetanus toxoid [*e.g*. chapter 4 of ref. 128].
- a saccharide antigen from *Haemophilus influenzae* B [*e.g.* 98].
- an antigen from hepatitis C virus [*e.g*. 110].
- an antigen from *N.gonorrhoeae* [*e.g.* 111, 112, 113, 114].
- an antigen from *Chlamydia pneumoniae* [*e.g* refs. 115 to 121].
- an antigen from *Chlamydia trachomatis* [*e.g.* 122].
- an antigen from *Porphyromonas gingivalis* [*e.g.* 123]
- poho antigen(s) [*e.g.* 124, 125] such as OPV or, preferably, IPV
- rabies antigen(s) [*e.g* 126] such as lyophilised inactivated virus [*e.g.* 127, RabAvert™]
- measles, mumps and/or rubella antigens [*e.g.* chapters 9, 10 & 11 of ref. 128].
- influenza antigen(s) [*e.g*. chapter 19 of ref. 128], such as the haemagglutinin and/or neuraminidase surface proteins
- an antigen from *Moraxella catarrhalis* [*e.g* 129].
- an antigen from *Streptococcus pyogenes* (group A streptococcus) [*e.g*. 3, 130, 131].
- an antigen from *Staphylococcus aureus* [*e.g.* 132].
- an antigen from *Bacillus anthracis* [*e.g.* 133, 134, 135].
- an antigen from a virus in the flaviviridae family (genus flavivirus), such as from yellow fever virus, Japanese encephalitis virus, four serotypes of Dengue viruses, tick-borne encephalitis virus, West Nile virus.
- a pestivirus antigen, such as from classical porcine fever virus, bovine viral diarrhoea virus, and/or border disease virus.
- a parvovirus antigen *e.g.* from parvovirus B19.
- a pnon protein (*e.g.* the CJD prion protein)
- an amyloid protein, such as a beta peptide [136]
- a cancer antigen, such as those listed in Table 1 of ref. 137 or in tables 3 & 4 of ref. 138.

The composition may comprise one or more of these further antigens.

Toxic protein antigens may be detoxified where necessary (*e.g.* detoxification of pertussis toxin by chemical and/or genetic means [107])

Where a diphtheria antigen is included in the composition it is preferred also to include tetanus antigen and pertussis antigens. Similarly, where a tetanus antigen is included it is preferred also to include diphtheria and pertussis antigens Similarly, where a pertussis antigen is included it is preferred also to include diphtheria and tetanus antigens DTP combinations are thus preferred Sacchande antigens are preferably in the form of conjugates. Carrier proteins for the conjugates are the same as those described above for GBS sacchande conjugation, with CRM197 being preferred.

Antigens in the composition will typically be present at a concentration of at least 1µg/ml each. In general, the concentration of any given antigen will be sufficient to elicit an immune response against that antigen.

As an alternative to using protein antigens in the composition of the invention, nucleic acid encoding the antigen may be used Protein components of the compositions of the invention may thus be replaced by nucleic acid (preferably DNA *e.g.* m the form of a plasmid) that encodes the protein.

### Methods of treating patients

The invention provides polypeptide/saccharide combinations of the invention for use as medicaments The medicament is preferably able to raise an immune response in a mammal (*i.e.* it is an immunogenic composition) and is more preferably a vaccine.

The invention also provides a method of raising an immune response in a patient, comprising administering to a patient a composition of the invention. The immune response is preferably protective against streptococcal disease, and may comprise a humoral immune response and/or a cellular immune response.

The invention also provides the use of polypeptide/saccharide combination of the invention in the manufacture of a medicament for raising an immune response m an patient. The medicament is preferably an immunogenic composition (*e.g*. a vaccine). The medicament is preferably for the prevention and/or treatment of a disease caused by GBS (*e.g*. meningitis, sepsis, chorioamnionitis).

The invention also provides for a kit comprising a first component comprising the immunogenic compositions of the invention. The kit may further include a second component comprising one or more of the following, instructions, syringe or other delivery device, adjuvant, or pharmaceutically acceptable formulating solution

The invention also provides a delivery device pre-filled with the immunogenic compositions of the invention

The invention also provides a method for raising an immune response m a mammal comprising the step of administering an effective amount of a composition of the invention. The immune response is preferably protective and preferably involves antibodies and/or cell-mediated immunity The method may raise a booster response

### Process for manufacturing

The invention provides a process for preparing a composition of the invention, comprising the step of mixing (i) one or more GBS polypeptide antigens with (ii) one or more GBS saccharide antigens

The process may comprise the step of covalently linking the GBS polypeptide to the GBS saccharide m order to form a conjugate

### Definitions

The term "comprising" means "including" as well as "consisting" *e.g.* a composition "comprising" X may consist exclusively of X or may include something additional *e.g*. X + Y.

The term "about" in relation to a numerical value x means, for example, *x*±10%.

The word "substantially" does not exclude "completely" *e.g*. a composition which is "substantially free" from Y may be completely free from Y. Where necessary, the word "substantially" may be omitted from the definition of the invention.

### MODES FOR CARRYING OUT THE INVENTION

GBS serotype III is grown in Todd-Hewitt broth as described in reference 36 and its capsular polysaccharide was purified. The polysacchande is depolymensed, sized and purified as described m reference 14 to give ohgosacchande antigen Similar procedures are used to prepare capsular polysacchandes from other GBS serotypes.

The oligosaccharide is either adnuxed with or covalently conjugated (directly or via a linker) to purified serotype V protein Preferably, the protem comprises a GBS antigen or a fragment thereof selected from the group consisting of GBS 80, GBS 91, GBS 104, GBS 147, GBS 173, GBS 276, GBS 305, GBS 313, GBS 322, GBS 328, GBS 330, GBS 338, GBS 358, GBS 361, GBS 404, GBS 656, GBS 690, and GBS 691.

### REFERENCES

[1] Schuchat (1999) Lancet 353(9146):51-6
[2] Tettelin et al (2002) Proc. Natl Acad. Sci. USA, 10 1073/pnas.182380799.
[3] International patent application WO02/34771.
[4] Kasper (1995) Proc Assoc Am Physicians 107:369-373.
[5] US patent 6,426,074.
[6] European patent application EP-A-0866133.
[7] US patent 5,820,860.
[8] US patent 5,648,241.
[9] US patent 5,834,444
[10] International patent application WO91/04049.
[11] International patent application WO94/10317.
[12] International patent application WO87/06267.
[13] International patent application WO91/04335.
[14] US patent 6,372,222.
[15] International patent application WO96/40795.
[16] WO00/10599.
[17] US patent 5,302,386
[18] International patent application WO94/06467.
[19] US patent 5,993,825
[20] US patent 5,843,461.
[21] US patent 5,795,580.
[22] International patent application WO98/09648.
[23] US patent 6,280,738.
[24] EP-A-0372501
[25] EP-A-0378881
[26] EP-A-0427347
[27] WO93/17712
[28] WO94/03208
[29] WO98/58668
[30] EP-A-0471177
[31] WO00/56360
[32] WO91/01146
[33] WO00/61761
[34] WO01/72337
[35] Research Disclosure, 453077 (Jan 2002)
[36] US patent 4,207,414.
[37] US patent 4,324,887.
[38] US patent 4,367,221.
[39] US patent 4,367,223.
[40] Pincus et al. (1998) J. Immunology 160:293-298
[41] International patent application WO99/54457.
[42] Gennaro (2000) Remington: The Science and Practice of Pharmacy 20th edition, ISBN: 0683306472.
*43.* Vaccine design:the subunit and adjuvant approach (1995) Powell & Newman ISBN 0-306-44867-X.
44. WO00/23105.
45. WO90/14837.
46. WO00/07621.
47 Barr, et al., "ISCOMs and other saponin based adjuvants", Advanced Drug Delivery Reviews (1998) 32:247 - 271. See also Sjolander, et al., "Uptake and adjuvant activity of orally delivered saponin and ISCOM vaccines", Advanced Drug Delivery Reviews (1998) 32 321 - 338.
48 Niikura et al., "Chimenc Recombinant Hepatitis E Virus-Like Particles as an Oral Vaccine Vehicle Presenting Foreign Epitopes", Virology (2002) 293:273 - 280.
49. Lenz et al , "Papillomarivurs-Like Particles Induce Acute Activation of Dendritic Cells", Journal of Immunology (2001) 5246 - 5355.
50. Pmto, et al., "Cellular Immune Responses to Human Papillomavirus (HPV)-16 L1 Healthy Volunteers Immunized with Recombinant HPV-16 L1 Virus-Like Particles", Journal of Infectious Diseases (2003) 188:327 - 338.
51 Gerber et al., "Human Papillomavirsu Virus-Like Particles Are Efficient Oral Immunogens when Coadministered with Escherichia coli Heat-Labile Entertoxin Mutant R192G or CpG", Journal of Virology (2001) 75(10):4752 - 4760
52. Gluck et al., "New Technology Platforms in the Development of Vaccines for the Future", Vaccine (2002) 20:B10-B16.
53. Johnson et al. (1999) Bioorg Med Chem Lett 9:2273-2278.
54. Meraldi et al., "OM-174, a New Adjuvant with a Potential for Human Use, Induces a Protective Response with Administered with the Synthetic C-Terminal Fragment 242-310 from the circumsporozoite protein of Plasmodium berghei", Vaccine (2003) 21-2485 - 2491.
55 Pajak, et al., "The Adjuvant OM-174 induces both the migration and maturation of munne dendritic cells in vivo", Vaccine (2003) 21:836 - 842.
56 Kandimalla, et al., "Divergent synthetic nucleotide motif recognition pattern: design and development of potent immunomodulatory oligodeoxyribonucleotide agents with distinct cytokine induction profiles", Nucleic Acids Research (2003) 31(9): 2393 - 2400.
57 Kneg, "CpG motifs: the active ingredient in bacterial extracts?", Nature Medicine (2003) 9(7). 831- 835.
58. McCluskie, et al., "Parenteral and mucosal pnme-boost immunization strategies in mice with hepatitis B surface antigen and CpG DNA", FEMS Immunology and Medical Microbiology (2002) 32:179- 185.
59. Kandimalla, et al., "Toll-like receptor 9 modulation of recognition and cytokine induction by novel synthetic CpG DNAs", Biochemical Society Transactions (2003) 31 (part 3) 654 - 658 60 Blackwell, et al., "CpG-A-Induced Monocyte IFN-gamma-Inducible Protein-10 Production is Regulated by Plasmacytoid Dendritic Cell Derived IFN-alpha", J. Immunol. (2003) 170(8)-4061 - 4068.
61 Krieg, "From A to Z on CpG", TRENDS in Immunology (2002) 23(2): 64 - 65.
62 Kandimalla, et al., "Secondary structures in CpG oligonucleotides affect immunostimulatory activity", BBRC (2003) 306:948 - 953
63. Kandimalla, et al., "Toll-like receptor 9 modulation of recognition and cytokine induction by novel synthetic GpG DNAs", Biochemical Society Transactions (2003) 31(part 3) 664 - 658.
64. Bhagat et al., "CpG penta- and hexadeoxynbonucleotides as potent immunomodulatory agents" BBRC (2003) 300:853 - 861
65 Beignon, et al., "The LTR72 Mutant of Heat-Labile Enterotoxin of Eschenchia coli Enahnces the Ability of Peptide Antigens to Elicit CD4+ T Cells and Secrete Gamma Interferon after Coapplication onto Bare Skin", Infection and Immunity (2002) 70(6):3012 - 3019
66 Pizza, et al., "Mucosal vaccines non toxic derivatives of LT and CT as mucosal adjuvants", Vaccine (2001) 19:2534-2541.
67 Pizza, et al., "LTK63 and LTR72, two mucosal adjuvants ready for clinical trials" Int. J. Med. Microbiol (2000) 290(4-5) 455-461.
68 Scharton-Kersten et al., "Transcutaneous Immumzation with Bacterial ADP-Ribosylating Exotoxins, Subunits and Unrelated Adjuvants", Infection and Immunity (2000) 68(9):5306 - 5313
69 Ryan et al., "Mutants of Escherichia coli Heat-Labile Toxin Act as Effective Mucosal Adjuvants for Nasal Delivery of an Acellular Pertussis Vaccine: Differential Effects of the Nontoxic AB Complex and Enzyme Activity on Th1 and Th2 Cells" Infection and Immunity (1999) 67(12):6270 - 6280
70 Partidos et al., "Heat-labile enterotoxin of Escherichia coli and its site-directed mutant LTK63 enhance the proliferative and cytotoxic T-cell responses to intranasally co-immunized synthetic peptides", Immunol. Lett. (1999) 67(3):209 - 216.
71 Peppoloni et al., "Mutants of the Eschenchia coli heat-labile enterotoxin as safe and strong adjuvants for intranasal delivery of vaccines", Vaccines (2003) 2(2):285 - 293.
72 Pine et al., (2002) "Intranasal immunization with influenza vaccine and a detoxified mutant of heat labile enterotoxin from Eschenchia coli (LTK63)" J. Control Release (2002) 85(1-3):263 - 270
73 Singh et al. (2001) J. Cont. Rele. 70:267-276
74 WO99/27960.
75 WO99/52549.
76. WO01/21207.
77 WO01/21152.
78. Andnanov et al., "preparation of hydrogel microspheres by coacervation of aqueous polyphophazene solution", Biomaterials (1998) 19(1 - 3):109 -115.
79 Payne et al., "Protein Release from Polyphosphazene Matrices", Adv Drug. Delivery Review (1998) 31(3):185 -196.
80. Stanley, "Imiquimod and the imidazoquinolones: mechanism of action and therapeutic potential" Clin Exp Dermatol (2002) 27(7):571 - 577.
81. Jones, "Resiquimod 3M", Curr Opm Investig Drugs (2003) 4(2):214 - 218.
82 WO99/11241.
83. WO98/57659.
84. European patent applications 0835318, 0735898 and 0761231.
[85] Bakke et al. (2001) Infect. Immun. 69:5010-5015.
[86] Katial et al (2002) Infect. Immun. 70:702-707.
[87] Covacci & Rappuoli (2000) J. Exp. Med. 19:587-592
[88] WO93/18150.
[89] Covacci et al. (1993) Proc Natl. Acad Sci. USA 90:5791-5795
[90] Tummuru et al. (1994) Infect. Immun. 61:1799-1809
[91] Marchetti et al. (1998) Vaccine 16 33-37.
[92] Telford et al. (1994) J. Exp. Med. 179.1653-1658
[93] Evans et al. (1995) Gene 153.123-127
[94] WO96/01272 & WO96/01273, especially SEQ ID NO:6.
[95] WO97/25429.
[96] WO98/04702.
[97] Costantino et al. (1992) Vaccine 10:691-698
[98] Costantmo et al. (1999) Vaccine 17.1251-1263
[99] International patent application PCT/IB02/03191.
[100] Watson (2000) Pediatr Infect Dis J 19 331-332.
[101] Rubin (2000) Pediatr Clin North Am 47.269-285, v.
[102] Jedrzejas (2001) Microbiol Mol Biol Rev 65:187-207.
[103] Bell (2000) Pediatr Infect Dis J 19:1187-1188.
[104] Iwarson (1995) APMIS 103:321-326
[105] Gerlich et al. (1990) Vaccine 8 Suppl S63-68 & 79-80
[106] Gustafsson et al (1996) N. Engl. J. Med 334.349-355
[107] Rappuoh et al. (1991) TIBTECH 9:232-238.
[108] Vaccines (1988) eds. Plotkin & Mortimer. ISBN 0-7216-1946-0
[109] Del Guidice et al. (1998) Molecular Aspects of Medicine 19.1-70.
[110] Hsu et al. (1999) Clin Liver Dis 3-901-915.
[111] International patent application WO99/24578.
[112] International patent application WO99/36544.
[113] International patent application WO99/57280.
[114] International patent application PCT/IB02/02069
[115] International patent application WO02/02606.
[116] Kalman et al (1999) Nature Genetics 21 385-389.
[117] Read et al. (2000) Nucleic Acids Res 28:1397-406.
[118] Shirai et al (2000) J. Infect. Dis. 181(Suppl 3):S524-S527
[119] International patent application WO99/27105.
[120] International patent application WO00/27994.
[121] International patent application WO00/37494.
[122] International patent application WO99/28475.
[123] Ross et al (2001) Vaccine 19:4135-4142.
[124] Sutter et al. (2000) Pediatr Clin North Am 47 287-308
[125] Zimmerman & Spann (1999) Am Fam Physician 59 113-118, 125-126.
[126] Dreesen (1997) Vaccine 15 Suppl:S2-6.
[127] MMWR Morb Mortal Wkly Rep 1998 Jan 16;47(1):12, 19.
[128] Vaccines (1988) eds. Plotkin & Mortimer. ISBN 0-7216-1946-0
[129] McMichael (2000) Vaccine 19 Suppl 1:S101-107.
[130] Dale (1999) Infect Dis Clin North Am 13.227-43, viii.
[131] Ferretti et al. (2001) PNAS USA 98: 4658-4663.
[132] Kuroda et al. (2001) Lancet 357(9264) 1225-1240, see also pages 1218-1219
[133] J Toxicol Clin Toxicol (2001) 39:85-100
[134] Dernicheli et al. (1998) Vaccine 16 880-884.
[135] Stepanov et al. (1996) J Biotechnol 44:155-160.
[136] Ingram (2001) Trends Neurosci 24:305-307.
[137] Rosenberg (2001) Nature 411:380-384.
[138] Moingeon (2001) Vaccine 19:1305-1326.

## Claims

1. An immunogenic composition comprising:
a. one or more GBS polypeptide antigens, and
b. GBS saccharide antigens of GBS serotypes Ia, Ib and III,
wherein the composition comprises GBS80 or an immunogenic fragment thereof.

2. The immunogenic composition of claim 1, wherein said GBS polypeptide antigens further comprise a GBS polypeptide or an immunogenic fragment thereof of serogroup II.

3. The immunogenic composition of claim 1, wherein said GBS polypeptide antigens comprise a combination of two GBS antigens or fragments thereof selected from the group consisting of (1) GBS 80 and GBS 91, (2) GBS 80 and GBS 104, (3) GBS 80 and GBS 147, (4) GBS 80 and GBS 173, (5) GBS 80 and GBS 276, (6) GBS 80 and GBS 305, (7) GBS 80 and GBS 313, (8) GBS 80 and GBS 322, (9) GBS 80 and GBS 328, (10) GBS 80 and GBS 330, (11) GBS 80 and GBS 338, (12) GBS 80 and GBS 358, (13) GBS 80 and GBS 361, (14) GBS 80 and GBS 404, (14) GBS 80 and GBS 404, (15) GBS 80 and GBS 656, (16) GBS 80 and GBS 690, and (17) GBS 80 and GBS 691.

4. The immunogenic composition of claim 3, wherein said combination is selected from the group consisting of (1) GBS 80 and GBS 338; (2) GBS 80 and GBS 361, (3) GBS 80 and GBS 305, (4) GBS 80 and GBS 328, (5) GBS 80 and GBS 690, (6) GBS 80 and GBS 691 and (7) GBS 80 and GBS 147.

5. The immunogenic composition of claim 3, wherein said combination comprises GBS 80 and GBS 691.

6. The immunogenic composition of claim 1, wherein said composition comprises a combination of at least three GBS polypeptide antigens.

7. The immunogenic composition of claim 6, wherein said combination comprises GBS 80 and GBS 691.

8. The immunogenic composition of claim 1, wherein at least one GBS polypeptide antigen is covalently linked to the GBS saccharide antigen.

9. The immunogenic composition of claim 1, wherein said GBS saccharide antigen is covalently linked to a carrier protein.

10. The immunogenic composition of claim 9, wherein said carrier protein is selected from the group consisting of tetanus toxoid, diphtheria toxoid, *N. meningitides* outer membrane protein, heat shock protein, pertusis protein, protein D from *H*. *influenzae,* and toxin A or B from *C. difficile.*

11. The immunogenic composition of claim 10, wherein said carrier protein is selected from the group consisting of tetanus toxoid and diphtheria toxoid.

12. The immunogenic composition of claim 11, wherein said carrier protein is a diphtheria toxoid.

13. The immunogenic composition of claim 12, wherein said diphtheria toxoid is CRM197.

14. The immunogenic composition of claim 1 for use in raising an immune response.

## Patentansprüche

1. Immunogene Zusammensetzung, umfassend:
a. ein oder mehrere GBS-Polypeptid-Antigene und
b. GBS-Saccharid-Antigene der GBS-Serotypen Ia, Ib
und III,
wobei die Zusammensetzung GBS 80 oder ein immunogenes Fragment davon umfasst.

2. Immunogene Zusammensetzung nach Anspruch 1, wobei die GBS-Polypeptid-Antigene ferner ein GBS-Polypeptid oder ein immunogenes Fragment davon der Serogruppe II umfassen.

3. Immunogene Zusammensetzung nach Anspruch 1, wobei die GBS-Polypeptid-Antigene eine Kombination von zwei GBS-Antigenen oder Fragmenten davon umfassen, die aus der aus (1) GBS 80 und GBS 91, (2) GBS 80 und GBS 104, (3) GBS 80 und GBS 147, (4) GBS 80 und GBS 173, (5) GBS 80 und GBS 276, (6) GBS 80 und GBS 305, (7) GBS 80 und GBS 313, (8) GBS 80 und GBS 322, (9) GBS 80 und GBS 328, (10) GBS 80 und GBS 330, (11) GBS 80 und GBS 338, (12) GBS 80 und GBS 358, (13) GBS 80 und GBS 361, (14) GBS 80 und GBS 404, (14) GBS 80 und GBS 404, (15) GBS 80 und GBS 656, (16) GBS 80 und GBS 690 und (17) GBS 80 und GBS 691 bestehenden Gruppe ausgewählt ist.

4. Immunogene Zusammensetzung nach Anspruch 3, wobei die Kombination aus der aus (1) GBS 80 und GBS 338; (2) GBS 80 und GBS 361, (3) GBS 80 und GBS 305, (4) GBS 80 und GBS 328, (5) GBS 80 und GBS 690, (6) GBS 80 und GBS 691 und (7) GBS 80 und GBS 147 bestehenden Gruppe ausgewählt ist.

5. Immunogene Zusammensetzung nach Anspruch 3, wobei die Kombination GBS 80 und GBS 691 umfasst.

6. Immunogene Zusammensetzung nach Anspruch 1, wobei die Kombination eine Kombination von wenigstens drei GBS-Polypeptid-Antigenen umfasst.

7. Immunogene Zusammensetzung nach Anspruch 6, wobei die Kombination GBS 80 und GBS 691 umfasst.

8. Immunogene Zusammensetzung nach Anspruch 1, wobei wenigstens ein GBS-Polypeptid-Antigen kovalent mit dem GBS-Saccharid-Antigen verknüpft ist.

9. Immunogene Zusammensetzung nach Anspruch 1, wobei das GBS-Saccharid-Antigen kovalent mit einem Trägerprotein verknüpft ist.

10. Immunogene Zusammensetzung nach Anspruch 9, wobei das Trägerprotein aus der aus Tetanustoxoid, Diphtherietoxoid, N. meningitides-Außenmembranprotein, Hitzeschockprotein, Pertussis-Protein, Protein D aus *H. influenzae* und Toxin A oder B aus *C*. *difficile* bestehenden Gruppe ausgewählt ist.

11. Immunogene Zusammensetzung nach Anspruch 10, wobei das Trägerprotein aus der aus Tetanustoxoid und Diphtherietoxoid bestehenden Gruppe ausgewählt ist.

12. Immunogene Zusammensetzung nach Anspruch 11, wobei es sich bei dem Trägerprotein um ein Diphtherietoxoid handelt.

13. Immunogene Zusammensetzung nach Anspruch 12, wobei es sich bei dem Diphtherietoxoid um CRM197 handelt.

14. Immunogene Zusammensetzung nach Anspruch 1 zur Verwendung beim Hervorrufen einer Immunantwort.

## Revendications

1. Composition immunogène, comprenant :
a. un ou plusieurs antigènes polypeptidiques GBS, et
b. des antigènes saccharidiques GBS de sérotypes GBS Ia, Ib et III,
**caractérisée en ce que** la composition comprend du GBS 80 ou un fragment immunogène de celui-ci.

2. Composition immunogène selon la revendication 1, **caractérisée en ce que** lesdits antigènes polypeptidiques GBS comprennent en outre un polypeptide GBS ou un fragment de celui-ci de sérogroupe II.

3. Composition immunogène selon la revendication 1, **caractérisée en ce que** lesdits antigènes polypeptidiques GBS comprennent une combinaison de deux antigènes GBS ou de fragments de ceux-ci, choisis dans le groupe constitué par (1) GBS 80 et GBS 91, (2) GBS 80 et GBS 104, (3) GBS 80 et GBS 147, (4) GBS 80 et GBS 173, (5) GBS 80 et GBS 276, (6) GBS 80 et GBS 305, (7) GBS 80 et GBS 313, (8) GBS 80 et GBS 322, (9) GBS 80 et GBS 328, (10) GBS 80 et GBS 330, (11) GBS 80 et GBS 338, (12) GBS 80 et GBS 358, (13) GBS 80 et GBS 361, (14) GBS 80 et GBS 404, (14) GBS 80 et GBS 404, (15) GBS 80 et GBS 656, (16) GBS 80 et GBS 690, et (17) GBS 80 et GBS 691.

4. Composition immunogène selon la revendication 3, **caractérisée en ce que** ladite combinaison est choisie dans le groupe constitué par (1) GBS 80 et GBS 338, (2) GBS 80 et GBS 361, (3) GBS 80 et GBS 305, (4) GBS 80 et GBS 328, (5) GBS 80 et GBS 690, (6) GBS 80 et GBS 691, et (7) GBS 80 et GBS 147.

5. Composition immunogène selon la revendication 3, **caractérisée en ce que** ladite combinaison comprend GBS 80 et GBS 691.

6. Composition immunogène selon la revendication 1, **caractérisée en ce que** ladite composition comprend une combinaison d'au moins trois antigènes polypeptidiques GBS.

7. Composition immunogène selon la revendication 6, **caractérisée en ce que** ladite combinaison comprend GBS 80 et GBS 691.

8. Composition immunogène selon la revendication 1, **caractérisée en ce qu'**au moins un antigène polypeptidique GBS est lié de manière covalente à l'antigène saccharidique GBS.

9. Composition immunogène selon la revendication 1, **caractérisée en ce que** ledit antigène saccharidique GBS est lié de manière covalente à une protéine véhicule.

10. Composition immunogène selon la revendication 9, **caractérisée en ce que** ladite protéine véhicule est choisie dans le groupe constitué par le toxoïde du tétanos, le toxoïde de la diphtérie, la protéine de membrane externe de *N*. *meningitidis,* une protéine de chou thermique, une protéine de la coqueluche, la protéine D de *H*. *influenzae,* et la toxine A ou B de *C*. *difficile.*

11. Composition immunogène selon la revendication 10, **caractérisée en ce que** ladite protéine véhicule est choisie dans le groupe constitué par le toxoïde du tétanos et le toxoïde de la diphtérie.

12. Composition immunogène selon la revendication 11, **caractérisée en ce que** ladite protéine véhicule est un toxoïde de la diphtérie.

13. Composition immunogène selon la revendication 12, **caractérisée en ce que** ledit toxoïde de la diphtérie est CRM197.

14. Composition immunogène selon la revendication 1, pour une utilisation dans le déclenchement d'une réponse immunitaire.


### SEQUENCE LISTING

<110> Chiron Corporation
<120> Group B Streptococcus Vaccine
<130> 002441.00084
<140> PCT/US03/29167
   <141> 2003-09-15
<160> 42
<170> PatentIn version 3.1
<210> 1
   <211> 1662
   <212> DNA
   <213> group B streptococcus
<400> 1
   atgaaattat cgaagaagtt attgttttcg gctgctgttt taacaatggt ggcggggtca     60
   actgttgaac cagtagctca gtttgcgact ggaatgagta ttgtaagagc tgcagaagtg    120
   tcacaagaac gcccagcgaa aacaacagta aatatctata aattacaagc tgatagttat    180
   aaatcggaaa ttacttctaa tggtggtatc gagaataaag acggcgaagt aatatctaac    240
   tatgctaaac ttggtgacaa tgtaaaaggt ttgcaaggtg tacagtttaa acgttataaa    300
   gtcaagacgg atatttctgt tgatgaattg aaaaaattga caacagttga agcagcagat    360
   gcaaaagttg gaacgattct tgaagaaggt gtcagtctac ctcaaaaaac taatgctcaa    420
   ggtttggtcg tcgatgctct ggattcaaaa agtaatgtga gatacttgta tgtagaagat    480
   ttaaagaatt caccttcaaa cattaccaaa gcttatgctg taccgtttgt gttggaatta    540
   ccagttgcta actctacagg tacaggtttc ctttctgaaa ttaatattta ccctaaaaac    600
   gttgtaactg atgaaccaaa aacagataaa gatgttaaaa aattaggtca ggacgatgca    660
   ggttatacga ttggtgaaga attcaaatgg ttcttgaaat ctacaatccc tgccaattta    720
   ggtgactatg aaaaatttga aattactgat aaatttgcag atggcttgac ttataaatct    780
   gttggaaaaa tcaagattgg ttcgaaaaca ctgaatagag atgagcacta cactattgat    840
   gaaccaacag ttgataacca aaatacatta aaaattacgt ttaaaccaga gaaatttaaa    900
   gaaattgctg agctacttaa aggaatgacc cttgttaaaa atcaagatgc tcttgataaa    960
   gctactgcaa atacagatga tgcggcattt ttggaaattc cagttgcatc aactattaat   1020
   gaaaaagcag ttttaggaaa agcaattgaa aatacttttg aacttcaata tgaccatact   1080
   cctgataaag ctgacaatcc aaaaccatct aatcctccaa gaaaaccaga agttcatact   1140
   ggtgggaaac gatttgtaaa gaaagactca acagaaacac aaacactagg tggtgctgag   1200
   tttgatttgt tggcttctga tgggacagca gtaaaatgga cagatgctct tattaaagcg   1260
   aatactaata aaaactatat tgctggagaa gctgttactg ggcaaccaat caaattgaaa   1320
   tcacatacag acggtacgtt tgagattaaa ggtttggctt atgcagttga tgcgaatgca   1380
   gagggtacag cagtaactta caaattaaaa gaaacaaaag caccagaagg ttatgtaatc   1440
   cctgataaag aaatcgagtt tacagtatca caaacatctt ataatacaaa accaactgac   1500
   atcacggttg atagtgctga tgcaacacct gatacaatta aaaacaacaa acgtccttca   1560
   atccctaata ctggtggtat tggtacggct atctttgtcg ctatcggtgc tgcggtgatg   1620
   gcttttgctg ttaaggggat gaagcgtcgt acaaaagata ac                       1662
<210> 2
   <211> 554
   <212> PRT
   <213> group B streptococcus
<220>
   <221> MISC_FEATURE
   <223> Strain 80
<400> 2
   Met Lys Leu Ser Lys Lys Leu Leu Phe Ser Ala Ala Val Leu Thr Met
   1               5                   10                  15
   Val Ala Gly Ser Thr Val Glu Pro Val Ala Gln Phe Ala Thr Gly Met
               20                  25                  30
   Ser Ile Val Arg Ala Ala Glu Val Ser Gln Glu Arg Pro Ala Lys Thr
           35                  40                  45
   Thr Val Asn Ile Tyr Lys Leu Gln Ala Asp Ser Tyr Lys Ser Glu Ile
       50                  55                  60
   Thr Ser Asn Gly Gly Ile Glu Asn Lys Asp Gly Glu Val Ile Ser Asn
   65                  70                  75                  80
   Tyr Ala Lys Leu Gly Asp Asn Val Lys Gly Leu Gln Gly Val Gln Phe
                   85                  90                  95
   Lys Arg Tyr Lys Val Lys Thr Asp Ile Ser Val Asp Glu Leu Lys Lys
               100                 105                 110
   Leu Thr Thr Val Glu Ala Ala Asp Ala Lys Val Gly Thr Ile Leu Glu
           115                 120                 125
   Glu Gly Val Ser Leu Pro Gln Lys Thr Asn Ala Gln Gly Leu Val Val
   130 135 140
   Asp Ala Leu Asp Ser Lys Ser Asn Val Arg Tyr Leu Tyr Val Glu Asp
   145 150 155 160
   Leu Lys Asn Ser Pro Ser Asn Ile Thr Lys Ala Tyr Ala Val Pro Phe
   165 170 175
   Val Leu Glu Leu Pro Val Ala Asn Ser Thr Gly Thr Gly Phe Leu Ser
   180 185 190
   Glu Ile Asn Ile Tyr Pro Lys Asn Val Val Thr Asp Glu Pro Lys Thr
   195 200 205
   Asp Lys Asp Val Lys Lys Leu Gly Gln Asp Asp Ala Gly Tyr Thr Ile
   210 215 220
   Gly Glu Glu Phe Lys Trp Phe Leu Lys Ser Thr Ile Pro Ala Asn Leu
   225 230 235 240
   Gly Asp Tyr Glu Lys Phe Glu Ile Thr Asp Lys Phe Ala Asp Gly Leu
   245 250 255
   Thr Tyr Lys Ser Val Gly Lys Ile Lys Ile Gly Ser Lys Thr Leu Asn
   260 265 270
   Arg Asp Glu His Tyr Thr Ile Asp Glu Pro Thr Val Asp Asn Gln Asn
   275 280 285
   Thr Leu Lys Ile Thr Phe Lys Pro Glu Lys Phe Lys Glu Ile Ala Glu
   290 295 300
   Leu Leu Lys Gly Met Thr Leu Val Lys Asn Gln Asp Ala Leu Asp Lys
   305 310 315 320
   Ala Thr Ala Asn Thr Asp Asp Ala Ala Phe Leu Glu Ile Pro Val Ala
   325 330 335
   Ser Thr Ile Asn Glu Lys Ala Val Leu Gly Lys Ala Ile Glu Asn Thr
   340 345 350
   Phe Glu Leu Gln Tyr Asp His Thr Pro Asp Lys Ala Asp Asn Pro Lys
   355 360 365
   Pro Ser Asn Pro Pro Arg Lys Pro Glu Val His Thr Gly Gly Lys Arg
   370 375 380
   Phe Val Lys Lys Asp Ser Thr Glu Thr Gln Thr Leu Gly Gly Ala Glu
   385 390 395 400
   Phe Asp Leu Leu Ala Ser Asp Gly Thr Ala Val Lys Trp Thr Asp Ala
   405 410 415
   Leu Ile Lys Ala Asn Thr Asn Lys Asn Tyr Ile Ala Gly Glu Ala Val
   420 425 430
   Thr Gly Gln Pro Ile Lys Leu Lys Ser His Thr Asp Gly Thr Phe Glu
   435 440 445
   Ile Lys Gly Leu Ala Tyr Ala Val Asp Ala Asn Ala Glu Gly Thr Ala
   450 455 460
   Val Thr Tyr Lys Leu Lys Glu Thr Lys Ala Pro Glu Gly Tyr Val Ile
   465 470 475 480
   Pro Asp Lys Glu Ile Glu Phe Thr Val Ser Gln Thr Ser Tyr Asn Thr
   485 490 495
   Lys Pro Thr Asp Ile Thr Val Asp Ser Ala Asp Ala Thr Pro Asp Thr
   500 505 510
   Ile Lys Asn Asn Lys Arg Pro Ser Ile Pro Asn Thr Gly Gly Ile Gly
   515 520 525
   Thr Ala Ile Phe Val Ala Ile Gly Ala Ala Val Met Ala Phe Ala Val
   530 535 540
   Lys Gly Met Lys Arg Arg Thr Lys Asp Asn
   545 550
<210> 3
   <211> 1629
   <212> DNA
   <213> group B streptococcus
<400> 3
   atgaaaaaag gacaagtaaa tgatactaag caatcttact ctctacgtaa atataaattt      60
   ggtttagcat cagtaatttt agggtcattc ataatggtca caagtcctgt ttttgcggat     120
   caaactacat cggttcaagt taataatcag acaggcacta gtgtggatgc taataattct     180
   tccaatgaga caagtgcgtc aagtgtgatt acttccaata atgatagtgt tcaagcgtct     240
   gataaagttg taaatagtca aaatacggca acaaaggaca ttactactcc tttagtagag     300
   acaaagccaa tggtggaaaa aacattacct gaacaaggga attatgttta tagcaaagaa     360
   accgaggtga aaaatacacc ttcaaaatca gccccagtag ctttctatgc aaagaaaggt     420
   gataaagttt tctatgacca agtatttaat aaagataatg tgaaatggat ttcatataag     480
   tctttttgtg gcgtacgtcg atacgcagct attgagtcac tagatccatc aggaggttca     540
   gagactaaag cacctactcc tgtaacaaat tcaggaagca ataatcaaga gaaaatagca     600
   acgcaaggaa attatacatt ttcacataaa gtagaagtaa aaaatgaagc taaggtagcg     660
   agtccaactc aatttacatt ggacaaagga gacagaattt tttacgacca aatactaact     720
   attgaaggaa atcagtggtt atcttataaa tcattcaatg gtgttcgtcg ttttgttttg     780
   ctaggtaaag catcttcagt agaaaaaact gaagataaag aaaaagtgtc tcctcaacca     840
   caagcccgta ttactaaaac tggtagactg actatttcta acgaaacaac tacaggtttt     900
   gatattttaa ttacgaatat taaagatgat aacggtatcg ctgctgttaa ggtaccggtt     960
   tggactgaac aaggagggca agatgatatt aaatggtata cagctgtaac tactggggat    1020
   ggcaactaca aagtagctgt atcatttgct gaccataaga atgagaaggg tctttataat    1080
   attcatttat actaccaaga agctagtggg acacttgtag gtgtaacagg aactaaagtg    1140
   acagtagctg gaactaattc ttctcaagaa cctattgaaa atggtttagc aaagactggt    1200
   gtttataata ttatcggaag tactgaagta aaaaatgaag ctaaaatatc aagtcagacc    1260
   caatttactt tagaaaaagg tgacaaaata aattatgatc aagtattgac agcagatggt    1320
   taccagtgga tttcttacaa atcttatagt ggtgttcgtc gctatattcc tgtgaaaaag    1380
   ctaactacaa gtagtgaaaa agcgaaagat gaggcgacta aaccgactag ttatcccaac    1440
   ttacctaaaa caggtaccta tacatttact aaaactgtag atgtgaaaag tcaacctaaa    1500
   gtatcaagtc cagtggaatt taattttcaa aagggtgaaa aaatacatta tgatcaagtg    1560
   ttagtagtag atggtcatca gtggatttca tacaagagtt attccggtat tcgtcgctat    1620
   attgaaatt                                                            1629
<210> 4
   <211> 543
   <212> PRT
   <213> group B streptococcus
<400> 4
   Met Lys Lys Gly Gln Val Asn Asp Thr Lys Gln Ser Tyr Ser Leu Arg
   1               5                   10                  15
   Lys Tyr Lys Phe Gly Leu Ala Ser Val Ile Leu Gly Ser Phe Ile Met
               20                  25                  30
   Val Thr Ser Pro Val Phe Ala Asp Gln Thr Thr Ser Val Gln Val Asn
           35                  40                  45
   Asn Gln Thr Gly Thr Ser Val Asp Ala Asn Asn Ser Ser Asn Glu Thr
       50                  55                  60
   Ser Ala Ser Ser Val Ile Thr Ser Asn Asn Asp Ser Val Gln Ala Ser
   65                  70                  75                  80
   Asp Lys Val Val Asn Ser Gln Asn Thr Ala Thr Lys Asp Ile Thr Thr
                   85                  90                  95
   Pro Leu Val Glu Thr Lys Pro Met Val Glu Lys Thr Leu Pro Glu Gln
               100                 105                 110
   Gly Asn Tyr Val Tyr Ser Lys Glu Thr Glu Val Lys Asn Thr Pro Ser
           115                 120                 125
   Lys Ser Ala Pro Val Ala Phe Tyr Ala Lys Lys Gly Asp Lys Val Phe
       130                 135                 140
   Tyr Asp Gln Val Phe Asn Lys Asp Asn Val Lys Trp Ile Ser Tyr Lys
   145                 150                 155                 160
   Ser Phe Cys Gly Val Arg Arg Tyr Ala Ala Ile Glu Ser Leu Asp Pro
                   165                 170                 175
   Ser Gly Gly Ser Glu Thr Lys Ala Pro Thr Pro Val Thr Asn Ser Gly
               180                 185                 190
   Ser Asn Asn Gln Glu Lys Ile Ala Thr Gln Gly Asn Tyr Thr Phe Ser
           195                 200                 205
   His Lys Val Glu Val Lys Asn Glu Ala Lys Val Ala Ser Pro Thr Gln
       210                 215                 220
   Phe Thr Leu Asp Lys Gly Asp Arg Ile Phe Tyr Asp Gln Ile Leu Thr
   225                 230                 235                 240
   Ile Glu Gly Asn Gln Trp Leu Ser Tyr Lys Ser Phe Asn Gly Val Arg
                   245                 250                 255
   Arg Phe Val Leu Leu Gly Lys Ala Ser Ser Val Glu Lys Thr Glu Asp
               260                 265                 270
   Lys Glu Lys Val Ser Pro Gln Pro Gln Ala Arg Ile Thr Lys Thr Gly
           275                 280                 285
   Arg Leu Thr Ile Ser Asn Glu Thr Thr Thr Gly Phe Asp Ile Leu Ile
       290                 295                 300
   Thr Asn Ile Lys Asp Asp Asn Gly Ile Ala Ala Val Lys Val Pro Val
   305                 310                 315                     320
   Trp Thr Glu Gln Gly Gly Gln Asp Asp Ile Lys Trp Tyr Thr Ala Val
                   325                 330                 335
   Thr Thr Gly Asp Gly Asn Tyr Lys Val Ala Val Ser Phe Ala Asp His
               340                 345                 350
   Lys Asn Glu Lys Gly Leu Tyr Asn Ile His Leu Tyr Tyr Gln Glu Ala
           355                 360                 365
   Ser Gly Thr Leu Val Gly Val Thr Gly Thr Lys Val Thr Val Ala Gly
       370                 375                 380
   Thr Asn Ser Ser Gln Glu Pro Ile Glu Asn Gly Leu Ala Lys Thr Gly
   385                 390                 395                     400
   Val Tyr Asn Ile Ile Gly Ser Thr Glu Val Lys Asn Glu Ala Lys Ile
                   405                 410                 415
   Ser Ser Gln Thr Gln Phe Thr Leu Glu Lys Gly Asp Lys Ile Asn Tyr
               420                 425                 430
   Asp Gln Val Leu Thr Ala Asp Gly Tyr Gln Trp Ile Ser Tyr Lys Ser
           435                 440                 445
   Tyr Ser Gly Val Arg Arg Tyr Ile Pro Val Lys Lys Leu Thr Thr Ser
       450                 455                 460
   Ser Glu Lys Ala Lys Asp Glu Ala Thr Lys Pro Thr Ser Tyr Pro Asn
   465                 470                 475                     480
   Leu Pro Lys Thr Gly Thr Tyr Thr Phe Thr Lys Thr Val Asp Val Lys
                   485                 490                 495
   Ser Gln Pro Lys Val Ser Ser Pro Val Glu Phe Asn Phe Gln Lys Gly
               500                 505                 510
   Glu Lys Ile His Tyr Asp Gln Val Leu Val Val Asp Gly His Gln Trp
           515                 520                 525
   Ile Ser Tyr Lys Ser Tyr Ser Gly Ile Arg Arg Tyr Ile Glu Ile
       530                 535                 540
<210> 5
   <211> 2670
   <212> DNA
   <213> group B streptococcus
<400> 5
   atgaaaaaga gacaaaaaat atggagaggg ttatcagtta ctttactaat cctgtcccaa    60
   attccatttg gtatattggt acaaggtgaa acccaagata ccaatcaagc acttggaaaa   120
   gtaattgtta aaaaaacggg agacaatgct acaccattag gcaaagcgac ttttgtgtta   180
   aaaaatgaca atgataagtc agaaacaagt cacgaaacgg tagagggttc tggagaagca   240
   acctttgaaa acataaaacc tggagactac acattaagag aagaaacagc accaattggt   300
   tataaaaaaa ctgataaaac ctggaaagtt aaagttgcag ataacggagc aacaataatc   360
   gagggtatgg atgcagataa agcagagaaa cgaaaagaag ttttgaatgc ccaatatcca   420
   aaatcagcta tttatgagga tacaaaagaa aattacccat tagttaatgt agagggttcc   480
   aaagttggtg aacaatacaa agcattgaat ccaataaatg gaaaagatgg tcgaagagag   540
   attgctgaag gttggttatc aaaaaaaatt acaggggtca atgatctcga taagaataaa   600
   tataaaattg aattaactgt tgagggtaaa accactgttg aaacgaaaga acttaatcaa   660
   ccactagatg tcgttgtgct attagataat tcaaatagta tgaataatga aagagccaat   720
   aattctcaaa gagcattaaa agctggggaa gcagttgaaa agctgattga taaaattaca   780
   tcaaataaag acaatagagt agctcttgtg acatatgcct caaccatttt tgatggtact   840
   gaagcgaccg tatcaaaggg agttgccgat caaaatggta aagcgctgaa tgatagtgta   900
   tcatgggatt atcataaaac tacttttaca gcaactacac ataattacag ttatttaaat   960
   ttaacaaatg atgctaacga agttaatatt ctaaagtcaa gaattccaaa ggaagcggag  1020
   catataaatg ggatcgcacg gctctatcaa tttggtgcga catttactca aaaagctcta  1080
   atgaaagcaa atgaaatttt agagacacaa agttctaatg ctagaaaaaa acttattttt  1140
   cacgtaactg atggtgtccc tacgatgtct tatgccataa attttaatcc ttatatatca  1200
   acatcttacc aaaaccagtt taattctttt ttaaataaaa taccagatag aagtggtatt  1260
   ctccaagagg attttataat caatggtgat gattatcaaa tagtaaaagg agatggagag  1320
   agttttaaac tgttttcgga tagaaaagtt cctgttactg gaggaacgac acaagcagct  1380
   tatcgagtac cgcaaaatca actctctgta atgagtaatg agggatatgc aattaatagt    1440
   ggatatattt atctctattg gagagattac aactgggtct atccatttga tcctaagaca    1500
   aagaaagttt ctgcaacgaa acaaatcaaa actcatggtg agccaacaac attatacttt    1560
   aatggaaata taagacctaa aggttatgac atttttactg ttgggattgg tgtaaacgga    1620
   gatcctggtg caactcctct tgaagctgag aaatttatgc aatcaatatc aagtaaaaca    1680
   gaaaattata ctaatgttga tgatacaaat aaaatttatg atgagctaaa taaatacttt    1740
   aaaacaattg ttgaggaaaa acattctatt gttgatggaa atgtgactga tcctatggga    1800
   gagatgattg aattccaatt aaaaaatggt caaagtttta cacatgatga ttacgttttg    1860
   gttggaaatg atggcagtca attaaaaaat ggtgtggctc ttggtggacc aaacagtgat    1920
   gggggaattt taaaagatgt tacagtgact tatgataaga catctcaaac catcaaaatc    1980
   aatcatttga acttaggaag tggacaaaaa gtagttctta cctatgatgt acgtttaaaa    2040
   gataactata taagtaacaa attttacaat acaaataatc gtacaacgct aagtccgaag    2100
   agtgaaaaag aaccaaatac tattcgtgat ttcccaattc ccaaaattcg tgatgttcgt    2160
   gagtttccgg tactaaccat cagtaatcag aagaaaatgg gtgaggttga atttattaaa    2220
   gttaataaag acaaacattc agaatcgctt ttgggagcta agtttcaact tcagatagaa    2280
   aaagattttt ctgggtataa gcaatttgtt ccagagggaa gtgatgttac aacaaagaat    2340
   gatggtaaaa tttattttaa agcacttcaa gatggtaact ataaattata tgaaatttca    2400
   agtccagatg gctatataga ggttaaaacg aaacctgttg tgacatttac aattcaaaat    2460
   ggagaagtta cgaacctgaa agcagatcca aatgctaata aaaatcaaat cgggtatctt    2520
   gaaggaaatg gtaaacatct tattaccaac actcccaaac gcccaccagg tgtttttcct    2580
   aaaacagggg gaattggtac aattgtctat atattagttg gttctacttt tatgatactt    2640
   accatttgtt ctttccgtcg taaacaattg                                      2670
<210> 6
   <211> 890
   <212> PRT
   <213> group B streptococcus
<400> 6
   Met Lys Lys Arg Gln Lys Ile Trp Arg Gly Leu Ser Val Thr Leu Leu
   1               5                   10                  15
   Ile Leu Ser Gln Ile Pro Phe Gly Ile Leu Val Gln Gly Glu Thr Gln
               20                  25                  30
   Asp Thr Asn Gln Ala Leu Gly Lys Val Ile Val Lys Lys Thr Gly Asp
   35 40 45
   Asn Ala Thr Pro Leu Gly Lys Ala Thr Phe Val Leu Lys Asn Asp Asn
   50 55 60
   Asp Lys Ser Glu Thr Ser His Glu Thr Val Glu Gly Ser Gly Glu Ala
   65 70 75 80
   Thr Phe Glu Asn Ile Lys Pro Gly Asp Tyr Thr Leu Arg Glu Glu Thr
   85 90 95
   Ala Pro Ile Gly Tyr Lys Lys Thr Asp Lys Thr Trp Lys Val Lys Val
   100 105 110
   Ala Asp Asn Gly Ala Thr Ile Ile Glu Gly Met Asp Ala Asp Lys Ala
   115 120 125
   Glu Lys Arg Lys Glu Val Leu Asn Ala Gln Tyr Pro Lys Ser Ala Ile
   130 135 140
   Tyr Glu Asp Thr Lys Glu Asn Tyr Pro Leu Val Asn Val Glu Gly Ser
   145 150 155 160
   Lys Val Gly Glu Gln Tyr Lys Ala Leu Asn Pro Ile Asn Gly Lys Asp
   165 170 175
   Gly Arg Arg Glu Ile Ala Glu Gly Trp Leu Ser Lys Lys Ile Thr Gly
   180 185 190
   Val Asn Asp Leu Asp Lys Asn Lys Tyr Lys Ile Glu Leu Thr Val Glu
   195 200 205
   Gly Lys Thr Thr Val Glu Thr Lys Glu Leu Asn Gln Pro Leu Asp Val
   210 215 220
   Val Val Leu Leu Asp Asn Ser Asn Ser Met Asn Asn Glu Arg Ala Asn
   225 230 235 240
   Asn Ser Gln Arg Ala Leu Lys Ala Gly Glu Ala Val Glu Lys Leu Ile
   245 250 255
   Asp Lys Ile Thr Ser Asn Lys Asp Asn Arg Val Ala Leu Val Thr Tyr
   260 265 270
   Ala Ser Thr Ile Phe Asp Gly Thr Glu Ala Thr Val Ser Lys Gly Val
   275 280 285
   Ala Asp Gln Asn Gly Lys Ala Leu Asn Asp Ser Val Ser Trp Asp Tyr
       290                 295                 300
   His Lys Thr Thr Phe Thr Ala Thr Thr His Asn Tyr Ser Tyr Leu Asn
   305                 310                 315                 320
   Leu Thr Asn Asp Ala Asn Glu Val Asn Ile Leu Lys Ser Arg Ile Pro
                   325                 330                 335
   Lys Glu Ala Glu His Ile Asn Gly Asp Arg Thr Leu Tyr Gln Phe Gly
               340                 345                 350
   Ala Thr Phe Thr Gln Lys Ala Leu Met Lys Ala Asn Glu Ile Leu Glu
           355                 360                 365
   Thr Gln Ser Ser Asn Ala Arg Lys Lys Leu Ile Phe His Val Thr Asp
       370                 375                 380
   Gly Val Pro Thr Met Ser Tyr Ala Ile Asn Phe Asn Pro Tyr Ile Ser
   385                 390                 395                 400
   Thr Ser Tyr Gln Asn Gln Phe Asn Ser Phe Leu Asn Lys Ile Pro Asp
                   405                 410                 415
   Arg Ser Gly Ile Leu Gln Glu Asp Phe Ile Ile Asn Gly Asp Asp Tyr
               420                 425                 430
   Gln Ile Val Lys Gly Asp Gly Glu Ser Phe Lys Leu Phe Ser Asp Arg
           435                 440                 445
   Lys Val Pro Val Thr Gly Gly Thr Thr Gln Ala Ala Tyr Arg Val Pro
       450                 455                 460
   Gln Asn Gln Leu Ser Val Met Ser Asn Glu Gly Tyr Ala Ile Asn Ser
   465                 470                 475                 480
   Gly Tyr Ile Tyr Leu Tyr Trp Arg Asp Tyr Asn Trp Val Tyr Pro Phe
                   485                 490                 495
   Asp Pro Lys Thr Lys Lys Val Ser Ala Thr Lys Gln Ile Lys Thr His
               500                 505                 510
   Gly Glu Pro Thr Thr Leu Tyr Phe Asn Gly Asn Ile Arg Pro Lys Gly
           515                 520                 525
   Tyr Asp Ile Phe Thr Val Gly Ile Gly Val Asn Gly Asp Pro Gly Ala
       530                 535                 540
   Thr Pro Leu Glu Ala Glu Lys Phe Met Gln Ser Ile Ser Ser Lys Thr
   545                 550                 555                 560
   Glu Asn Tyr Thr Asn Val Asp Asp Thr Asn Lys Ile Tyr Asp Glu Leu
                   565                 570                 575
   Asn Lys Tyr Phe Lys Thr Ile Val Glu Glu Lys His Ser Ile Val Asp
               580                 585                 590
   Gly Asn Val Thr Asp Pro Met Gly Glu Met Ile Glu Phe Gln Leu Lys
           595                 600                 605
   Asn Gly Gln Ser Phe Thr His Asp Asp Tyr Val Leu Val Gly Asn Asp
       610                 615                 620
   Gly Ser Gln Leu Lys Asn Gly Val Ala Leu Gly Gly Pro Asn Ser Asp
   625                 630                 635                 640
   Gly Gly Ile Leu Lys Asp Val Thr Val Thr Tyr Asp Lys Thr Ser Gln
                   645                 650                 655
   Thr Ile Lys Ile Asn His Leu Asn Leu Gly Ser Gly Gln Lys Val Val
               660                 665                 670
   Leu Thr Tyr Asp Val Arg Leu Lys Asp Asn Tyr Ile Ser Asn Lys Phe
           675                 680                 685
   Tyr Asn Thr Asn Asn Arg Thr Thr Leu Ser Pro Lys Ser Glu Lys Glu
       690                 695                 700
   Pro Asn Thr Ile Arg Asp Phe Pro Ile Pro Lys Ile Arg Asp Val Arg
   705                 710                 715                 720
   Glu Phe Pro Val Leu Thr Ile Ser Asn Gln Lys Lys Met Gly Glu Val
                   725                 730                 735
   Glu Phe Ile Lys Val Asn Lys Asp Lys His Ser Glu Ser Leu Leu Gly
               740                 745                 750
   Ala Lys Phe Gln Leu Gln Ile Glu Lys Asp Phe Ser Gly Tyr Lys Gln
           755                 760                 765
   Phe Val Pro Glu Gly Ser Asp Val Thr Thr Lys Asn Asp Gly Lys Ile
       770                 775                 780
   Tyr Phe Lys Ala Leu Gln Asp Gly Asn Tyr Lys Leu Tyr Glu Ile Ser
   785                 790                 795                 800
   Ser Pro Asp Gly Tyr Ile Glu Val Lys Thr Lys Pro Val Val Thr Phe
                   805                 810                 815
   Thr Ile Gln Asn Gly Glu Val Thr Asn Leu Lys Ala Asp Pro Asn Ala
               820                 825                 830
   Asn Lys Asn Gln Ile Gly Tyr Leu Glu Gly Asn Gly Lys His Leu Ile
           835                 840                 845
   Thr Asn Thr Pro Lys Arg Pro Pro Gly Val Phe Pro Lys Thr Gly Gly
       850                 855                 860
   Ile Gly Thr Ile Val Tyr Ile Leu Val Gly Ser Thr Phe Met Ile Leu
   865                 870                 875                 880
   Thr Ile Cys Ser Phe Arg Arg Lys Gln Leu
                   885                 890
<210> 7
   <211> 3699
   <212> DNA
   <213> group B streptococcus
<400> 7
   gtggataaac atcactcaaa aaaggctatt ttaaagttaa cacttataac aactagtatt     60
   ttattaatgc atagcaatca agtgaatgca gaggagcaag aattaaaaaa ccaagagcaa    120
   tcacctgtaa ttgctaatgt tgctcaacag ccatcgccat cggtaactac taatactgtt    180
   gaaaaaacat ctgtaacagc tgcttctgct agtaatacag cgaaagaaat gggtgataca    240
   tctgtaaaaa atgacaaaac agaagatgaa ttattagaag agttatctaa aaaccttgat    300
   acgtctaatt tgggggctga tcttgaagaa gaatatccct ctaaaccaga gacaaccaac    360
   aataaagaaa gcaatgtagt aacaaatgct tcaactgcaa tagcacagaa agttccctca    420
   gcatatgaag aggtgaagcc agaaagcaag tcatcgcttg ctgttcttga tacatctaaa    480
   ataacaaaat tacaagccat aacccaaaga ggaaagggaa atgtagtagc tattattgat    540
   actggctttg atattaacca tgatattttt cgtttagata gcccaaaaga tgataagcac    600
   agctttaaaa ctaagacaga atttgaggaa ttaaaagcaa aacataatat cacttatggg    660
   aaatgggtta acgataagat tgtttttgca cataactacg ccaacaatac agaaacggtg    720
   gctgatattg cagcagctat gaaagatggt tatggttcag aagcaaagaa tatttcgcat    780
   ggtacacacg ttgctggtat ttttgtaggt aatagtaaac gtccagcaat caatggtctt    840
   cttttagaag gtgcagcgcc aaatgctcaa gtcttattaa tgcgtattcc agataaaatt    900
   gattcggaca aatttggtga agcatatgct aaagcaatca cagacgctgt taatctagga    960
   gcaaaaacga ttaatatgag tattggaaaa acagctgatt ctttaattgc tctcaatgat   1020
   aaagttaaat tagcacttaa attagcttct gagaagggcg ttgcagttgt tgtggctgcc   1080
   ggaaatgaag gcgcatttgg tatggattat agcaaaccat tatcaactaa tcctgactac   1140
   ggtacggtta atagtccagc tatttctgaa gatactttga gtgttgctag ctatgaatca   1200
   cttaaaacta tcagtgaggt cgttgaaaca actattgaag gtaagttagt taagttgccg   1260
   attgtgactt ctaaaccttt tgacaaaggt aaggcctacg atgtggttta tgccaattat   1320
   ggtgcaaaaa aagactttga aggtaaggac tttaaaggta agattgcatt aattgagcgt   1380
   ggtggtggac ttgattttat gactaaaatc actcatgcta caaatgcagg tgttgttggt   1440
   atcgttattt ttaacgatca agaaaaacgt ggaaattttc taattcctta ccgtgaatta   1500
   cctgtgggga ttattagtaa agtagatggc gagcgtataa aaaatacttc aagtcagtta   1560
   acatttaacc agagttttga agtagttgat agccaaggtg gtaatcgtat gctggaacaa   1620
   tcaagttggg gcgtgacagc tgaaggagca atcaagcctg atgtaacagc ttctggcttt   1680
   gaaatttatt cttcaaccta taataatcaa taccaaacaa tgtctggtac aagtatggct   1740
   tcaccacatg ttgcaggatt aatgacaatg cttcaaagtc atttggctga gaaatataaa   1800
   gggatgaatt tagattctaa aaaattgcta gaattgtcta aaaacatcct catgagctca   1860
   gcaacagcat tatatagtga agaggataag gcgttttatt caccacgtca gcaaggtgca   1920
   ggtgtagttg atgctgaaaa agctatccaa gctcaatatt atattactgg aaacgatggc   1980
   aaagctaaaa ttaatctcaa acgaatggga gataaatttg atatcacagt tacaattcat   2040
   aaacttgtag aaggtgtcaa agaattgtat tatcaagcta atgtagcaac agaacaagta   2100
   aataaaggta aatttgccct taaaccacaa gccttgctag atactaattg gcagaaagta   2160
   attcttcgtg ataaagaaac acaagttcga tttactattg atgctagtca atttagtcag   2220
   aaattaaaag aacagatggc aaatggttat ttcttagaag gttttgtacg ttttaaagaa   2280
   gccaaggata gtaatcagga gttaatgagt attccttttg taggatttaa tggtgatttt   2340
   gcgaacttac aagcacttga aacaccgatt tataagacgc tttctaaagg tagtttctac   2400
   tataaaccaa atgatacaac tcataaagac caattggagt acaatgaatc agctcctttt   2460
   gaaagcaaca actatactgc cttgttaaca caatcagcgt cttggggcta tgttgattat   2520
   gtcaaaaatg gtggggagtt agaattagca ccggagagtc caaaaagaat tattttagga   2580
   acttttgaga ataaggttga ggataaaaca attcatcttt tggaaagaga tgcagcgaat   2640
   aatccatatt ttgccatttc tccaaataaa gatggaaata gggacgaaat cactccccag   2700
   gcaactttct taagaaatgt taaggatatt tctgctcaag ttctagatca aaatggaaat   2760
   gttatttggc aaagtaaggt tttaccatct tatcgtaaaa atttccataa taatccaaag   2820
   caaagtgatg gtcattatcg tatggatgct cttcagtgga gtggtttaga taaggatggc   2880
   aaagttgtag cagatggttt ttatacttat cgcttacgtt acacaccagt agcagaagga   2940
   gcaaatagtc aggagtcaga ctttaaagta caagtaagta ctaagtcacc aaatcttcct   3000
   tcacgagctc agtttgatga aactaatcga acattaagct tagccatgcc taaggaaagt   3060
   agttatgttc ctacatatcg tttacaatta gttttatctc atgttgtaaa agatgaagaa   3120
   tatggggatg agacttctta ccattatttc catatagatc aagaaggtaa agtgacactt   3180
   cctaaaacgg ttaagatagg agagagtgag gttgcggtag accctaaggc cttgacactt   3240
   gttgtggaag ataaagctgg taatttcgca acggtaaaat tgtctgatct cttgaataag   3300
   gcagtagtat cagagaaaga aaacgctata gtaatttcta acagtttcaa atattttgat   3360
   aacttgaaaa aagaacctat gtttatttct aaaaaagaaa aagtagtaaa caagaatcta   3420
   gaagaaataa tattagttaa gccgcaaact acagttacta ctcaatcatt gtctaaagaa   3480
   ataactaaat caggaaatga gaaagtcctc acttctacaa acaataatag tagcagagta   3540
   gctaagatca tatcacctaa acataacggg gattctgtta accatacctt acctagtaca   3600
   tcagatagag caacgaatgg tctatttgtt ggtactttgg cattgttatc tagtttactt   3660
   ctttatttga aacccaaaaa gactaaaaat aatagtaaa                          3699
<210> 8
   <211> 1233
   <212> PRT
   <213> group B streptococcus
<400> 8
   Val Asp Lys His His Ser Lys Lys Ala Ile Leu Lys Leu Thr Leu Ile
   1               5                   10                  15
   Thr Thr Ser Ile Leu Leu Met His Ser Asn Gln Val Asn Ala Glu Glu
               20                  25                  30
   Gln Glu Leu Lys Asn Gln Glu Gln Ser Pro Val Ile Ala Asn Val Ala
           35                  40                  45
   Gln Gln Pro Ser Pro Ser Val Thr Thr Asn Thr Val Glu Lys Thr Ser
   50                  55                  60
   Val Thr Ala Ala Ser Ala Ser Asn Thr Ala Lys Glu Met Gly Asp Thr
   65                  70                  75                  80
   Ser Val Lys Asn Asp Lys Thr Glu Asp Glu Leu Leu Glu Glu Leu Ser
                   85                  90                  95
   Lys Asn Leu Asp Thr Ser Asn Leu Gly Ala Asp Leu Glu Glu Glu Tyr
               100                 105                 110
   Pro Ser Lys Pro Glu Thr Thr Asn Asn Lys Glu Ser Asn Val Val Thr
           115                 120                 125
   Asn Ala Ser Thr Ala Ile Ala Gln Lys Val Pro Ser Ala Tyr Glu Glu
       130                 135                 140
   Val Lys Pro Glu Ser Lys Ser Ser Leu Ala Val Leu Asp Thr Ser Lys
   145                 150                 155                 160
   Ile Thr Lys Leu Gln Ala Ile Thr Gln Arg Gly Lys Gly Asn Val Val
                   165                 170                 175
   Ala Ile Ile Asp Thr Gly Phe Asp Ile Asn His Asp Ile Phe Arg Leu
               180                 185                 190
   Asp Ser Pro Lys Asp Asp Lys His Ser Phe Lys Thr Lys Thr Glu Phe
           195                 200                 205
   Glu Glu Leu Lys Ala Lys His Asn Ile Thr Tyr Gly Lys Trp Val Asn
       210                 215                 220
   Asp Lys Ile Val Phe Ala His Asn Tyr Ala Asn Asn Thr Glu Thr Val
   225                 230                 235                 240
   Ala Asp Ile Ala Ala Ala Met Lys Asp Gly Tyr Gly Ser Glu Ala Lys
                   245                 250                 255
   Asn Ile Ser His Gly Thr His Val Ala Gly Ile Phe Val Gly Asn Ser
               260                 265                 270
   Lys Arg Pro Ala Ile Asn Gly Leu Leu Leu Glu Gly Ala Ala Pro Asn
           275                 280                 285
   Ala Gln Val Leu Leu Met Arg Ile Pro Asp Lys Ile Asp Ser Asp Lys
       290                 295                 300
   Phe Gly Glu Ala Tyr Ala Lys Ala Ile Thr Asp Ala Val Asn Leu Gly
   305 310 315 320
   Ala Lys Thr Ile Asn Met Ser Ile Gly Lys Thr Ala Asp Ser Leu Ile
   325 330 335
   Ala Leu Asn Asp Lys Val Lys Leu Ala Leu Lys Leu Ala Ser Glu Lys
   340 345 350
   Gly Val Ala Val Val Val Ala Ala Gly Asn Glu Gly Ala Phe Gly Met
   355 360 365
   Asp Tyr Ser Lys Pro Leu Ser Thr Asn Pro Asp Tyr Gly Thr Val Asn
   370 375 380
   Ser Pro Ala Ile Ser Glu Asp Thr Leu Ser Val Ala Ser Tyr Glu Ser
   385 390 395 400
   Leu Lys Thr Ile Ser Glu Val Val Glu Thr Thr Ile Glu Gly Lys Leu
   405 410 415
   Val Lys Leu Pro Ile Val Thr Ser Lys Pro Phe Asp Lys Gly Lys Ala
   420 425 430
   Tyr Asp Val Val Tyr Ala Asn Tyr Gly Ala Lys Lys Asp Phe Glu Gly
   435 440 445
   Lys Asp Phe Lys Gly Lys Ile Ala Leu Ile Glu Arg Gly Gly Gly Leu
   450 455 460
   Asp Phe Met Thr Lys Ile Thr His Ala Thr Asn Ala Gly Val Val Gly
   465 470 475 480
   Ile Val Ile Phe Asn Asp Gln Glu Lys Arg Gly Asn Phe Leu Ile Pro
   485 490 495
   Tyr Arg Glu Leu Pro Val Gly Ile Ile Ser Lys Val Asp Gly Glu Arg
   500 505 510
   Ile Lys Asn Thr Ser Ser Gln Leu Thr Phe Asn Gln Ser Phe Glu Val
   515 520 525
   Val Asp Ser Gln Gly Gly Asn Arg Met Leu Glu Gln Ser Ser Trp Gly
   530 535 540
   Val Thr Ala Glu Gly Ala Ile Lys Pro Asp Val Thr Ala Ser Gly Phe
   545                 550                 555                 560
   Glu Ile Tyr Ser Ser Thr Tyr Asn Asn Gln Tyr Gln Thr Met Ser Gly
                   565                 570                 575
   Thr Ser Met Ala Ser Pro His Val Ala Gly Leu Met Thr Met Leu Gln
               580                 585                 590
   Ser His Leu Ala Glu Lys Tyr Lys Gly Met Asn Leu Asp Ser Lys Lys
           595                 600                 605
   Leu Leu Glu Leu Ser Lys Asn Ile Leu Met Ser Ser Ala Thr Ala Leu
       610                 615                 620
   Tyr Ser Glu Glu Asp Lys Ala Phe Tyr Ser Pro Arg Gln Gln Gly Ala
   625                 630                 635                 640
   Gly Val Val Asp Ala Glu Lys Ala Ile Gln Ala Gln Tyr Tyr Ile Thr
                   645                 650                 655
   Gly Asn Asp Gly Lys Ala Lys Ile Asn Leu Lys Arg Met Gly Asp Lys
               660                 665                 670
   Phe Asp Ile Thr Val Thr Ile His Lys Leu Val Glu Gly Val Lys Glu
           675                 680                 685
   Leu Tyr Tyr Gln Ala Asn Val Ala Thr Glu Gln Val Asn Lys Gly Lys
       690                 695                 700
   Phe Ala Leu Lys Pro Gln Ala Leu Leu Asp Thr Asn Trp Gln Lys Val
   705                 710                 715                 720
   Ile Leu Arg Asp Lys Glu Thr Gln Val Arg Phe Thr Ile Asp Ala Ser
                   725                 730                 735
   Gln Phe Ser Gln Lys Leu Lys Glu Gln Met Ala Asn Gly Tyr Phe Leu
               740                 745                 750
   Glu Gly Phe Val Arg Phe Lys Glu Ala Lys Asp Ser Asn Gln Glu Leu
           755                 760                 765
   Met Ser Ile Pro Phe Val Gly Phe Asn Gly Asp Phe Ala Asn Leu Gln
       770                 775                 780
   Ala Leu Glu Thr Pro Ile Tyr Lys Thr Leu Ser Lys Gly Ser Phe Tyr
   785                 790                 795                 800
   Tyr Lys Pro Asn Asp Thr Thr His Lys Asp Gln Leu Glu Tyr Asn Glu
                   805                 810                 815
   Ser Ala Pro Phe Glu Ser Asn Asn Tyr Thr Ala Leu Leu Thr Gln Ser
               820                 825                 830
   Ala Ser Trp Gly Tyr Val Asp Tyr Val Lys Asn Gly Gly Glu Leu Glu
           835                 840                 845
   Leu Ala Pro Glu Ser Pro Lys Arg Ile Ile Leu Gly Thr Phe Glu Asn
       850                 855                 860
   Lys Val Glu Asp Lys Thr Ile His Leu Leu Glu Arg Asp Ala Ala Asn
   865                 870                 875                 880
   Asn Pro Tyr Phe Ala Ile Ser Pro Asn Lys Asp Gly Asn Arg Asp Glu
                   885                 890                 895
   Ile Thr Pro Gln Ala Thr Phe Leu Arg Asn Val Lys Asp Ile Ser Ala
               900                 905                 910
   Gln Val Leu Asp Gln Asn Gly Asn Val Ile Trp Gln Ser Lys Val Leu
           915                 920                 925
   Pro Ser Tyr Arg Lys Asn Phe His Asn Asn Pro Lys Gln Ser Asp Gly
       930                 935                 940
   His Tyr Arg Met Asp Ala Leu Gln Trp Ser Gly Leu Asp Lys Asp Gly
   945                 950                 955                 960
   Lys Val Val Ala Asp Gly Phe Tyr Thr Tyr Arg Leu Arg Tyr Thr Pro
                   965                 970                 975
   Val Ala Glu Gly Ala Asn Ser Gln Glu Ser Asp Phe Lys Val Gln Val
               980                 985                 990
   Ser Thr Lys Ser Pro Asn Leu Pro  Ser Arg Ala Gln Phe  Asp Glu Thr
           995                 1000                1005
   Asn Arg  Thr Leu Ser Leu Ala  Met Pro Lys Glu Ser  Ser Tyr Val
       1010                1015                1020
   Pro Thr  Tyr Arg Leu Gln Leu  Val Leu Ser His Val  Val Lys Asp
   1025 1030 1035
   Glu Glu Tyr Gly Asp Glu Thr Ser Tyr His Tyr Phe His Ile Asp
   1040 1045 1050
   Gln Glu Gly Lys Val Thr Leu Pro Lys Thr Val Lys Ile Gly Glu
   1055 1060 1065
   Ser Glu Val Ala Val Asp Pro Lys Ala Leu Thr Leu Val Val Glu
   1070 1075 1080
   Asp Lys Ala Gly Asn Phe Ala Thr Val Lys Leu Ser Asp Leu Leu
   1085 1090 1095
   Asn Lys Ala Val Val Ser Glu Lys Glu Asn Ala Ile Val Ile Ser
   1100 1105 1110
   Asn Ser Phe Lys Tyr Phe Asp Asn Leu Lys Lys Glu Pro Met Phe
   1115 1120 1125
   Ile Ser Lys Lys Glu Lys Val Val Asn Lys Asn Leu Glu Glu Ile
   1130 1135 1140
   Ile Leu Val Lys Pro Gln Thr Thr Val Thr Thr Gln Ser Leu Ser
   1145 1150 1155
   Lys Glu Ile Thr Lys Ser Gly Asn Glu Lys Val Leu Thr Ser Thr
   1160 1165 1170
   Asn Asn Asn Ser Ser Arg Val Ala Lys Ile Ile Ser Pro Lys His
   1175 1180 1185
   Asn Gly Asp Ser Val Asn His Thr Leu Pro Ser Thr Ser Asp Arg
   1190 1195 1200
   Ala Thr Asn Gly Leu Phe Val Gly Thr Leu Ala Leu Leu Ser Ser
   1205 1210 1215
   Leu Leu Leu Tyr Leu Lys Pro Lys Lys Thr Lys Asn Asn Ser Lys
   1220 1225 1230
<210> 9
   <211> 2040
   <212> DNA
   <213> group B streptococcus
<400> 9
   atgaaacgta aatactttat tcttaatacg gtgacggttt taacgttagc tgctgcaatg     60
   aatactagca gtatctatgc taatagtact gagacaagtg cttcagtagt tcctactaca    120
   aatactatcg ttcaaactaa tgacagtaat cctaccgcaa aatttgtatc agaatcagga    180
   caatctgtaa taggtcaagt aaaaccagat aattctgcgg cgcttacaac agttgacacg    240
   cctcatcata tttcagctcc agatgcttta aaaacaactc aatcaagtcc tgtcgttgag    300
   agtacttcta ctaagttaac tgaagagact tacaaacaaa aagatggtca agatttagcc    360
   aacatggtga gaagtggtca agttactagt gaggaactcg ttaatatggc atacgatatt    420
   attgctaaag aaaacccatc tttaaatgca gtcattacta ctagacgcca agaagctatt    480
   gaagaggcta gaaaacttaa agataccaat cagccgtttt taggtgttcc cttgttagtc    540
   aaggggttag ggcacagtat taaaggtggt gaaaccaata atggcttgat ctatgcagat    600
   ggaaaaatta gcacatttga cagtagctat gtcaaaaaat ataaagattt aggatttatt    660
   attttaggac aaacgaactt tccagagtat gggtggcgta atataacaga ttctaaatta    720
   tacggtctaa cgcataatcc ttgggatctt gctcataatg ctggtggctc ttctggtgga    780
   agtgcagcag ccattgctag cggaatgacg ccaattgcta gcggtagtga tgctggtggt    840
   tctatccgta ttccatcttc ttggacgggc ttggtaggtt taaaaccaac aagaggattg    900
   gtgagtaatg aaaagccaga ttcgtatagt acagcagttc attttccatt aactaagtca    960
   tctagagacg cagaaacatt attaacttat ctaaagaaaa gcgatcaaac gctagtatca   1020
   gttaatgatt taaaatcttt accaattgct tatactttga aatcaccaat gggaacagaa   1080
   gttagtcaag atgctaaaaa cgctattatg gacaacgtca cattcttaag aaaacaagga   1140
   ttcaaagtaa cagagataga cttaccaatt gatggtagag cattaatgcg tgattattca   1200
   accttggcta ttggcatggg aggagctttt tcaacaattg aaaaagactt aaaaaaacat   1260
   ggttttacta aagaagacgt tgatcctatt acttgggcag ttcatgttat ttatcaaaat   1320
   tcagataagg ctgaacttaa gaaatctatt atggaagccc aaaaacatat ggatgattat   1380
   cgtaaggcaa tggagaagct tcacaagcaa tttcctattt tcttatcgcc aacgaccgca   1440
   agtttagccc ctctaaatac agatccatat gtaacagagg aagataaaag agcgatttat   1500
   aatatggaaa acttgagcca agaagaaaga attgctctct ttaatcgcca gtgggagcct   1560
   atgttgcgta gaacaccttt tacacaaatt gctaatatga caggactccc agctatcagt   1620
   atcccgactt acttatctga gtctggttta cccataggga cgatgttaat ggcaggtgca   1680
   aactatgata tggtattaat taaatttgca actttctttg aaaaacatca tggttttaat   1740
   gttaaatggc aaagaataat agataaagaa gtgaaaccat ctactggcct aatacagcct   1800
   actaactccc tctttaaagc tcattcatca ttagtaaatt tagaagaaaa ttcacaagtt   1860
   actcaagtat ctatctctaa aaaatggatg aaatcgtctg ttaaaaataa accatccgta 1920
   atggcatatc aaaaagcact tcctaaaaca ggtgatacag aatcaagcct atctccagtt 1980
   ttagtagtaa cccttttatt agcttgtttt agctttgtaa caaaaaagaa tcagaaaagt 2040
<210> 10
   <211> 680
   <212> PRT
   <213> group B streptococcus
<400> 10
   Met Lys Arg Lys Tyr Phe Ile Leu Asn Thr Val Thr Val Leu Thr Leu
   1               5                   10                  15
   Ala Ala Ala Met Asn Thr Ser Ser Ile Tyr Ala Asn Ser Thr Glu Thr
               20                  25                  30
   Ser Ala Ser Val Val Pro Thr Thr Asn Thr Ile Val Gln Thr Asn Asp
           35                  40                  45
   Ser Asn Pro Thr Ala Lys Phe Val Ser Glu Ser Gly Gln Ser Val Ile
       50                  55                  60
   Gly Gln Val Lys Pro Asp Asn Ser Ala Ala Leu Thr Thr Val Asp Thr
   65                  70                  75                  80
   Pro His His Ile Ser Ala Pro Asp Ala Leu Lys Thr Thr Gln Ser Ser
                   85                  90                  95
   Pro Val Val Glu Ser Thr Ser Thr Lys Leu Thr Glu Glu Thr Tyr Lys
               100                 105                 110
   Gln Lys Asp Gly Gln Asp Leu Ala Asn Met Val Arg Ser Gly Gln Val
           115                 120                 125
   Thr Ser Glu Glu Leu Val Asn Met Ala Tyr Asp Ile Ile Ala Lys Glu
       130                 135                 140
   Asn Pro Ser Leu Asn Ala Val Ile Thr Thr Arg Arg Gln Glu Ala Ile
   145                 150                 155                 160
   Glu Glu Ala Arg Lys Leu Lys Asp Thr Asn Gln Pro Phe Leu Gly Val
                   165                 170                 175
   Pro Leu Leu Val Lys Gly Leu Gly His Ser Ile Lys Gly Gly Glu Thr
               180                 185                 190
   Asn Asn Gly Leu Ile Tyr Ala Asp Gly Lys Ile Ser Thr Phe Asp Ser
           195                 200                 205
   Ser Tyr Val Lys Lys Tyr Lys Asp Leu Gly Phe Ile Ile Leu Gly Gln
       210                 215                 220
   Thr Asn Phe Pro Glu Tyr Gly Trp Arg Asn Ile Thr Asp Ser Lys Leu
   225                 230                 235                 240
   Tyr Gly Leu Thr His Asn Pro Trp Asp Leu Ala His Asn Ala Gly Gly
                   245                 250                 255
   Ser Ser Gly Gly Ser Ala Ala Ala Ile Ala Ser Gly Met Thr Pro Ile
               260                 265                 270
   Ala Ser Gly Ser Asp Ala Gly Gly Ser Ile Arg Ile Pro Ser Ser Trp
           275                 280                 285
   Thr Gly Leu Val Gly Leu Lys Pro Thr Arg Gly Leu Val Ser Asn Glu
       290                 295                 300
   Lys Pro Asp Ser Tyr Ser Thr Ala Val His Phe Pro Leu Thr Lys Ser
   305                 310                 315                 320
   Ser Arg Asp Ala Glu Thr Leu Leu Thr Tyr Leu Lys Lys Ser Asp Gln
                   325                 330                 335
   Thr Leu Val Ser Val Asn Asp Leu Lys Ser Leu Pro Ile Ala Tyr Thr
               340                 345                 350
   Leu Lys Ser Pro Met Gly Thr Glu Val Ser Gln Asp Ala Lys Asn Ala
           355                 360                 365
   Ile Met Asp Asn Val Thr Phe Leu Arg Lys Gln Gly Phe Lys Val Thr
       370                 375                 380
   Glu Ile Asp Leu Pro Ile Asp Gly Arg Ala Leu Met Arg Asp Tyr Ser
   385                 390                 395                 400
   Thr Leu Ala Ile Gly Met Gly Gly Ala Phe Ser Thr Ile Glu Lys Asp
                   405                 410                 415
   Leu Lys Lys His Gly Phe Thr Lys Glu Asp Val Asp Pro Ile Thr Trp
               420                 425                 430
   Ala Val His Val Ile Tyr Gln Asn Ser Asp Lys Ala Glu Leu Lys Lys
           435                 440                 445
   Ser Ile Met Glu Ala Gln Lys His Met Asp Asp Tyr Arg Lys Ala Met
       450                 455                 460
   Glu Lys Leu His Lys Gln Phe Pro Ile Phe Leu Ser Pro Thr Thr Ala
   465                 470                 475                 480
   Ser Leu Ala Pro Leu Asn Thr Asp Pro Tyr Val Thr Glu Glu Asp Lys
                   485                 490                 495
   Arg Ala Ile Tyr Asn Met Glu Asn Leu Ser Gln Glu Glu Arg Ile Ala
               500                 505                 510
   Leu Phe Asn Arg Gln Trp Glu Pro Met Leu Arg Arg Thr Pro Phe Thr
           515                 520                 525
   Gln Ile Ala Asn Met Thr Gly Leu Pro Ala Ile Ser Ile Pro Thr Tyr
       530                 535                 540
   Leu Ser Glu Ser Gly Leu Pro Ile Gly Thr Met Leu Met Ala Gly Ala
   545                 550                 555                 560
   Asn Tyr Asp Met Val Leu Ile Lys Phe Ala Thr Phe Phe Glu Lys His
                   565                 570                 575
   His Gly Phe Asn Val Lys Trp Gln Arg Ile Ile Asp Lys Glu Val Lys
               580                 585                 590
   Pro Ser Thr Gly Leu Ile Gln Pro Thr Asn Ser Leu Phe Lys Ala His
           595                 600                 605
   Ser Ser Leu Val Asn Leu Glu Glu Asn Ser Gln Val Thr Gln Val Ser
       610                 615                 620
   Ile Ser Lys Lys Trp Met Lys Ser Ser Val Lys Asn Lys Pro Ser Val
   625                 630                 635                 640
   Met Ala Tyr Gln Lys Ala Leu Pro Lys Thr Gly Asp Thr Glu Ser Ser
                   645                 650                 655
   Leu Ser Pro Val Leu Val Val Thr Leu Leu Leu Ala Cys Phe Ser Phe
               660                 665                 670
   Val Thr Lys Lys Asn Gln Lys Ser
   675 680
<210> 11
   <211> 3402
   <212> DNA
   <213> group B streptococcus
<400> 11' .
<210> 12
   <211> 1134
   <212> PRT
   <213> group B streptococcus
<400> 12
   Met Arg Lys Lys Gln Lys Leu Pro Phe Asp Lys Leu Ala Ile Ala Leu
   1               5                   10                  15
   Ile Ser Thr Ser Ile Leu Leu Asn Ala Gln Ser Asp Ile Lys Ala Asn
               20                  25                  30
   Thr Val Thr Glu Asp Thr Pro Ala Thr Glu Gln Ala Val Glu Pro Pro
           35                  40                  45
   Gln Pro Ile Ala Val Ser Glu Glu Ser Arg Ser Ser Lys Glu Thr Lys
       50                  55                  60
   Thr Ser Gln Thr Pro Ser Asp Val Gly Glu Thr Val Ala Asp Asp Ala
   65                  70                  75                  80
   Asn Asp Leu Ala Pro Gln Ala Pro Ala Lys Thr Ala Asp Thr Pro Ala
                   85                  90                  95
   Thr Ser Lys Ala Thr Ile Arg Asp Leu Asn Asp Pro Ser His Val Lys
               100                 105                 110
   Thr Leu Gln Glu Lys Ala Gly Lys Gly Ala Gly Thr Val Val Ala Val
           115                 120                 125
   Ile Asp Ala Gly Phe Asp Lys Asn His Glu Ala Trp Arg Leu Thr Asp
       130                 135                 140
   Lys Thr Lys Ala Arg Tyr Gln Ser Lys Glu Asn Leu Glu Lys Ala Lys
   145                 150                 155                 160
   Lys Glu His Gly Ile Thr Tyr Gly Glu Trp Val Asn Asp Lys Val Ala
                   165                 170                 175
   Tyr Tyr His Asp Tyr Ser Lys Asp Gly Lys Asn Ala Val Asp Gln Glu
               180                 185                 190
   His Gly Thr His Val Ser Gly Ile Leu Ser Gly Asn Ala Pro Ser Glu
           195                 200                 205
   Met Lys Glu Pro Tyr Arg Leu Glu Gly Ala Met Pro Glu Ala Gln Leu
       210                 215                 220
   Leu Leu Met Arg Val Glu Ile Val Asn Gly Leu Ala Asp Tyr Ala Arg
   225                 230                 235                 240
   Asn Tyr Ala Gln Ala Ile Arg Asp Ala Val Asn Leu Gly Ala Lys Val
                   245                 250                 255
   Ile Asn Met Ser Phe Gly Asn Ala Ala Leu Ala Tyr Ala Asn Leu Pro
               260                 265                 270
   Asp Glu Thr Lys Lys Ala Phe Asp Tyr Ala Lys Ser Lys Gly Val Ser
           275                 280                 285
   Ile Val Thr Ser Ala Gly Asn Asp Ser Ser Phe Gly Gly Lys Pro Arg
       290                 295                 300
   Leu Pro Leu Ala Asp His Pro Asp Tyr Gly Val Val Gly Thr Pro Ala
   305                 310                 315                 320
   Ala Ala Asp Ser Thr Leu Thr Val Ala Ser Tyr Ser Pro Asp Lys Gln
                   325                 330                 335
   Leu Thr Glu Thr Ala Thr Val Lys Thr Asp Asp His Gln Asp Lys Glu
               340                 345                 350
   Met Pro Val Ile Ser Thr Asn Arg Phe Glu Pro Asn Lys Ala Tyr Asp
           355                 360                 365
   Tyr Ala Tyr Ala Asn Arg Gly Thr Lys Glu Asp Asp Phe Lys Asp Val
       370                 375                 380
   Glu Gly Lys Ile Ala Leu Ile Glu Arg Gly Asp Ile Asp Phe Lys Asp
   385                 390                 395                 400
   Lys Ile Ala Asn Ala Lys Lys Ala Gly Ala Val Gly Val Leu Ile Tyr
                   405                 410                 415
   Asp Asn Gln Asp Lys Gly Phe Pro Ile Glu Leu Pro Asn Val Asp Gln
               420                 425                 430
   Met Pro Ala Ala Phe Ile Ser Arg Arg Asp Gly Leu Leu Leu Lys Asp
           435                 440                 445
   Asn Pro Pro Lys Thr Ile Thr Phe Asn Ala Thr Pro Lys Val Leu Pro
   450 455 460
   Thr Ala Ser Gly Thr Lys Leu Ser Arg Phe Ser Ser Trp Gly Leu Thr
   465 470 475 480
   Ala Asp Gly Asn Ile Lys Pro Asp Ile Ala Ala Pro Gly Gln Asp Ile
   485 490 495
   Leu Ser Ser Val Ala Asn Asn Lys Tyr Ala Lys Leu Ser Gly Thr Ser
   500 505 510
   Met Ser Ala Pro Leu Val Ala Gly Ile Met Gly Leu Leu Gln Lys Gln
   515 520 525
   Tyr Glu Thr Gln Tyr Pro Asp Met Thr Pro Ser Glu Arg Leu Asp Leu
   530 535 540
   Ala Lys Lys Val Leu Met Ser Ser Ala Thr Ala Leu Tyr Asp Glu Asp
   545 550 555 560
   Glu Lys Ala Tyr Phe Ser Pro Arg Gln Gln Gly Ala Gly Ala Val Asp
   565 570 575
   Ala Lys Lys Ala Ser Ala Ala Thr Met Tyr Val Thr Asp Lys Asp Asn
   580 585 590
   Thr Ser Ser Lys Val His Leu Asn Asn Val Ser Asp Lys Phe Glu Val
   595 600 605
   Thr Val Thr Val His Asn Lys Ser Asp Lys Pro Gln Glu Leu Tyr Tyr
   610 615 620
   Gln Val Thr Val Gln Thr Asp Lys Val Asp Gly Lys His Phe Ala Leu
   625 630 635 640
   Ala Pro Lys Ala Leu Tyr Glu Thr Ser Trp Gln Lys Ile Thr Ile Pro
   645 650 655
   Ala Asn Ser Ser Lys Gln Val Thr Val Pro Ile Asp Ala Ser Arg Phe
   660 665 670
   Ser Lys Asp Leu Leu Ala Gln Met Lys Asn Gly Tyr Phe Leu Glu Gly
   675 680 685
   Phe Val Arg Phe Lys Gln Asp Pro Thr Lys Glu Glu Leu Met Ser Ile
   690 695 700
   Pro Tyr Ile Gly Phe Arg Gly Asp Phe Gly Asn Leu Ser Ala Leu Glu
   705 710 715 720
   Lys Pro Ile Tyr Asp Ser Lys Asp Gly Ser Ser Tyr Tyr His Glu Ala
   725 730 735
   Asn Ser Asp Ala Lys Asp Gln Leu Asp Gly Asp Gly Leu Gln Phe Tyr
   740 745 750
   Ala Leu Lys Asn Asn Phe Thr Ala Leu Thr Thr Glu Ser Asn Pro Trp
   755 760 765
   Thr Ile Ile Lys Ala Val Lys Glu Gly Val Glu Asn Ile Glu Asp Ile
   770 775 780
   Glu Ser Ser Glu Ile Thr Glu Thr Ile Phe Ala Gly Thr Phe Ala Lys
   785 790 795 800
   Gln Asp Asp Asp Ser His Tyr Tyr Ile His Arg His Ala Asn Gly Lys
   805 810 815
   Pro Tyr Ala Ala Ile Ser Pro Asn Gly Asp Gly Asn Arg Asp Tyr Val
   820 825 830
   Gln Phe Gln Gly Thr Phe Leu Arg Asn Ala Lys Asn Leu Val Ala Glu
   835 840 845
   Val Leu Asp Lys Glu Gly Asn Val Val Trp Thr Ser Glu Val Thr Glu
   850 855 860
   Gln Val Val Lys Asn Tyr Asn Asn Asp Leu Ala Ser Thr Leu Gly Ser
   865 870 875 880
   Thr Arg Phe Glu Lys Thr Arg Trp Asp Gly Lys Asp Lys Asp Gly Lys
   885 890 895
   Val Val Ala Asn Gly Thr Tyr Thr Tyr Arg Val Arg Tyr Thr Pro Ile
   900 905 910
   Ser Ser Gly Ala Lys Glu Gln His Thr Asp Phe Asp Val Ile Val Asp
   915 920 925
   Asn Thr Thr Pro Glu Val Ala Thr Ser Ala Thr Phe Ser Thr Glu Asp
   930 935 940
   Ser Arg Leu Thr Leu Ala Ser Lys Pro Lys Thr Ser Gln Pro Val Tyr
   945                 950                 955                 960
   Arg Glu Arg Ile Ala Tyr Thr Tyr Met Asp Glu Asp Leu Pro Thr Thr
                   965                 970                 975
   Glu Tyr Ile Ser Pro Asn Glu Asp Gly Thr Phe Thr Leu Pro Glu Glu
               980                 985                 990
   Ala Glu Thr Met Glu Gly Ala Thr  Val Pro Leu Lys Met  Ser Asp Phe
           995                 1000                1005
   Thr Tyr  Val Val Glu Asp Met  Ala Gly Asn Ile Thr  Tyr Thr Pro
       1010                 1015                 1020
   Val Thr  Lys Leu Leu Glu Gly  His Ser Asn Lys Pro  Glu Gln Asp
       1025                 1030                 1035
   Gly Ser  Asp Gln Ala Pro Asp  Lys Lys Pro Glu Ala  Lys Pro Glu
       1040                 1045                 1050
   Gln Asp  Gly Ser Gly Gln Thr  Pro Asp Lys Lys Lys  Glu Thr Lys
       1055                 1060                 1065
   Pro Glu  Lys Asp Ser Ser Gly  Gln Thr Pro Gly Lys  Thr Pro Gln
       1070                 1075                 1080
   Lys Gly  Gln Ser Ser Arg Thr  Leu Glu Lys Arg Ser  Ser Lys Arg
       1085                 1090                 1095
   Ala Leu  Ala Thr Lys Ala Ser  Thr Arg Asp Gln Leu  Pro Thr Thr
       1100                 1105                 1110
   Asn Asp  Lys Asp Thr Asn Arg  Leu His Leu Leu Lys  Leu Val Met
       1115                 1120                 1125
   Thr Thr  Phe Phe Leu Gly
       1130
<210> 13
   <211> 1365
   <212> DNA
   <213> group B streptococcus
<400> 13
   atgggacgag taatgaaaac aataacaaca tttgaaaata aaaaagtttt agtccttggt      60
   ttagcacgat ctggagaagc tgctgcacgt ttgttagcta agttaggagc aatagtgaca     120
   gttaatgatg gcaaaccatt tgatgaaaat ccaacagcac agtctttgtt ggaagagggt     180
   attaaagtgg tttgtggtag tcatccttta gaattgttag atgaggattt ttgttacatg     240
   attaaaaatc caggaatacc ttataacaat cctatggtca aaaaagcatt agaaaaacaa     300
   atccctgttt tgactgaagt ggaattagca tacttagttt cagaatctca gctaataggt     360
   attacaggct ctaacgggaa aacgacaacg acaacgatga ttgcagaagt cttaaatgct     420
   ggaggtcaga gaggtttgtt agctgggaat atcggctttc ctgctagtga agttgttcag     480
   gctgcgaatg ataaagatac tctagttatg gaattatcaa gttttcagct aatgggagtt     540
   aaggaatttc gtcctcatat tgcagtaatt actaatttaa tgccaactca tttagattat     600
   catgggtctt ttgaagatta tgttgctgca aaatggaata tccaaaatca aatgtcttca     660
   tctgattttt tggtacttaa ttttaatcaa ggtatttcta aagagttagc taaaactact     720
   aaagcaacaa tcgttccttt ctctactacg gaaaaagttg atggtgctta cgtacaagac     780
   aagcaacttt tctataaagg ggagaatatt atgtcagtag atgacattgg tgtcccagga     840
   agccataacg tagagaatgc tctagcaact attgcggttg ctaaactggc tggtatcagt     900
   aatcaagtta ttagagaaac tttaagcaat tttggaggtg ttaaacaccg cttgcaatca     960
   ctcggtaagg ttcatggtat tagtttctat aacgacagca agtcaactaa tatattggca    1020
   actcaaaaag cattatctgg ctttgataat actaaagtta tcctaattgc aggaggtctt    1080
   gatcgcggta atgagtttga tgaattgata ccagatatca ctggacttaa acatatggtt    1140
   gttttagggg aatcggcatc tcgagtaaaa cgtgctgcac aaaaagcagg agtaacttat    1200
   agcgatgctt tagatgttag agatgcggta cataaagctt atgaggtggc acaacagggc    1260
   gatgttatct tgctaagtcc tgcaaatgca tcatgggaca tgtataagaa tttcgaagtc    1320
   cgtggtgatg aattcattga tactttcgaa agtcttagag gagag                    1365
<210> 14
   <211> 455
   <212> PRT
   <213> group B streptococcus
<400> 14
   Met Gly Arg Val Met Lys Thr Ile Thr Thr Phe Glu Asn Lys Lys Val
   1               5                   10                  15
   Leu Val Leu Gly Leu Ala Arg Ser Gly Glu Ala Ala Ala Arg Leu Leu
               20                  25                  30
   Ala Lys Leu Gly Ala Ile Val Thr Val Asn Asp Gly Lys Pro Phe Asp
           35                  40                  45
   Glu Asn Pro Thr Ala Gln Ser Leu Leu Glu Glu Gly Ile Lys Val Val
       50                  55                  60
   Cys Gly Ser His Pro Leu Glu Leu Leu Asp Glu Asp Phe Cys Tyr Met
   65                  70                  75                  80
   Ile Lys Asn Pro Gly Ile Pro Tyr Asn Asn Pro Met Val Lys Lys Ala
                   85                  90                  95
   Leu Glu Lys Gln Ile Pro Val Leu Thr Glu Val Glu Leu Ala Tyr Leu
               100                 105                 110
   Val Ser Glu Ser Gln Leu Ile Gly Ile Thr Gly Ser Asn Gly Lys Thr
           115                 120                 125
   Thr Thr Thr Thr Met Ile Ala Glu Val Leu Asn Ala Gly Gly Gln Arg
       130                 135                 140
   Gly Leu Leu Ala Gly Asn Ile Gly Phe Pro Ala Ser Glu Val Val Gln
   145                 150                 155                 160
   Ala Ala Asn Asp Lys Asp Thr Leu Val Met Glu Leu Ser Ser Phe Gln
                   165                 170                 175
   Leu Met Gly Val Lys Glu Phe Arg Pro His Ile Ala Val Ile Thr Asn
               180                 185                 190
   Leu Met Pro Thr His Leu Asp Tyr His Gly Ser Phe Glu Asp Tyr Val
           195                 200                 205
   Ala Ala Lys Trp Asn Ile Gln Asn Gln Met Ser Ser Ser Asp Phe Leu
       210                 215                 220
   Val Leu Asn Phe Asn Gln Gly Ile Ser Lys Glu Leu Ala Lys Thr Thr
   225                 230                 235                 240
   Lys Ala Thr Ile Val Pro Phe Ser Thr Thr Glu Lys Val Asp Gly Ala
                   245                 250                 255
   Tyr Val Gln Asp Lys Gln Leu Phe Tyr Lys Gly Glu Asn Ile Met Ser
               260                 265                 270
   Val Asp Asp Ile Gly Val Pro Gly Ser His Asn Val Glu Asn Ala Leu
   275 280 285
   Ala Thr Ile Ala Val Ala Lys Leu Ala Gly Ile Ser Asn Gln Val Ile
   290 295 300
   Arg Glu Thr Leu Ser Asn Phe Gly Gly Val Lys His Arg Leu Gln Ser
   305 310 315 320
   Leu Gly Lys Val His Gly Ile Ser Phe Tyr Asn Asp Ser Lys Ser Thr
   325 330 335
   Asn Ile Leu Ala Thr Gln Lys Ala Leu Ser Gly Phe Asp Asn Thr Lys
   340 345 350
   Val Ile Leu Ile Ala Gly Gly Leu Asp Arg Gly Asn Glu Phe Asp Glu
   355 360 365
   Leu Ile Pro Asp Ile Thr Gly Leu Lys His Met Val Val Leu Gly Glu
   370 375 380
   Ser Ala Ser Arg Val Lys Arg Ala Ala Gln Lys Ala Gly Val Thr Tyr
   385 390 395 400
   Ser Asp Ala Leu Asp Val Arg Asp Ala Val His Lys Ala Tyr Glu Val
   405 410 415
   Ala Gln Gln Gly Asp Val Ile Leu Leu Ser Pro Ala Asn Ala Ser Trp
   420 425 430
   Asp Met Tyr Lys Asn Phe Glu Val Arg Gly Asp Glu Phe Ile Asp Thr
   435 440 445
   Phe Glu Ser Leu Arg Gly Glu
   450 455
<210> 15
   <211> 1020
   <212> DNA
   <213> group B streptococcus
<400> 15
   atgaaacgta ttgctgtttt aactagtggt ggtgacgccc ctggtatgaa cgctgctatc      60
   cgtgcagttg ttcgtaaagc aatttctgaa ggtatggaag tttacggcat caaccaaggt     120
   tactatggta tggtgacagg ggatattttc cctttggatg ctaattctgt tggggatact     180
   atcaaccgtg gaggaacgtt tttacgttca gcacgttatc ctgaatttgc tgaacttgaa     240
   ggtcagctta aagggattga acagcttaaa aaacacggta ttgaaggtgt agtagttatc     300
   ggtggtgatg gttcttatca tggtgctatg cgtctaactg agcacggttt cccagctgtt     360
   ggtttgccgg gtacaattga taacgatatc gttggcactg actatactat tggttttgac     420
   acagcagttg cgacagcagt tgagaatctt gaccgtcttc gtgatacatc agcaagtcat     480
   aaccgtactt ttgttgttga ggttatggga agaaatgcag gagatatcgc tctttggtca     540
   ggtatcgctg caggtgcaga tcaaattatt gttcctgaag aagagttcaa tattgatgaa     600
   gttgtctcaa atgttagagc tggctatgca gctggtaaac atcaccaaat catcgtcctt     660
   gcagaaggtg ttatgagtgg tgatgagttt gcaaaaacaa tgaaagcagc aggagacgat     720
   agcgatcttc gtgtgacgaa tttaggacat ctgctccgtg gtggtagtcc gacggctcgt     780
   gatcgtgtct tagcatctcg tatgggagcg tacgctgttc aattgttgaa agaaggtcgt     840
   ggtggtttag ccgttggtgt ccacaacgaa gaaatggttg aaagtccaat tttaggttta     900
   gcagaagaag gtgctttgtt cagcttgact gatgaaggaa aaatcgttgt taataatccg     960
   cataaagcgg accttcgctt ggcagcactt aatcgtgacc ttgccaacca aagtagtaaa    1020
<210> 16
   <211> 340
   <212> PRT
   <213> group B streptococcus
<400> 16
   Met Lys Arg Ile Ala Val Leu Thr Ser Gly Gly Asp Ala Pro Gly Met
   1               5                   10                  15
   Asn Ala Ala Ile Arg Ala Val Val Arg Lys Ala Ile Ser Glu Gly Met
               20                  25                  30
   Glu Val Tyr Gly Ile Asn Gln Gly Tyr Tyr Gly Met Val Thr Gly Asp
           35                  40                  45
   Ile Phe Pro Leu Asp Ala Asn Ser Val Gly Asp Thr Ile Asn Arg Gly
       50                  55                  60
   Gly Thr Phe Leu Arg Ser Ala Arg Tyr Pro Glu Phe Ala Glu Leu Glu
   65                  70                  75                  80
   Gly Gln Leu Lys Gly Ile Glu Gln Leu Lys Lys His Gly Ile Glu Gly
                   85                  90                  95
   Val Val Val Ile Gly Gly Asp Gly Ser Tyr His Gly Ala Met Arg Leu
               100                 105                 110
   Thr Glu His Gly Phe Pro Ala Val Gly Leu Pro Gly Thr Ile Asp Asn
           115                 120                 125
   Asp Ile Val Gly Thr Asp Tyr Thr Ile Gly Phe Asp Thr Ala Val Ala
       130                 135                 140
   Thr Ala Val Glu Asn Leu Asp Arg Leu Arg Asp Thr Ser Ala Ser His
   145                 150                 155                 160
   Asn Arg Thr Phe Val Val Glu Val Met Gly Arg Asn Ala Gly Asp Ile
                   165                 170                 175
   Ala Leu Trp Ser Gly Ile Ala Ala Gly Ala Asp Gln Ile Ile Val Pro
               180                 185                 190
   Glu Glu Glu Phe Asn Ile Asp Glu Val Val Ser Asn Val Arg Ala Gly
           195                 200                 205
   Tyr Ala Ala Gly Lys His His Gln Ile Ile Val Leu Ala Glu Gly Val
       210                 215                 220
   Met Ser Gly Asp Glu Phe Ala Lys Thr Met Lys Ala Ala Gly Asp Asp
   225                 230                 235                 240
   Ser Asp Leu Arg Val Thr Asn Leu Gly His Leu Leu Arg Gly Gly Ser
                   245                 250                 255
   Pro Thr Ala Arg Asp Arg Val Leu Ala Ser Arg Met Gly Ala Tyr Ala
               260                 265                 270
   Val Gln Leu Leu Lys Glu Gly Arg Gly Gly Leu Ala Val Gly Val His
           275                 280                 285
   Asn Glu Glu Met Val Glu Ser Pro Ile Leu Gly Leu Ala Glu Glu Gly
       290                 295                 300
   Ala Leu Phe Ser Leu Thr Asp Glu Gly Lys Ile Val Val Asn Asn Pro
   305                 310                 315                 320
   His Lys Ala Asp Leu Arg Leu Ala Ala Leu Asn Arg Asp Leu Ala Asn
                   325                 330                 335
   Gln Ser Ser Lys
               340
<210> 17
   <211> 1376
   <212> DNA
   <213> group B streptococcus
<400> 17
   atgaataaaa aggtactatt gacatcgaca atggcagctt cgctattatc agtcgcaagt      60
   gttcaagcac aagaaacaga tacgacgtgg acagcacgta ctgtttcaga ggtaaaggct     120
   gatttggtaa agcaagacaa taaatcatca tatactgtga aatatggtga tacactaagc     180
   gttatttcag aagcaatgtc aattgatatg aatgtcttag caaaaataaa taacattgca     240
   gatatcaatc ttatttatcc tgagacaaca ctgacagtaa cttacgatca gaagagtcat     300
   actgccactt caatgaaaat agaaacacca gcaacaaatg ctgctggtca aacaacagct     360
   actgtggatt tgaaaaccaa tcaagtttct gttgcagacc aaaaagtttc tctcaataca     420
   atttcggaag gtatgacacc agaagcagca acaacgattg tttcgccaat gaagacatat     480
   tcttctgcgc cagctttgaa atcaaaagaa gtattagcac aagagcaagc tgttagtcaa     540
   gcagcagcta atgaacaggt atcaccagct cctgtgaagt cgattacttc agaagttcca     600
   gcagctaaag aggaagttaa accaactcag acgtcagtca gtcagtcaac aacagtatca     660
   ccagcttctg ttgccgctga aacaccagct ccagtagcta aagtagcacc ggtaagaact     720
   gtagcagccc ctagagtggc aagtgttaaa gtagtcactc ctaaagtaga aactggtgca     780
   tcaccagagc atgtatcagc tccagcagtt cctgtgacta cgacttcacc agctacagac     840
   agtaagttac aagcgactga agttaagagc gttccggtag cacaaaaagc tccaacagca     900
   acaccggtag cacaaccagc ttcaacaaca aatgcagtag ctgcacatcc tgaaaatgca     960
   gggctccaac ctcatgttgc agcttataaa gaaaaagtag cgtcaactta tggagttaat    1020
   gaattcagta cataccgtgc gggagatcca ggtgatcatg gtaaaggttt agcagttgac    1080
   tttattgtag gtactaatca agcacttggt aataaagttg cacagtactc tacacaaaat    1140
   atggcagcaa ataacatttc atatgttatc tggcaacaaa agttttactc aaatacaaac    1200
   agtatttatg gacctgctaa tacttggaat gcaatgccag atcgtggtgg cgttactgcc    1260
   aaccactatg accacgttca cgtatcattt aacaaataat ataaaaaagg aagctatttg    1320
   gcttcttttt tatatgcctt gaatagactt tcaaggttct tatataattt ttatta        1376
<210> 18
   <211> 432
   <212> PRT
   <213> group B streptococcus
<400> 18
   Met Asn Lys Lys Val Leu Leu Thr Ser Thr Met Ala Ala Ser Leu Leu
   1               5                   10                  15
   Ser Val Ala Ser Val Gln Ala Gln Glu Thr Asp Thr Thr Trp Thr Ala
               20                  25                  30
   Arg Thr Val Ser Glu Val Lys Ala Asp Leu Val Lys Gln Asp Asn Lys
           35                  40                  45
   Ser Ser Tyr Thr Val Lys Tyr Gly Asp Thr Leu Ser Val Ile Ser Glu
       50                  55                  60
   Ala Met Ser Ile Asp Met Asn Val Leu Ala Lys Ile Asn Asn Ile Ala
   65                  70                  75                  80
   Asp Ile Asn Leu Ile Tyr Pro Glu Thr Thr Leu Thr Val Thr Tyr Asp
                   85                  90                  95
   Gln Lys Ser His Thr Ala Thr Ser Met Lys Ile Glu Thr Pro Ala Thr
               100                 105                 110
   Asn Ala Ala Gly Gln Thr Thr Ala Thr Val Asp Leu Lys Thr Asn Gln
           115                 120                 125
   Val Ser Val Ala Asp Gln Lys Val Ser Leu Asn Thr Ile Ser Glu Gly
       130                 135                 140
   Met Thr Pro Glu Ala Ala Thr Thr Ile Val Ser Pro Met Lys Thr Tyr
   145                 150                 155                 160
   Ser Ser Ala Pro Ala Leu Lys Ser Lys Glu Val Leu Ala Gln Glu Gln
                   165                 170                 175
   Ala Val Ser Gln Ala Ala Ala Asn Glu Gln Val Ser Pro Ala Pro Val
               180                 185                 190
   Lys Ser Ile Thr Ser Glu Val Pro Ala Ala Lys Glu Glu Val Lys Pro
           195                 200                 205
   Thr Gln Thr Ser Val Ser Gln Ser Thr Thr Val Ser Pro Ala Ser Val
       210                 215                 220
   Ala Ala Glu Thr Pro Ala Pro Val Ala Lys Val Ala Pro Val Arg Thr
   225                 230                 235                 240
   Val Ala Ala Pro Arg Val Ala Ser Val Lys Val Val Thr Pro Lys Val
                   245                 250                 255
   Glu Thr Gly Ala Ser Pro Glu His Val Ser Ala Pro Ala Val Pro Val
               260                 265                 270
   Thr Thr Thr Ser Pro Ala Thr Asp Ser Lys Leu Gln Ala Thr Glu Val
           275                 280                 285
   Lys Ser Val Pro Val Ala Gln Lys Ala Pro Thr Ala Thr Pro Val Ala
       290                 295                 300
   Gln Pro Ala Ser Thr Thr Asn Ala Val Ala Ala His Pro Glu Asn Ala
   305                 310                 315                     320
   Gly Leu Gln Pro His Val Ala Ala Tyr Lys Glu Lys Val Ala Ser Thr
                   325                 330                     335
   Tyr Gly Val Asn Glu Phe Ser Thr Tyr Arg Ala Gly Asp Pro Gly Asp
               340                 345                 350
   His Gly Lys Gly Leu Ala Val Asp Phe Ile Val Gly Thr Asn Gln Ala
           355                 360                 365
   Leu Gly Asn Lys Val Ala Gln Tyr Ser Thr Gln Asn Met Ala Ala Asn
       370                 375                 380
   Asn Ile Ser Tyr Val Ile Trp Gln Gln Lys Phe Tyr Ser Asn Thr Asn
   385                 390                 395                     400
   Ser Ile Tyr Gly Pro Ala Asn Thr Trp Asn Ala Met Pro Asp Arg Gly
                   405                 410                     415
   Gly Val Thr Ala Asn His Tyr Asp His Val His Val Ser Phe Asn Lys
               420                 425                 430
<210> 19
   <211> 2070
   <212> DNA
   <213> group B streptococcus
<400> 19
   atgaaaaaga aaattatttt gaaaagtagt gttcttggtt tagtcgctgg gacttctatt     60
   atgttctcaa gcgtgttcgc ggaccaagtc ggtgtccaag ttataggcgt caatgacttt    120
   catggtgcac ttgacaatac tggaacagca aatatgcctg atggaaaagt tgctaatgct    180
   ggtactgctg ctcaattaga tgcttatatg gatgacgctc aaaaagattt caaacaaact    240
   aaccctaatg gtgaaagcat tagggttcaa gcaggcgata tggttggagc aagtccagcc    300
   aactctgggc ttcttcaaga tgaaccaact gtcaaaaatt ttaatgcaat gaatgttgag     360
   tatggcacat tgggtaacca tgaatttgat gaagggttgg cagaatataa tcgtatcgtt     420
   actggtaaag cccctgctcc agattctaat attaataata ttacgaaatc atacccacat     480
   gaagctgcaa aacaagaaat tgtagtggca aatgttattg ataaagttaa caaacaaatt     540
   ccttacaatt ggaagcctta cgctattaaa aatattcctg taaataacaa aagtgtgaac     600
   gttggcttta tcgggattgt caccaaagac atcccaaacc ttgtcttacg taaaaattat     660
   gaacaatatg aatttttaga tgaagctgaa acaatcgtta aatacgccaa agaattacaa     720
   gctaaaaatg tcaaagctat tgtagttctc gcacatgtac ctgcaacaag taaaaatgat     780
   attgctgaag gtgaagcagc agaaatgatg aaaaaagtca atcaactctt ccctgaaaat     840
   agcgtagata ttgtctttgc tggacacaat catcaatata caaatggtct tgttggtaaa     900
   actcgtattg tacaagcgct ctctcaagga aaagcctatg ctgatgtacg tggtgtctta     960
   gatactgata cacaagattt cattgagacc ccttcagcta aagtaattgc agttgctcct    1020
   ggtaaaaaaa caggtagtgc cgatattcaa gccattgttg accaagctaa tactatcgtt    1080
   aaacaagtaa cagaagctaa aattggtact gccgaggtaa gtgtcatgat tacgcgttct    1140
   gttgatcaag ataatgttag tccggtaggc agcctcatca cagaggctca actagcaatt    1200
   gctcgaaaaa gctggccaga tatcgatttt gccatgacaa ataatggtgg cattcgtgct    1260
   gacttactca tcaaaccaga tggaacaatc acctggggag ctgcacaagc agttcaacct    1320
   tttggtaata tcttacaagt cgtcgaaatt actggtagag atctttataa agcactcaac    1380
   gaacaatacg accaaaaaca aaatttcttc cttcaaatag ctggtctgcg atacacttac    1440
   acagataata aagagggcgg ggaagaaaca ccatttaaag ttgtaaaagc ttataaatca    1500
   aatggtgagg aaatcaatcc tgatgcaaaa tacaaattag ttatcaatga ctttttattc    1560
   ggtggtggtg atggctttgc aagcttcaga aatgccaaac ttctaggagc cattaacccc    1620
   gatacagagg tatttatggc ctatatcact gatttagaaa aagctggtaa aaaagtgagc    1680
   gttccaaata ataaacctaa aatctatgtc actatgaaga tggttaatga aactattaca    1740
   caaaatgatg gtacacatag cattattaag aaactttatt tagatcgaca aggaaatatt    1800
   gtagcacaag agattgtatc agacacttta aaccaaacaa aatcaaaatc tacaaaaatc    1860
   aaccctgtaa ctacaattca caaaaaacaa ttacaccaat ttacagctat taaccctatg    1920
   agaaattatg gcaaaccatc aaactccact actgtaaaat caaaacaatt accaaaaaca    1980
   aactctgaat atggacaatc attccttatg tctgtctttg gtgttggact tataggaatt    2040
   gctttaaata caaagaaaaa acatatgaaa                                     2070
<210> 20
   <211> 690
   <212> PRT
   <213> group B streptococcus
<400> 20
   Met Lys Lys Lys Ile Ile Leu Lys Ser Ser Val Leu Gly Leu Val Ala
   1               5                   10                  15
   Gly Thr Ser Ile Met Phe Ser Ser Val Phe Ala Asp Gln Val Gly Val
               20                  25                  30
   Gln Val Ile Gly Val Asn Asp Phe His Gly Ala Leu Asp Asn Thr Gly
           35                  40                  45
   Thr Ala Asn Met Pro Asp Gly Lys Val Ala Asn Ala Gly Thr Ala Ala
       50                  55                  60
   Gln Leu Asp Ala Tyr Met Asp Asp Ala Gln Lys Asp Phe Lys Gln Thr
   65                  70                  75                  80
   Asn Pro Asn Gly Glu Ser Ile Arg Val Gln Ala Gly Asp Met Val Gly
                   85                  90                  95
   Ala Ser Pro Ala Asn Ser Gly Leu Leu Gln Asp Glu Pro Thr Val Lys
               100                 105                 110
   Asn Phe Asn Ala Met Asn Val Glu Tyr Gly Thr Leu Gly Asn His Glu
           115                 120                 125
   Phe Asp Glu Gly Leu Ala Glu Tyr Asn Arg Ile Val Thr Gly Lys Ala
       130                 135                 140
   Pro Ala Pro Asp Ser Asn Ile Asn Asn Ile Thr Lys Ser Tyr Pro His
   145                 150                 155                 160
   Glu Ala Ala Lys Gln Glu Ile Val Val Ala Asn Val Ile Asp Lys Val
                   165                 170                 175
   Asn Lys Gln Ile Pro Tyr Asn Trp Lys Pro Tyr Ala Ile Lys Asn Ile
               180                 185                 190
   Pro Val Asn Asn Lys Ser Val Asn Val Gly Phe Ile Gly Ile Val Thr
           195                 200                 205
   Lys Asp Ile Pro Asn Leu Val Leu Arg Lys Asn Tyr Glu Gln Tyr Glu
   210 215 220
   Phe Leu Asp Glu Ala Glu Thr Ile Val Lys Tyr Ala Lys Glu Leu Gln
   225 230 235 240
   Ala Lys Asn Val Lys Ala Ile Val Val Leu Ala His Val Pro Ala Thr
   245 250 255
   Ser Lys Asn Asp Ile Ala Glu Gly Glu Ala Ala Glu Met Met Lys Lys
   260 265 270
   Val Asn Gln Leu Phe Pro Glu Asn Ser Val Asp Ile Val Phe Ala Gly
   275 280 285
   His Asn His Gln Tyr Thr Asn Gly Leu Val Gly Lys Thr Arg Ile Val
   290 295 300
   Gln Ala Leu Ser Gln Gly Lys Ala Tyr Ala Asp Val Arg Gly Val Leu
   305 310 315 320
   Asp Thr Asp Thr Gln Asp Phe Ile Glu Thr Pro Ser Ala Lys Val Ile
   325 330 335
   Ala Val Ala Pro Gly Lys Lys Thr Gly Ser Ala Asp Ile Gln Ala Ile
   340 345 350
   Val Asp Gln Ala Asn Thr Ile Val Lys Gln Val Thr Glu Ala Lys Ile
   355 360 365
   Gly Thr Ala Glu Val Ser Val Met Ile Thr Arg Ser Val Asp Gln Asp
   370 375 380
   Asn Val Ser Pro Val Gly Ser Leu Ile Thr Glu Ala Gln Leu Ala Ile
   385 390 395 400
   Ala Arg Lys Ser Trp Pro Asp Ile Asp Phe Ala Met Thr Asn Asn Gly
   405 410 415
   Gly Ile Arg Ala Asp Leu Leu Ile Lys Pro Asp Gly Thr Ile Thr Trp
   420 425 430
   Gly Ala Ala Gln Ala Val Gln Pro Phe Gly Asn Ile Leu Gln Val Val
   435 440 445
   Glu Ile Thr Gly Arg Asp Leu Tyr Lys Ala Leu Asn Glu Gln Tyr Asp
   450 455 460
   Gln Lys Gln Asn Phe Phe Leu Gln Ile Ala Gly Leu Arg Tyr Thr Tyr
   465                 470                 475                 480
   Thr Asp Asn Lys Glu Gly Gly Glu Glu Thr Pro Phe Lys Val Val Lys
                   485                 490                 495
   Ala Tyr Lys Ser Asn Gly Glu Glu Ile Asn Pro Asp Ala Lys Tyr Lys
               500                 505                 510
   Leu Val Ile Asn Asp Phe Leu Phe Gly Gly Gly Asp Gly Phe Ala Ser
           515                 520                 525
   Phe Arg Asn Ala Lys Leu Leu Gly Ala Ile Asn Pro Asp Thr Glu Val
       530                 535                 540
   Phe Met Ala Tyr Ile Thr Asp Leu Glu Lys Ala Gly Lys Lys Val Ser
   545                 550                 555                 560
   Val Pro Asn Asn Lys Pro Lys Ile Tyr Val Thr Met Lys Met Val Asn
                   565                 570                 575
   Glu Thr Ile Thr Gln Asn Asp Gly Thr His Ser Ile Ile Lys Lys Leu
               580                 585                 590
   Tyr Leu Asp Arg Gln Gly Asn Ile Val Ala Gln Glu Ile Val Ser Asp
           595                 600                 605
   Thr Leu Asn Gln Thr Lys Ser Lys Ser Thr Lys Ile Asn Pro Val Thr
       610                 615                 620
   Thr Ile His Lys Lys Gln Leu His Gln Phe Thr Ala Ile Asn Pro Met
   625                 630                 635                 640
   Arg Asn Tyr Gly Lys Pro Ser Asn Ser Thr Thr Val Lys Ser Lys Gln
                   645                 650                 655
   Leu Pro Lys Thr Asn Ser Glu Tyr Gly Gln Ser Phe Leu Met Ser Val
               660                 665                 670
   Phe Gly Val Gly Leu Ile Gly Ile Ala Leu Asn Thr Lys Lys Lys His
           675                 680                 685
   Met Lys
       690
<210> 21
   <211> 1500
   <212> DNA
   <213> group B streptococcus
<400> 21
   atgaataaac gcgtaaaaat cgttgcaaca cttggtcctg cggttgaatt ccgtggtggt   60
   aagaagtttg gtgagtctgg atactggggt gaaagccttg acgtagaagc ttcagcagaa  120
   aaaattgctc aattgattaa agaaggtgct aacgttttcc gtttcaactt ctcacatgga  180
   gatcatgctg agcaaggagc tcgtatggct actgttcgta aagcagaaga gattgcagga  240
   caaaaagttg gcttcctcct tgatactaaa ggacctgaaa ttcgtacaga actttttgaa  300
   gatggtgcag atttccattc atatacaaca ggtacaaaat tacgtgttgc tactaagcaa  360
   ggtatcaaat caactccaga agtgattgca ttgaatgttg ctggtggact tgacatcttt  420
   gatgacgttg aagttggtaa gcaaatcctt gttgatgatg gtaaactagg tcttactgtg  480
   tttgcaaaag ataaagacac tcgtgaattt gaagtagttg ttgagaatga tggccttatt  540
   ggtaaacaaa aaggtgtaaa catcccttat actaaaattc ctttcccagc acttgcagaa  600
   cgcgataatg ctgatatccg ttttggactt gagcaaggac ttaactttat tgctatctca  660
   tttgtacgta ctgctaaaga tgttaatgaa gttcgtgcta tttgtgaaga aactggsmat  720
   ggacacgtta agttgtttgc taaaattgaa aatcaacaag gtatcgataa tattgatgag  780
   attatcgaag cagcagatgg tattatgatt gctcgtggtg atatgggtat cgaagttcca  840
   tttgaaatgg ttccagttta ccaaaaaatg atcattacta aagttaatgc agctggtaaa  900
   gcagttatta cagcaacaaa tatgcttgaa acaatgactg ataaaccacg tgcgactcgt  960
   tcagaagtat ctgatgtctt caatgctgtt attgatggta ctgatgctac aatgctttca 1020
   ggtgagtcag ctaatggtaa atacccagtt gagtcagttc gtacaatggc tactattgat 1080
   aaaaatgctc aaacattact caatgagtat ggtcgcttag actcatctgc attcccacgt 1140
   aataacaaaa ctgatgttat tgcatctgcg gttaaagatg caacacactc aatggatatc 1200
   aaacttgttg taacaattac tgaaacaggt aatacagctc gtgccatttc taaattccgt 1260
   ccagatgcag acattttggc tgttacattt gatgaaaaag tacaacgttc attgatgatt 1320
   aactggggtg ttatccctgt ccttgcagac aaaccagcat ctacagatga tatgtttgag 1380
   gttgcagaac gtgtagcact tgaagcagga tttgttgaat caggcgataa tatcgttatc 1440
   gttgcaggtg ttcctgtagg tacaggtgga actaacacaa tgcgtgttcg tactgttaaa 1500
<210> 22
   <211> 500
   <212> PRT
   <213> group B streptococcus
<220>
   <221> MISC_FEATURE
   <222> (240)..(240)
   <223> X is Asn or His
<400> 22
   Met Asn Lys Arg Val Lys Ile Val Ala Thr Leu Gly Pro Ala Val Glu
   1               5                   10                  15
   Phe Arg Gly Gly Lys Lys Phe Gly Glu Ser Gly Tyr Trp Gly Glu Ser
               20                  25                  30
   Leu Asp Val Glu Ala Ser Ala Glu Lys Ile Ala Gln Leu Ile Lys Glu
           35                  40                  45
   Gly Ala Asn Val Phe Arg Phe Asn Phe Ser His Gly Asp His Ala Glu
       50                  55                  60
   Gln Gly Ala Arg Met Ala Thr Val Arg Lys Ala Glu Glu Ile Ala Gly
   65                  70                  75                  80
   Gln Lys Val Gly Phe Leu Leu Asp Thr Lys Gly Pro Glu Ile Arg Thr
                   85                  90                  95
   Glu Leu Phe Glu Asp Gly Ala Asp Phe His Ser Tyr Thr Thr Gly Thr
               100                 105                 110
   Lys Leu Arg Val Ala Thr Lys Gln Gly Ile Lys Ser Thr Pro Glu Val
           115                 120                 125
   Ile Ala Leu Asn Val Ala Gly Gly Leu Asp Ile Phe Asp Asp Val Glu
       130                 135                 140
   Val Gly Lys Gln Ile Leu Val Asp Asp Gly Lys Leu Gly Leu Thr Val
   145                 150                 155                 160
   Phe Ala Lys Asp Lys Asp Thr Arg Glu Phe Glu Val Val Val Glu Asn
                   165                 170                 175
   Asp Gly Leu Ile Gly Lys Gln Lys Gly Val Asn Ile Pro Tyr Thr Lys
               180                 185                 190
   Ile Pro Phe Pro Ala Leu Ala Glu Arg Asp Asn Ala Asp Ile Arg Phe
           195                 200                 205
   Gly Leu Glu Gln Gly Leu Asn Phe Ile Ala Ile Ser Phe Val Arg Thr
       210                 215                 220
   Ala Lys Asp Val Asn Glu Val Arg Ala Ile Cys Glu Glu Thr Gly Xaa
   225                 230                 235                 240
   Gly His Val Lys Leu Phe Ala Lys Ile Glu Asn Gln Gln Gly Ile Asp
                   245                 250                 255
   Asn Ile Asp Glu Ile Ile Glu Ala Ala Asp Gly Ile Met Ile Ala Arg
               260                 265                 270
   Gly Asp Met Gly Ile Glu Val Pro Phe Glu Met Val Pro Val Tyr Gln
           275                 280                 285
   Lys Met Ile Ile Thr Lys Val Asn Ala Ala Gly Lys Ala Val Ile Thr
       290                 295                 300
   Ala Thr Asn Met Leu Glu Thr Met Thr Asp Lys Pro Arg Ala Thr Arg
   305                 310                 315                 320
   Ser Glu Val Ser Asp Val Phe Asn Ala Val Ile Asp Gly Thr Asp Ala
                   325                 330                 335
   Thr Met Leu Ser Gly Glu Ser Ala Asn Gly Lys Tyr Pro Val Glu Ser
               340                 345                 350
   Val Arg Thr Met Ala Thr Ile Asp Lys Asn Ala Gln Thr Leu Leu Asn
           355                 360                 365
   Glu Tyr Gly Arg Leu Asp Ser Ser Ala Phe Pro Arg Asn Asn Lys Thr
       370                 375                 380
   Asp Val Ile Ala Ser Ala Val Lys Asp Ala Thr His Ser Met Asp Ile
   385                 390                 395                 400
   Lys Leu Val Val Thr Ile Thr Glu Thr Gly Asn Thr Ala Arg Ala Ile
                   405                 410                 415
   Ser Lys Phe Arg Pro Asp Ala Asp Ile Leu Ala Val Thr Phe Asp Glu
               420                 425                 430
   Lys Val Gln Arg Ser Leu Met Ile Asn Trp Gly Val Ile Pro Val Leu
           435                 440                 445
   Ala Asp Lys Pro Ala Ser Thr Asp Asp Met Phe Glu Val Ala Glu Arg
       450                 455                 460
   Val Ala Leu Glu Ala Gly Phe Val Glu Ser Gly Asp Asn Ile Val Ile
   465                 470                 475                 480
   Val Ala Gly Val Pro Val Gly Thr Gly Gly Thr Asn Thr Met Arg Val
                   485                 490                 495
   Arg Thr Val Lys
               500
<210> 23
   <211> 720
   <212> DNA
   <213> group B streptococcus
<400> 23
   ttgtctgcta taatagacaa aaaggtggtg atatttatgt atttagcatt aatcggtgat     60
   atcattaatt caaaacagat acttgaacgt gaaactttcc aacagtcttt tcagcaacta    120
   atgaccgaac tatctgatgt atatggtgaa gagctgattt ctccattcac tattacagct    180
   ggtgatgaat ttcaagcttt attgaaacca tcaaaaaagg tatttcaaat tattgaccat    240
   attcaactag ctctaaaacc tgttaatgta aggttcggcc tcggtacagg aaacattata    300
   acatccatca attcaaatga aagtatcggt gctgatggtc ctgcctactg gcatgctcgc    360
   tcagctatta atcatataca tgataaaaat gattatggaa cagttcaagt agctatttgc    420
   cttgatgatg aagaccaaaa ccttgaatta acactaaata gtctcatttc agctggtgat    480
   tttatcaagt caaaatggac tacaaaccat tttcaaatgc ttgagcactt aatacttcaa    540
   gataattatc aagaacaatt tcaacatcaa aagttagccc aactggaaaa tattgaacct    600
   agtgcgctga ctaaacgcct taaagcaagc ggtctgaaga tttacttaag aacgagaaca    660
   caggcagccg atctattagt taaaagttgc actcaaacta aaggggaaag ctatgatttc    720
<210> 24
   <211> 240
   <212> PRT
   <213> group B streptococcus
<400> 24
   Met Ser Ala Ile Ile Asp Lys Lys Val Val Ile Phe Met Tyr Leu Ala
   1               5                   10                  15
   Leu Ile Gly Asp Ile Ile Asn Ser Lys Gln Ile Leu Glu Arg Glu Thr
               20                  25                  30
   Phe Gln Gln Ser Phe Gln Gln Leu Met Thr Glu Leu Ser Asp Val Tyr
           35                  40                  45
   Gly Glu Glu Leu Ile Ser Pro Phe Thr Ile Thr Ala Gly Asp Glu Phe
       50                  55                  60
   Gln Ala Leu Leu Lys Pro Ser Lys Lys Val Phe Gln Ile Ile Asp His
   65                  70                  75                  80
   Ile Gln Leu Ala Leu Lys Pro Val Asn Val Arg Phe Gly Leu Gly Thr
                   85                  90                  95
   Gly Asn Ile Ile Thr Ser Ile Asn Ser Asn Glu Ser Ile Gly Ala Asp
               100                 105                 110
   Gly Pro Ala Tyr Trp His Ala Arg Ser Ala Ile Asn His Ile His Asp
           115                 120                 125
   Lys Asn Asp Tyr Gly Thr Val Gln Val Ala Ile Cys Leu Asp Asp Glu
       130                 135                 140
   Asp Gln Asn Leu Glu Leu Thr Leu Asn Ser Leu Ile Ser Ala Gly Asp
   145                 150                 155                 160
   Phe Ile Lys Ser Lys Trp Thr Thr Asn His Phe Gln Met Leu Glu His
                   165                 170                 175
   Leu Ile Leu Gln Asp Asn Tyr Gln Glu Gln Phe Gln His Gln Lys Leu
               180                 185                 190
   Ala Gln Leu Glu Asn Ile Glu Pro Ser Ala Leu Thr Lys Arg Leu Lys
           195                 200                 205
   Ala Ser Gly Leu Lys Ile Tyr Leu Arg Thr Arg Thr Gln Ala Ala Asp
       210                 215                 220
   Leu Leu Val Lys Ser Cys Thr Gln Thr Lys Gly Gly Ser Tyr Asp Phe
   225                 230                 235                 240
<210> 25
   <211> 870
   <212> DNA
   <213> group B streptococcus
<400> 25
   atgttttata caattgaaga gctggtagag caagctaata gccaacataa gggtaacata    60
   gcagagctca tgatccaaac ggaaattgaa atgactggta gaagtcgtga agaaattcgt   120
   tatattatgt cccgaaatct tgaagtcatg aaagcttctg ttattgatgg attaacccct   180
   agtaaatcaa tcagtggttt aacaggcggt gatgctgtca agatggatca atatttacaa   240
   tcaggaaaaa ctatttcaga taccacaatc ctagctgccg ttaggaatgc tatggctgtt   300
   aatgagttaa atgctaagat gggactggtc tgtgcaacac caactgcagg tagtgcagga   360
   tgtttaccag ctgtgatttc tacagccatt gaaaagctta atttaacaga agaagagcaa   420
   cttgattttc tatttacagc cggcgcattt ggtctcgtca ttggtaataa tgcctctatc   480
   tcaggtgcag aaggaggttg ccaagctgaa gttgggtcag ctagtgctat ggctgcggct   540
   gctttagtta tggctgctgg aggtactcct ttccaagcta gccaagctat agcatttgtt   600
   attaaaaata tgcttggact tatctgtgac cctgttgcag gtttagttga agtcccttgt   660
   gtgaagcgga atgctcttgg atcaagtttt gcacttgttg ctgctgatat ggccttggct   720
   ggtattgaat cgcaaattcc agtagatgaa gttattgatg caatgtatca agttggatca   780
   agtttaccga ctgcttttcg tgagactgca gaaggaggac ttgctgccac gccgacagga   840
   agacgttata gtaaagaaat ttttggggaa                                      870
<210> 26
   <211> 290
   <212> PRT
   <213> group B streptococcus
<400> 26
   Met Phe Tyr Thr Ile Glu Glu Leu Val Glu Gln Ala Asn Ser Gln His
   1               5                   10                  15
   Lys Gly Asn Ile Ala Glu Leu Met Ile Gln Thr Glu Ile Glu Met Thr
               20                  25                  30
   Gly Arg Ser Arg Glu Glu Ile Arg Tyr Ile Met Ser Arg Asn Leu Glu
           35                  40                  45
   Val Met Lys Ala Ser Val Ile Asp Gly Leu Thr Pro Ser Lys Ser Ile
       50                  55                  60
   Ser Gly Leu Thr Gly Gly Asp Ala Val Lys Met Asp Gln Tyr Leu Gln
   65                  70                  75                  80
   Ser Gly Lys Thr Ile Ser Asp Thr Thr Ile Leu Ala Ala Val Arg Asn
                   85                  90                  95
   Ala Met Ala Val Asn Glu Leu Asn Ala Lys Met Gly Leu Val Cys Ala
   100 105 110
   Thr Pro Thr Ala Gly Ser Ala Gly Cys Leu Pro Ala Val Ile Ser Thr
   115 120 125
   Ala Ile Glu Lys Leu Asn Leu Thr Glu Glu Glu Gln Leu Asp Phe Leu
   130 135 140
   Phe Thr Ala Gly Ala Phe Gly Leu Val Ile Gly Asn Asn Ala Ser Ile
   145 150 155 160
   Ser Gly Ala Glu Gly Gly Cys Gln Ala Glu Val Gly Ser Ala Ser Ala
   165 170 175
   Met Ala Ala Ala Ala Leu Val Met Ala Ala Gly Gly Thr Pro Phe Gln
   180 185 190
   Ala Ser Gln Ala Ile Ala Phe Val Ile Lys Asn Met Leu Gly Leu Ile
   195 200 205
   Cys Asp Pro Val Ala Gly Leu Val Glu Val Pro Cys Val Lys Arg Asn
   210 215 220
   Ala Leu Gly Ser Ser Phe Ala Leu Val Ala Ala Asp Met Ala Leu Ala
   225 230 235 240
   Gly Ile Glu Ser Gln Ile Pro Val Asp Glu Val Ile Asp Ala Met Tyr
   245 250 255
   Gln Val Gly Ser Ser Leu Pro Thr Ala Phe Arg Glu Thr Ala Glu Gly
   260 265 270
   Gly Leu Ala Ala Thr Pro Thr Gly Arg Arg Tyr Ser Lys Glu Ile Phe
   275 280 285
   Gly Glu
   290
<210> 27
   <211> 2193
   <212> DNA
   <213> group B streptococcus
<400> 27
   atgagcgtat atgttagtgg aataggaatt atttcttctt tgggaaagaa ttatagcgag 60
   cataaacagc atctcttcga cttaaaagaa ggaatttcta aacatttata taaaaatcac 120
   gactctattt tagaatctta tacaggaagc ataactagtg acccagaggt tcctgagcaa    180
   tacaaagatg agacacgtaa ttttaaattt gcttttaccg cttttgaaga ggctcttgct    240
   tcttcaggtg ttaatttaaa agcttatcat aatattgctg tgtgtttagg gacctcactt    300
   gggggaaaga gtgctggtca aaatgccttg tatcaatttg aagaaggaga gcgtcaagta    360
   gatgctagtt tattagaaaa agcatctgtt taccatattg ctgatgaatt gatggcttat    420
   catgatattg tgggagcttc gtatgttatt tcaaccgcct gttctgcaag taataatgcc    480
   gtaatattag gaacacaatt acttcaagat ggcgattgtg atttagctat ttgtggtggc    540
   tgtgatgagt taagtgatat ttctttagca ggcttcacat cactaggagc tattaataca    600
   gaaatggcat gtcagcccta ttcttctgga aaaggaatca atttgggtga gggcgctggt    660
   tttgttgttc ttgtcaaaga tcagtcctta gctaaatatg gaaaaattat cggtggtctt    720
   attacttcag atggttatca tataacagca cctaagccaa caggtgaagg ggcggcacag    780
   attgcaaagc agctagtgac tcaagcaggt attgactaca gtgagattga ctatattaac    840
   ggtcacggta caggtactca agctaatgat aaaatggaaa aaaatatgta tggtaagttt    900
   ttcccgacaa cgacattgat cagcagtacc aaggggcaaa cgggtcatac tctaggggct    960
   gcaggtatta tcgaattgat taattgttta gcggcaatag aggaacagac tgtaccagca   1020
   actaaaaatg agattgggat agaaggtttt ccagaaaatt ttgtctatca tcaaaagaga   1080
   gaatacccaa taagaaatgc tttaaatttt tcgtttgctt ttggtggaaa taatagtggt   1140
   gtcttattgt catctttaga ttcacctcta gaaacattac ctgctagaga aaatcttaaa   1200
   atggctatct tatcatctgt tgcttccatt tctaagaatg aatcactttc tataacctat   1260
   gaaaaagttg ctagtaattt caacgacttt gaagcattac gctttaaagg ggctagacca   1320
   cccaaaactg tcaacccagc acaatttagg aaaatggatg atttttccaa aatggttgcc   1380
   gtaacaacag ctcaagcact aatagaaagc aatattaatc taaaaaaaca agatacttca   1440
   aaagtaggaa ttgtatttac aacactttct ggaccagttg aggttgttga aggtattgaa   1500
   aagcaaatca caacagaagg atatgcacat gtttctgctt cacgattccc gtttacagta   1560
   atgaatgcag cagctggtat gctttctatc atttttaaaa taacaggtcc tttatctgtc   1620
   atttcgacaa atagtggagc gcttgatggt atacaatatg ccaaggaaat gatgcgtaac   1680
   gataatctag actatgtgat tcttgtttct gctaatcagt ggacagacat gagttttatg   1740
   tggtggcaac aattaaacta tgatagtcaa atgtttgtcg gttctgatta ttgttcagca   1800
   caagtcctct ctcgtcaagc attggataat tctcctataa tattaggtag taaacaatta   1860
   aaatatagcc ataaaacatt cacagatgtg atgactattt ttgatgctgc gcttcaaaat   1920
   ttattatcag acttaggact aaccataaaa gatatcaaag gtttcgtttg gaatgagcgg   1980
   aagaaggcag ttagttcaga ttatgatttc ttagcgaact tgtctgagta ttataatatg 2040
   ccaaaccttg cttctggtca gtttggattt tcatctaatg gtgctggtga agaactggac 2100
   tatactgtta atgaaagtat agaaaagggc tattatttag tcctatctta ttcgatcttc 2160
   ggtggtatct cttttgctat tattgaaaaa agg 2193
<210> 28
   <211> 731
   <212> PRT
   <213> group B streptococcus
<400> 28
   Met Ser Val Tyr Val Ser Gly Ile Gly Ile Ile Ser Ser Leu Gly Lys
   1               5                   10                  15
   Asn Tyr Ser Glu His Lys Gln His Leu Phe Asp Leu Lys Glu Gly Ile
               20                  25                  30
   Ser Lys His Leu Tyr Lys Asn His Asp Ser Ile Leu Glu Ser Tyr Thr
           35                  40                  45
   Gly Ser Ile Thr Ser Asp Pro Glu Val Pro Glu Gln Tyr Lys Asp Glu
       50                  55                  60
   Thr Arg Asn Phe Lys Phe Ala Phe Thr Ala Phe Glu Glu Ala Leu Ala
   65                  70                  75                  80
   Ser Ser Gly Val Asn Leu Lys Ala Tyr His Asn Ile Ala Val Cys Leu
                   85                  90                  95
   Gly Thr Ser Leu Gly Gly Lys Ser Ala Gly Gln Asn Ala Leu Tyr Gln
               100                 105                 110
   Phe Glu Glu Gly Glu Arg Gln Val Asp Ala Ser Leu Leu Glu Lys Ala
           115                 120                 125
   Ser Val Tyr His Ile Ala Asp Glu Leu Met Ala Tyr His Asp Ile Val
       130                 135                 140
   Gly Ala Ser Tyr Val Ile Ser Thr Ala Cys Ser Ala Ser Asn Asn Ala
   145                 150                 155                 160
   Val Ile Leu Gly Thr Gln Leu Leu Gln Asp Gly Asp Cys Asp Leu Ala
                   165                 170                 175
   Ile Cys Gly Gly Cys Asp Glu Leu Ser Asp Ile Ser Leu Ala Gly Phe
   180 185 190
   Thr Ser Leu Gly Ala Ile Asn Thr Glu Met Ala Cys Gln Pro Tyr Ser
   195 200 205
   Ser Gly Lys Gly Ile Asn Leu Gly Glu Gly Ala Gly Phe Val Val Leu
   210 215 220
   Val Lys Asp Gln Ser Leu Ala Lys Tyr Gly Lys Ile Ile Gly Gly Leu
   225 230 235 240
   Ile Thr Ser Asp Gly Tyr His Ile Thr Ala Pro Lys Pro Thr Gly Glu
   245 250 255
   Gly Ala Ala Gln Ile Ala Lys Gln Leu Val Thr Gln Ala Gly Ile Asp
   260 265 270
   Tyr Ser Glu Ile Asp Tyr Ile Asn Gly His Gly Thr Gly Thr Gln Ala
   275 280 285
   Asn Asp Lys Met Glu Lys Asn Met Tyr Gly Lys Phe Phe Pro Thr Thr
   290 295 300
   Thr Leu Ile Ser Ser Thr Lys Gly Gln Thr Gly His Thr Leu Gly Ala
   305 310 315 320
   Ala Gly Ile Ile Glu Leu Ile Asn Cys Leu Ala Ala Ile Glu Glu Gln
   325 330 335
   Thr Val Pro Ala Thr Lys Asn Glu Ile Gly Ile Glu Gly Phe Pro Glu
   340 345 350
   Asn Phe Val Tyr His Gln Lys Arg Glu Tyr Pro Ile Arg Asn Ala Leu
   355 360 365
   Asn Phe Ser Phe Ala Phe Gly Gly Asn Asn Ser Gly Val Leu Leu Ser
   370 375 380
   Ser Leu Asp Ser Pro Leu Glu Thr Leu Pro Ala Arg Glu Asn Leu Lys
   385 390 395 400
   Met Ala Ile Leu Ser Ser Val Ala Ser Ile Ser Lys Asn Glu Ser Leu
   405 410 415
   Ser Ile Thr Tyr Glu Lys Val Ala Ser Asn Phe Asn Asp Phe Glu Ala
   420 425 430
   Leu Arg Phe Lys Gly Ala Arg Pro Pro Lys Thr Val Asn Pro Ala Gln
   435 440 445
   Phe Arg Lys Met Asp Asp Phe Ser Lys Met Val Ala Val Thr Thr Ala
   450 455 460
   Gln Ala Leu Ile Glu Ser Asn Ile Asn Leu Lys Lys Gln Asp Thr Ser
   465 470 475 480
   Lys Val Gly Ile Val Phe Thr Thr Leu Ser Gly Pro Val Glu Val Val
   485 490 495
   Glu Gly Ile Glu Lys Gln Ile Thr Thr Glu Gly Tyr Ala His Val Ser
   500 505 510
   Ala Ser Arg Phe Pro Phe Thr Val Met Asn Ala Ala Ala Gly Met Leu
   515 520 525
   Ser Ile Ile Phe Lys Ile Thr Gly Pro Leu Ser Val Ile Ser Thr Asn
   530 535 540
   Ser Gly Ala Leu Asp Gly Ile Gln Tyr Ala Lys Glu Met Met Arg Asn
   545 550 555 560
   Asp Asn Leu Asp Tyr Val Ile Leu Val Ser Ala Asn Gln Trp Thr Asp
   565 570 575
   Met Ser Phe Met Trp Trp Gln Gln Leu Asn Tyr Asp Ser Gln Met Phe
   580 585 590
   Val Gly Ser Asp Tyr Cys Ser Ala Gln Val Leu Ser Arg Gln Ala Leu
   595 600 605
   Asp Asn Ser Pro Ile Ile Leu Gly Ser Lys Gln Leu Lys Tyr Ser His
   610 615 620
   Lys Thr Phe Thr Asp Val Met Thr Ile Phe Asp Ala Ala Leu Gln Asn
   625 630 635 640
   Leu Leu Ser Asp Leu Gly Leu Thr Ile Lys Asp Ile Lys Gly Phe Val
   645 650 655
   Trp Asn Glu Arg Lys Lys Ala Val Ser Ser Asp Tyr Asp Phe Leu Ala
   660 665 670
   Asn Leu Ser Glu Tyr Tyr Asn Met Pro Asn Leu Ala Ser Gly Gln Phe
               675                 680                 685
   Gly Phe Ser Ser Asn Gly Ala Gly Glu Glu Leu Asp Tyr Thr Val Asn
       690                 695                 700
   Glu Ser Ile Glu Lys Gly Tyr Tyr Leu Val Leu Ser Tyr Ser Ile Phe
   705                 710                 715                 720
   Gly Gly Ile Ser Phe Ala Ile Ile Glu Lys Arg
                   725                 730
<210> 29
   <211> 900
   <212> DNA
   <213> group B streptococcus
<400> 29
   atgaaaatag atgacctaag aaaaagcgac aatgttgaag atcgtcgctc cagtagcgga      60
   ggttcattct ctagcggagg aagtggatta ccgattcttc aacttttatt gctgcgaggg     120
   agttggaaaa ccaagcttgt ggttttaatc atcttactgc tacttggcgg agggggacta     180
   accagcattt ttaatgactc atcctcacct tctagttacc aatctcagaa tgtctcacgt     240
   tctgttgata atagcgcaac gagagaacaa atcgatttcg ttaataaagt ccttggctca     300
   actgaggatt tctggtcaca agaattccaa acccaaggtt ttggaaatta taaggaacca     360
   aaacttgttc tttacaccaa ttcaattcaa acaggttgtg gtataggtga atctgcttca     420
   ggaccatttt attgttcagc agataaaaaa atctatcttg atatttcttt ttacaatgaa     480
   ttatcacata aatatggtgc tactggtgat tttgctatgg cctacgtcat cgcccacgaa     540
   gttggtcacc acattcaaac agagttaggc attatggata agtataatag aatgcgacac     600
   ggacttacta agaaagaagc aaatgcttta aatgttcggc tagaacttca agcagattat     660
   tatgcagggg tatgggctca ctacatcagg ggaaaaaatc tcttagaaca aggagacttt     720
   gaagaggcca tgaatgctgc ccacgccgtc ggagacgata cccttcagaa agaaacctac     780
   ggaaaattag tgcctgatag ctttacccat ggaacagctg aacaacgcca acgttggttt     840
   aacaaaggct ttcaatatgg tgacatccaa cacggtgata ctttctccgt agaacatcta     900
<210> 30
   <211> 300
   <212> PRT
   <213> group B streptococcus
<400> 30
   Met Lys Ile Asp Asp Leu Arg Lys Ser Asp Asn Val Glu Asp Arg Arg
   1               5                   10                  15
   Ser Ser Ser Gly Gly Ser Phe Ser Ser Gly Gly Ser Gly Leu Pro Ile
               20                  25                  30
   Leu Gln Leu Leu Leu Leu Arg Gly Ser Trp Lys Thr Lys Leu Val Val
           35                  40                  45
   Leu Ile Ile Leu Leu Leu Leu Gly Gly Gly Gly Leu Thr Ser Ile Phe
       50                  55                  60
   Asn Asp Ser Ser Ser Pro Ser Ser Tyr Gln Ser Gln Asn Val Ser Arg
   65                  70                  75                  80
   Ser Val Asp Asn Ser Ala Thr Arg Glu Gln Ile Asp Phe Val Asn Lys
                   85                  90                  95
   Val Leu Gly Ser Thr Glu Asp Phe Trp Ser Gln Glu Phe Gln Thr Gln
               100                 105                 110
   Gly Phe Gly Asn Tyr Lys Glu Pro Lys Leu Val Leu Tyr Thr Asn Ser
           115                 120                 125
   Ile Gln Thr Gly Cys Gly Ile Gly Glu Ser Ala Ser Gly Pro Phe Tyr
       130                 135                 140
   Cys Ser Ala Asp Lys Lys Ile Tyr Leu Asp Ile Ser Phe Tyr Asn Glu
   145                 150                 155                 160
   Leu Ser His Lys Tyr Gly Ala Thr Gly Asp Phe Ala Met Ala Tyr Val
                   165                 170                 175
   Ile Ala His Glu Val Gly His His Ile Gln Thr Glu Leu Gly Ile Met
               180                 185                 190
   Asp Lys Tyr Asn Arg Met Arg His Gly Leu Thr Lys Lys Glu Ala Asn
           195                 200                 205
   Ala Leu Asn Val Arg Leu Glu Leu Gln Ala Asp Tyr Tyr Ala Gly Val
       210                 215                 220
   Trp Ala His Tyr Ile Arg Gly Lys Asn Leu Leu Glu Gln Gly Asp Phe
   225                 230                 235                 240
   Glu Glu Ala Met Asn Ala Ala His Ala Val Gly Asp Asp Thr Leu Gln
   245 250 255
   Lys Glu Thr Tyr Gly Lys Leu Val Pro Asp Ser Phe Thr His Gly Thr
   260 265 270
   Ala Glu Gln Arg Gln Arg Trp Phe Asn Lys Gly Phe Gln Tyr Gly Asp
   275 280 285
   Ile Gln His Gly Asp Thr Phe Ser Val Glu His Leu
   290 295 300
<210> 31
   <211> 783
   <212> DNA
   <213> group B streptococcus
<400> 31
   atgaaaagat tacataaact gtttataacc gtaattgcta cattaggtat gttgggggta      60
   atgacctttg gtcttccaac gcagccgcaa aacgtaacgc cgatagtaca tgctgatgtc     120
   aattcatctg ttgatacgag ccaggaattt caaaataatt taaaaaatgc tattggtaac     180
   ctaccatttc aatatgttaa tggtatttat gaattaaata ataatcagac aaatttaaat     240
   gctgatgtca atgttaaagc gtatgttcaa aatacaattg acaatcaaca aagactatca     300
   actgctaatg caatgcttga tagaaccatt cgtcaatatc aaaatcgcag agataccact     360
   cttcccgatg caaattggaa accattaggt tggcatcaag tagctactaa tgaccattat     420
   ggacatgcag tcgacaaggg gcatttaatt gcctatgctt tagctggaaa tttcaaaggt     480
   tgggatgctt ccgtgtcaaa tcctcaaaat gttgtcacac aaacagctca ttccaaccaa     540
   tcaaatcaaa aaatcaatcg tggacaaaat tattatgaaa gcttagttcg taaggcggtt     600
   gaccaaaaca aacgtgttcg ttaccgtgta actccattgt accgtaatga tactgattta     660
   gttccatttg caatgcacct agaagctaaa tcacaagatg gcacattaga atttaatgtt     720
   gctattccaa acacacaagc atcatacact atggattatg caacaggaga aataacacta     780
   aat                                                                   783
<210> 32
   <211> 261
   <212> PRT
   <213> group B streptococcus
<400> 32
   Met Lys Arg Leu His Lys Leu Phe Ile Thr Val Ile Ala Thr Leu Gly
   1               5                   10                  15
   Met Leu Gly Val Met Thr Phe Gly Leu Pro Thr Gln Pro Gln Asn Val
               20                  25                  30
   Thr Pro Ile Val His Ala Asp Val Asn Ser Ser Val Asp Thr Ser Gln
           35                  40                  45
   Glu Phe Gln Asn Asn Leu Lys Asn Ala Ile Gly Asn Leu Pro Phe Gln
       50                  55                  60
   Tyr Val Asn Gly Ile Tyr Glu Leu Asn Asn Asn Gln Thr Asn Leu Asn
   65                  70                  75                  80
   Ala Asp Val Asn Val Lys Ala Tyr Val Gln Asn Thr Ile Asp Asn Gln
                   85                  90                  95
   Gln Arg Leu Ser Thr Ala Asn Ala Met Leu Asp Arg Thr Ile Arg Gln
               100                 105                 110
   Tyr Gln Asn Arg Arg Asp Thr Thr Leu Pro Asp Ala Asn Trp Lys Pro
           115                 120                 125
   Leu Gly Trp His Gln Val Ala Thr Asn Asp His Tyr Gly His Ala Val
       130                 135                 140
   Asp Lys Gly His Leu Ile Ala Tyr Ala Leu Ala Gly Asn Phe Lys Gly
   145                 150                 155                 160
   Trp Asp Ala Ser Val Ser Asn Pro Gln Asn Val Val Thr Gln Thr Ala
                   165                 170                 175
   His Ser Asn Gln Ser Asn Gln Lys Ile Asn Arg Gly Gln Asn Tyr Tyr
               180                 185                 190
   Glu Ser Leu Val Arg Lys Ala Val Asp Gln Asn Lys Arg Val Arg Tyr
           195                 200                 205
   Arg Val Thr Pro Leu Tyr Arg Asn Asp Thr Asp Leu Val Pro Phe Ala
       210                 215                 220
   Met His Leu Glu Ala Lys Ser Gln Asp Gly Thr Leu Glu Phe Asn Val
   225                 230                 235                 240
   Ala Ile Pro Asn Thr Gln Ala Ser Tyr Thr Met Asp Tyr Ala Thr Gly
                   245                 250                 255
   Glu Ile Thr Leu Asn
   260
<210> 33
   <211> 1242
   <212> DNA
   <213> group B streptococcus
<400> 33
   atgagtaaac gacaaaattt aggaattagt aaaaaaggag caattatatc agggctctca      60
   gtggcactaa ttgtagtaat aggtggcttt ttatgggtac aatctcaacc taataagagt     120
   gcagtaaaaa ctaactacaa agtttttaat gttagagaag gaagtgtttc gtcctcaact     180
   cttttgacag gaaaagctaa ggctaatcaa gaacagtatg tgtattttga tgctaataaa     240
   ggtaatcgag caactgtcac agttaaagtg ggtgataaaa tcacagctgg tcagcagtta     300
   gttcaatatg atacaacaac tgcacaagca gcctacgaca ctgctaatcg tcaattaaat     360
   aaagtagcgc gtcagattaa taatctaaag acaacaggaa gtcttccagc tatggaatca     420
   agtgatcaat cttcttcatc atcacaagga caagggactc aatcgactag tggtgcgacg     480
   aatcgtctac agcaaaatta tcaaagtcaa gctaatgctt catacaacca acaacttcaa     540
   gatttgaatg atgcttatgc agatgcacag gcagaagtaa ataaagcaca aaaagcattg     600
   aatgatactg ttattacaag tgacgtatca gggacagttg ttgaagttaa tagtgatatt     660
   gatccagctt caaaaactag tcaagtactt gtccatgtag caactgaagg taaactccaa     720
   gtacaaggaa cgatgagtga gtatgatttg gctaatgtta aaaaagacca ggctgttaaa     780
   ataaaatcta aggtctatcc tgacaaggaa tgggaaggta aaatttcata tatctcaaat     840
   tatccagaag cagaagcaaa caacaatgac tctaataacg gctctagtgc tgtaaattat     900
   aaatataaag tagatattac tagccctctc gatgcattaa aacaaggttt taccgtatca     960
   gttgaagtag ttaatggaga taagcacctt attgtcccta caagttctgt gataaacaaa    1020
   gataataaac actttgtttg ggtatacaat gattctaatc gtaaaatttc caaagttgaa    1080
   gtcaaaattg gtaaagctga tgctaagaca caagaaattt tatcaggttt gaaagcagga    1140
   caaatcgtgg ttactaatcc aagtaaaacc ttcaaggatg ggcaaaaaat tgataatatt    1200
   gaatcaatcg atcttaactc taataagaaa tcagaggtga aa                       1242
<210> 34
   <211> 414
   <212> PRT
   <213> group B streptococcus
<400> 34
   Met Ser Lys Arg Gln Asn Leu Gly Ile Ser Lys Lys Gly Ala Ile Ile
   1 5 10 15
   Ser Gly Leu Ser Val Ala Leu Ile Val Val Ile Gly Gly Phe Leu Trp
   20 25 30
   Val Gln Ser Gln Pro Asn Lys Ser Ala Val Lys Thr Asn Tyr Lys Val
   35 40 45
   Phe Asn Val Arg Glu Gly Ser Val Ser Ser Ser Thr Leu Leu Thr Gly
   50 55 60
   Lys Ala Lys Ala Asn Gln Glu Gln Tyr Val Tyr Phe Asp Ala Asn Lys
   65 70 75 80
   Gly Asn Arg Ala Thr Val Thr Val Lys Val Gly Asp Lys Ile Thr Ala
   85 90 95
   Gly Gln Gln Leu Val Gln Tyr Asp Thr Thr Thr Ala Gln Ala Ala Tyr
   100 105 110
   Asp Thr Ala Asn Arg Gln Leu Asn Lys Val Ala Arg Gln Ile Asn Asn
   115 120 125
   Leu Lys Thr Thr Gly Ser Leu Pro Ala Met Glu Ser Ser Asp Gln Ser
   130 135 140
   Ser Ser Ser Ser Gln Gly Gln Gly Thr Gln Ser Thr Ser Gly Ala Thr
   145 150 155 160
   Asn Arg Leu Gln Gln Asn Tyr Gln Ser Gln Ala Asn Ala Ser Tyr Asn
   165 170 175
   Gln Gln Leu Gln Asp Leu Asn Asp Ala Tyr Ala Asp Ala Gln Ala Glu
   180 185 190
   Val Asn Lys Ala Gln Lys Ala Leu Asn Asp Thr Val Ile Thr Ser Asp
   195 200 205
   Val Ser Gly Thr Val Val Glu Val Asn Ser Asp Ile Asp Pro Ala Ser
   210 215 220
   Lys Thr Ser Gln Val Leu Val His Val Ala Thr Glu Gly Lys Leu Gln
   225 230 235 240
   Val Gln Gly Thr Met Ser Glu Tyr Asp Leu Ala Asn Val Lys Lys Asp
   245 250 255
   Gln Ala Val Lys Ile Lys Ser Lys Val Tyr Pro Asp Lys Glu Trp Glu
               260                 265                 270
   Gly Lys Ile Ser Tyr Ile Ser Asn Tyr Pro Glu Ala Glu Ala Asn Asn
           275                 280                 285
   Asn Asp Ser Asn Asn Gly Ser Ser Ala Val Asn Tyr Lys Tyr Lys Val
       290                 295                 300
   Asp Ile Thr Ser Pro Leu Asp Ala Leu Lys Gln Gly Phe Thr Val Ser
   305                 310                 315                     320
   Val Glu Val Val Asn Gly Asp Lys His Leu Ile Val Pro Thr Ser Ser
                   325                 330                 335
   Val Ile Asn Lys Asp Asn Lys His Phe Val Trp Val Tyr Asn Asp Ser
               340                 345                 350
   Asn Arg Lys Ile Ser Lys Val Glu Val Lys Ile Gly Lys Ala Asp Ala
           355                 360                 365
   Lys Thr Gln Glu Ile Leu Ser Gly Leu Lys Ala Gly Gln Ile Val Val
       370                 375                 380
   Thr Asn Pro Ser Lys Thr Phe Lys Asp Gly Gln Lys Ile Asp Asn Ile
   385                 390                 395                     400
   Glu Ser Ile Asp Leu Asn Ser Asn Lys Lys Ser Glu Val Lys
                   405                 410
<210> 35
   <211> 930
   <212> DNA
   <213> group B streptococcus
<400> 35
   atgaaaaaaa ttggaattat tgtcctcaca ctactgacct tctttttggt atcttgcgga     60
   caacaaacta aacaagaaag cactaaaaca actatttcta aaatgcctaa aattgaaggc    120
   ttcacctatt atggaaaaat tcctgaaaat ccgaaaaaag taattaattt tacatattct    180
   tacactgggt atttattaaa actaggtgtt aatgtttcaa gttacagttt agacttagaa    240
   aaagatagcc ccgtttttgg taaacaactg aaagaagcta aaaaattaac tgctgatgat    300
   acagaagcta ttgccgcaca aaaacctgat ttaatcatgg ttttcgatca agatccaaac    360
   atcaatactc tgaaaaaaat tgcaccaact ttagttatta aatatggtgc acaaaattat    420
   ttagatatga tgccagcctt ggggaaagta ttcggtaaag aaaaagaagc taatcagtgg    480
   gttagccaat ggaaaactaa aactctcgct gtcaaaaaag atttacacca tatcttaaag    540
   cctaacacta cttttactat tatggatttt tatgataaaa atatctattt atatggtaat    600
   aattttggac gcggtggaga actaatctat gattcactag gttatgctgc cccagaaaaa    660
   gtcaaaaaag atgtctttaa aaaagggtgg tttaccgttt cgcaagaagc aatcggtgat    720
   tacgttggag attatgccct tgttaatata aacaaaacga ctaaaaaagc agcttcatca    780
   cttaaagaaa gtgatgtctg gaagaattta ccagctgtca aaaaagggca catcatagaa    840
   agtaactacg acgtgtttta tttctctgac cctctatctt tagaagctca attaaaatca    900
   tttacaaagg ctatcaaaga aaatacaaat                                     930
<210> 36
   <211> 310
   <212> PRT
   <213> group B streptococcus
<400> 36
   Met Lys Lys Ile Gly Ile Ile Val Leu Thr Leu Leu Thr Phe Phe Leu
   1               5                   10                  15
   Val Ser Cys Gly Gln Gln Thr Lys Gln Glu Ser Thr Lys Thr Thr Ile
               20                  25                  30
   Ser Lys Met Pro Lys Ile Glu Gly Phe Thr Tyr Tyr Gly Lys Ile Pro
           35                  40                  45
   Glu Asn Pro Lys Lys Val Ile Asn Phe Thr Tyr Ser Tyr Thr Gly Tyr
       50                  55                  60
   Leu Leu Lys Leu Gly Val Asn Val Ser Ser Tyr Ser Leu Asp Leu Glu
   65                  70                  75                  80
   Lys Asp Ser Pro Val Phe Gly Lys Gln Leu Lys Glu Ala Lys Lys Leu
                   85                  90                  95
   Thr Ala Asp Asp Thr Glu Ala Ile Ala Ala Gln Lys Pro Asp Leu Ile
               100                 105                 110
   Met Val Phe Asp Gln Asp Pro Asn Ile Asn Thr Leu Lys Lys Ile Ala
           115                 120                 125
   Pro Thr Leu Val Ile Lys Tyr Gly Ala Gln Asn Tyr Leu Asp Met Met
       130                 135                 140
   Pro Ala Leu Gly Lys Val Phe Gly Lys Glu Lys Glu Ala Asn Gln Trp
   145                 150                 155                 160
   Val Ser Gln Trp Lys Thr Lys Thr Leu Ala Val Lys Lys Asp Leu His
                   165                 170                 175
   His Ile Leu Lys Pro Asn Thr Thr Phe Thr Ile Met Asp Phe Tyr Asp
               180                 185                 190
   Lys Asn Ile Tyr Leu Tyr Gly Asn Asn Phe Gly Arg Gly Gly Glu Leu
           195                 200                 205
   Ile Tyr Asp Ser Leu Gly Tyr Ala Ala Pro Glu Lys Val Lys Lys Asp
       210                 215                 220
   Val Phe Lys Lys Gly Trp Phe Thr Val Ser Gln Glu Ala Ile Gly Asp
   225                 230                 235                 240
   Tyr Val Gly Asp Tyr Ala Leu Val Asn Ile Asn Lys Thr Thr Lys Lys
                   245                 250                 255
   Ala Ala Ser Ser Leu Lys Glu Ser Asp Val Trp Lys Asn Leu Pro Ala
               260                 265                 270
   Val Lys Lys Gly His Ile Ile Glu Ser Asn Tyr Asp Val Phe Tyr Phe
           275                 280                 285
   Ser Asp Pro Leu Ser Leu Glu Ala Gln Leu Lys Ser Phe Thr Lys Ala
       290                 295                 300
   Ile Lys Glu Asn Thr Asn
   305                 310
<210> 37
   <211> 576
   <212> DNA
   <213> group B streptococcus
<400> 37
<210> 38
   <211> 192
   <212> PRT
   <213> group B streptococcus
<400> 38
   Met Lys Val Lys Asn Lys Ile Leu Thr Met Val Ala Leu Thr Val Leu
   1               5                   10                  15
   Thr Cys Ala Thr Tyr Ser Ser Ile Gly Tyr Ala Asp Thr Ser Asp Lys
               20                  25                  30
   Asn Thr Asp Thr Ser Val Val Thr Thr Thr Leu Ser Glu Glu Lys Arg
           35                  40                  45
   Ser Asp Glu Leu Asp Gln Ser Ser Thr Gly Ser Ser Ser Glu Asn Glu
       50                  55                  60
   Ser Ser Ser Ser Ser Glu Pro Glu Thr Asn Pro Ser Thr Asn Pro Pro
   65                  70                  75                  80
   Thr Thr Glu Pro Ser Gln Pro Ser Pro Ser Glu Glu Asn Lys Pro Asp
                   85                  90                  95
   Gly Arg Thr Lys Thr Glu Ile Gly Asn Asn Lys Asp Ile Ser Ser Gly
               100                 105                 110
   Thr Lys Val Leu Ile Ser Glu Asp Ser Ile Lys Asn Phe Ser Lys Ala
           115                 120                 125
   Ser Ser Asp Gln Glu Glu Val Asp Arg Asp Glu Ser Ser Ser Ser Lys
       130                 135                 140
   Ala Asn Asp Gly Lys Lys Gly His Ser Lys Pro Lys Lys Glu Leu Pro
   145                 150                 155                 160
   Lys Thr Gly Asp Ser His Ser Asp Thr Val Ile Ala Ser Thr Gly Gly
                   165                 170                 175
   Ile Ile Leu Leu Ser Leu Ser Phe Tyr Asn Lys Lys Met Lys Leu Tyr
               180                 185                 190
<210> 39
   <211> 924
   <212> DNA
   <213> group B streptococcus
<400> 39
   atgaaaagga tacggaaaag ccttattttt gttctcggag tagttaccct aatttgctta     60
   tgtgcttgta ctaaacaaag ccagcaaaaa aatggcttgt cagtagtgac tagcttttat    120
   ccagtatatt ccattacaaa agcagtttct ggtgatttga atgatattaa aatgattcga    180
   tcacagtcag gtattcatgg ttttgaaccc tcatcaagtg atgttgctgc catttatgat    240
   gctgatctat ttctttatca ttcgcacaca ctagaagctt gggcgagacg tttggaacct    300
   agtttgcatc actctaaagt atctgtaatt gaagcttcaa aaggtatgac tttggataaa    360
   gttcatggct tagaagatgt agaggcagaa aaaggagtag atgagtcaac cttgtatgac    420
   cctcacactt ggaatgaccc tgtaaaagta tctgaggaag cacaactcat cgctacacaa    480
   ttagctaaaa aggatcctaa aaacgctaag gtttatcaaa aaaatgctga tcaatttagt    540
   gacaaggcaa tggctattgc agagaagtat aagccaaaat ttaaagctgc aaagtctaaa    600
   tactttgtga cttcacatac agcattctca tacttagcta agcgatacgg attgactcag    660
   ttaggtattg caggtgtctc aaccgagcaa gaacctagtg ctaaaaaatt agccgaaatt    720
   caggagtttg tgaaaacata taaggttaag actatttttg ttgaagaagg agtctcacct    780
   aaattagctc aagcagtagc ttcagctact cgagttaaaa ttgcaagttt aagtccttta    840
   raagcagttc ccaaaaacaa taaagattac ttagaaaatt tggaaactaa tcttaaggta    900
   cttgtcaaat cgttaaatca atag                                           924
<210> 40
   <211> 307
   <212> PRT
   <213> group B streptococcus
<220>
   <221> MISC_FEATURE
   <222> (281)..(281)
   <223> X is Lys or Glu
<400> 40
   Met Lys Arg Ile Arg Lys Ser Leu Ile Phe Val Leu Gly Val Val Thr
   1               5                   10                  15
   Leu Ile Cys Leu Cys Ala Cys Thr Lys Gln Ser Gln Gln Lys Asn Gly
           20                  25                      30
   Leu Ser Val Val Thr Ser Phe Tyr Pro Val Tyr Ser Ile Thr Lys Ala
       35                  40                  45
   Val Ser Gly Asp Leu Asn Asp Ile Lys Met Ile Arg Ser Gln Ser Gly
       50                  55                  60
   Ile His Gly Phe Glu Pro Ser Ser Ser Asp Val Ala Ala Ile Tyr Asp
   65                  70                  75                      80
   Ala Asp Leu Phe Leu Tyr His Ser His Thr Leu Glu Ala Trp Ala Arg
                   85                  90                  95
   Arg Leu Glu Pro Ser Leu His His Ser Lys Val Ser Val Ile Glu Ala
               100                 105                 110
   Ser Lys Gly Met Thr Leu Asp Lys Val His Gly Leu Glu Asp Val Glu
           115                 120                 125
   Ala Glu Lys Gly Val Asp Glu Ser Thr Leu Tyr Asp Pro His Thr Trp
       130                 135                 140
   Asn Asp Pro Val Lys Val Ser Glu Glu Ala Gln Leu Ile Ala Thr Gln
   145                 150                 155                     160
   Leu Ala Lys Lys Asp Pro Lys Asn Ala Lys Val Tyr Gln Lys Asn Ala
                   165                 170                 175
   Asp Gln Phe Ser Asp Lys Ala Met Ala Ile Ala Glu Lys Tyr Lys Pro
               180                 185                 190
   Lys Phe Lys Ala Ala Lys Ser Lys Tyr Phe Val Thr Ser His Thr Ala
           195                 200                 205
   Phe Ser Tyr Leu Ala Lys Arg Tyr Gly Leu Thr Gln Leu Gly Ile Ala
       210                 215                 220
   Gly Val Ser Thr Glu Gln Glu Pro Ser Ala Lys Lys Leu Ala Glu Ile
   225                 230                 235                     240
   Gln Glu Phe Val Lys Thr Tyr Lys Val Lys Thr Ile Phe Val Glu Glu
                   245                 250                 255
   Gly Val Ser Pro Lys Leu Ala Gln Ala Val Ala Ser Ala Thr Arg Val
               260                 265                 270
   Lys Ile Ala Ser Leu Ser Pro Leu Xaa Ala Val Pro Lys Asn Asn Lys
           275                 280                 285
   Asp Tyr Leu Glu Asn Leu Glu Thr Asn Leu Lys Val Leu Val Lys Ser
       290                 295                 300
   Leu Asn Gln
   305
<210> 41
   <211> 1134
   <212> DNA
   <213> group B streptococcus
<400> 41
   atgcctaaga agaaatcaga taccccagaa aaagaagaag ttgtcttaac ggaatggcaa     60
   aagcgtaacc ttgaattttt aaaaaaacgc aaagaagatg aagaagaaca aaaacgtatt    120
   aacgaaaaat tacgcttaga taaaagaagt aaattaaata tttcttctcc tgaagaacct    180
   caaaatacta ctaaaattaa gaagcttcat tttccaaaga tttcaagacc taagattgaa    240
   aagaaacaga aaaaagaaaa aatagtcaac agcttagcca aaactaatcg cattagaact    300
   gcacctatat ttgtagtagc attcctagtc attttagttt ccgttttcct actaactcct    360
   tttagtaagc aaaaaacaat aacagttagt ggaaatcagc atacacctga tgatattttg    420
   atagagaaaa cgaatattca aaaaaacgat tatttctttt ctttaatttt taaacataaa    480
   gctattgaac aacgtttagc tgcagaagat gtatgggtaa aaacagctca gatgacttat    540
   caatttccca ataagtttca tattcaagtt caagaaaata agattattgc atatgcacat    600
   acaaagcaag gatatcaacc tgtcttggaa actggaaaaa aggctgatcc tgtaaatagt    660
   tcagagctac caaagcactt cttaacaatt aaccttgata aggaagatag tattaagcta    720
   ttaattaaag atttaaaggc tttagaccct gatttaataa gtgagattca ggtgataagt    780
   ttagctgatt ctaaaacgac acctgacctc ctgctgttag atatgcacga tggaaatagt    840
   attagaatac cattatctaa atttaaagaa agacttcctt tttacaaaca aattaagaag    900
   aaccttaagg aaccttctat tgttgatatg gaagtgggag tttacacaac aacaaatacc    960
   attgaatcaa cccctgttaa agcagaagat acaaaaaata aatcaactga taaaacacaa   1020
   acacaaaatg gtcaggttgc ggaaaatagt caaggacaaa caaataactc aaatactaat   1080
   caacaaggac aacagatagc aacagagcag gcacctaacc ctcaaaatgt taat         1134
<210> 42
   <211> 378
   <212> PRT
   <213> group B streptococcus
<400> 42
   Met Pro Lys Lys Lys Ser Asp Thr Pro Glu Lys Glu Glu Val Val Leu
   1               5                   10                  15
   Thr Glu Trp Gln Lys Arg Asn Leu Glu Phe Leu Lys Lys Arg Lys Glu
               20                  25                  30
   Asp Glu Glu Glu Gln Lys Arg Ile Asn Glu Lys Leu Arg Leu Asp Lys
           35                  40                  45
   Arg Ser Lys Leu Asn Ile Ser Ser Pro Glu Glu Pro Gln Asn Thr Thr
       50                  55                  60
   Lys Ile Lys Lys Leu His Phe Pro Lys Ile Ser Arg Pro Lys Ile Glu
   65                  70                  75                  80
   Lys Lys Gln Lys Lys Glu Lys Ile Val Asn Ser Leu Ala Lys Thr Asn
                   85                  90                  95
   Arg Ile Arg Thr Ala Pro Ile Phe Val Val Ala Phe Leu Val Ile Leu
               100                 105                 110
   Val Ser Val Phe Leu Leu Thr Pro Phe Ser Lys Gln Lys Thr Ile Thr
           115                 120                 125
   Val Ser Gly Asn Gln His Thr Pro Asp Asp Ile Leu Ile Glu Lys Thr
       130                 135                 140
   Asn Ile Gln Lys Asn Asp Tyr Phe Phe Ser Leu Ile Phe Lys His Lys
   145                 150                 155                 160
   Ala Ile Glu Gln Arg Leu Ala Ala Glu Asp Val Trp Val Lys Thr Ala
                   165                 170                 175
   Gln Met Thr Tyr Gln Phe Pro Asn Lys Phe His Ile Gln Val Gln Glu
               180                 185                 190
   Asn Lys Ile Ile Ala Tyr Ala His Thr Lys Gln Gly Tyr Gln Pro Val
           195                 200                 205
   Leu Glu Thr Gly Lys Lys Ala Asp Pro Val Asn Ser Ser Glu Leu Pro
       210                 215                 220
   Lys His Phe Leu Thr Ile Asn Leu Asp Lys Glu Asp Ser Ile Lys Leu
   225 230 235 240
   Leu Ile Lys Asp Leu Lys Ala Leu Asp Pro Asp Leu Ile Ser Glu Ile
   245 250 255
   Gln Val Ile Ser Leu Ala Asp Ser Lys Thr Thr Pro Asp Leu Leu Leu
   260 265 270
   Leu Asp Met His Asp Gly Asn Ser Ile Arg Ile Pro Leu Ser Lys Phe
   275 280 285
   Lys Glu Arg Leu Pro Phe Tyr Lys Gln Ile Lys Lys Asn Leu Lys Glu
   290 295 300
   Pro Ser Ile Val Asp Met Glu Val Gly Val Tyr Thr Thr Thr Asn Thr
   305 310 315 320
   Ile Glu Ser Thr Pro Val Lys Ala Glu Asp Thr Lys Asn Lys Ser Thr
   325 330 335
   Asp Lys Thr Gln Thr Gln Asn Gly Gln Val Ala Glu Asn Ser Gln Gly
   340 345 350
   Gln Thr Asn Asn Ser Asn Thr Asn Gln Gln Gly Gln Gln Ile Ala Thr
   355 360 365
   Glu Gln Ala Pro Asn Pro Gln Asn Val Asn
   370 375